# FASCICULE DE BREVET EUROPEEN

(11) **EP 0 879 814 B1**
(45) Date de publication et mention de la délivrance du brevet: **28.11.2001**
(21) Numéro de dépôt: 98401144.5
(22) Date de dépôt: 13.05.1998
(51) Int. Cl.: C07C 63/66, C07C 63/74, C07C 65/19, C07C 65/28, C07C 233/55, C07C 233/65, C07C 233/75, C07C 327/44, C07C 33/28, C07C 39/21, C07C 47/548, C07C 69/618, C07D 213/55, C07D 213/79, C07D 213/80, C07D 295/18, C07D 333/24, C07D 333/38, A61K 31/19, A61K 31/045, A61K 31/05, A61K 31/11, A61K 31/165, A61K 31/215, A61K 31/38, A61K 31/44, A61K 31/535

(54) **Composés triaromatiques, compositions les contenant et utilisations**
Triaromatische Verbindungen, diese enthaltende Zusammensetzungen sowie ihre Anwendungen
Triaromatic compounds, compositions containing them and uses thereof

(30) Priorité: 23.05.1997 FR 9706340
(43) Date de publication de la demande: 25.11.1998
(73) Titulaire: CENTRE INTERNATIONAL DE RECHERCHES DERMATOLOGIQUES GALDERMA (C.I.R.D. GALDERMA), 06560 Valbonne (FR)
(72) Inventeur: Bernardon, Jean-Michel, 06650 Le Rouret (FR); Nedoncelle, Philippe, 06130 Grasse (FR)
(74) Mandataire: Tezier Herman, Béatrice

(56) Documents cités:
- CHANDRARATNA, R.A.S. ET AL: "Development of RAR subtype selective retinoids for dermatological diseases" EUROPEAN JOURNAL OF MEDICINAL CHEMISTRY. CHIMICA THERAPEUTICA, vol. 30, 1995, pages 505s-517s, XP000569537
- TORRADO, A. ; L PEZ, S. ; ALVAREZ, R. ; DE LERA, A.R.: "General synthesis of retinoids and arotinoids via palladium-catalyzed cross-coupling of boronic acids with electrophiles" SYNTHESIS, 1995, pages 285-293, XP002057741
- JONG, L. ; LEHMANN, J.M. ; HOBBS, P.D. ; HARLEV, E. ; HUFFMAN, J.C. ; PFAHL, M. ; DAWSON, M.I.: "Conformational effects on retinoid receptor selectivity. 1. Effect of 9-double bond geometry on retinoid X receptor activity" JOURNAL OF MEDICINAL CHEMISTRY, vol. 36, no. 18, 1993, pages 2605-2613, XP000563864
- KAGECHIKA, H. ; KAWACHI, E. ; HASHIMOTO, Y. ; HIMI, T. ; SHUDO, K.: "Retinobenzoic acids. 1. Structure-activity relationships of aromatic amides with retinoidal activity" JOURNAL OF MEDICINAL CHEMISTRY, vol. 31, no. 11, 1988, pages 2182-2192, XP000608417
- CHEMICAL ABSTRACTS, vol. 107, no. 19, 9 novembre 1987 Columbus, Ohio, US; abstract no. 175669e, page 686; XP002057742 -& CHEMICAL ABSTRACTS, CHEMICAL SUBSTANCE INDEX, 1987 - 1991, page 17371CS XP002057982 & JP 61 271 262 A (HISAMITSU PHARMACEUTICAL CO., INC., JP)

## Description

L'invention concerne, à titre de produits industriels nouveaux et utiles, des composés triaromatiques. Elle concerne également l'utilisation de ces nouveaux composés dans des compositions pharmaceutiques destinées à un usage en médecine humaine ou vétérinaire, ou bien encore dans des compositions cosmétiques.

Le document publié dans J. Med. Chem. (1988), vol. 31, pages 2182-2192 décrit des composés rétinoides de type carboxamidobenzoiques tels que le composé Am 160. Ces composés ne présentent pas de liaison entre les deux cycles aromatiques comprenant une double ou une triple liaison en position α du groupement -CO-NR-. Par ailleurs ces composés ne présentent pas d'activité biologique du type antagoniste des récepteurs RARs.

Les composés selon l'invention ont une activité marquée dans les domaines de la différenciation et de la prolifération cellulaire, et trouvent des applications plus particulièrement dans le traitement topique et systémique des affections dermatologiques liées à un désordre de la kératinisation, des affections dermatologiques (ou autres) à composante inflammatoire et/ou immunoallergique, et des proliférations dermiques- ou épidermiques qu'elles soient bénignes ou malignes. Ces composés peuvent en outre être utilisés dans le traitement des maladies de dégénérescence du tissu conjonctif, pour lutter contre le vieillissement de la peau, qu'il soit photoinduit ou chronologique, et traiter les troubles de la cicatrisation. Ils trouvent par ailleurs une application dans le domaine ophtalmologique, notamment dans le traitement des cornéopathies.

On peut également utiliser les composés selon l'invention dans des compositions cosmétiques pour l'hygiène corporelle et capillaire.

Les composés selon l'invention peuvent être représentés par la formule générale (I) suivante : 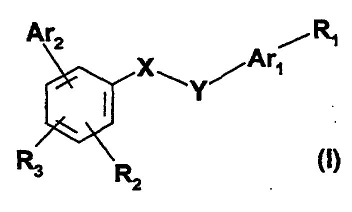 dans laquelle:
- R₁ représente
   (i) le radical -CH₃
   (ii) le radical -CH₂OH
   (iii) le radical -O-R₄
   (iv) le radical -CO-R₅
   R₄ et R₅ ayant les significations données ci-après.
- Ar₁ est un radical choisi parmi les radicaux de formules (a) ou (b) suivantes: 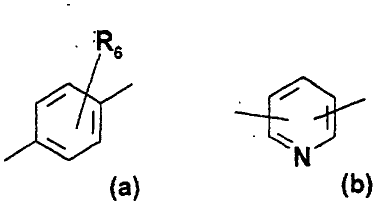 R₆ ayant la signification donnée ci-après.
- X-Y représente une liaison choisie parmi les liaisons de formules (e) à (m) suivantes pouvant être lues de gauche à droite ou inversement : 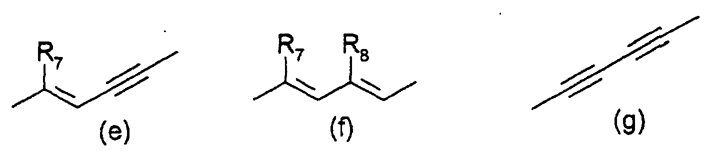 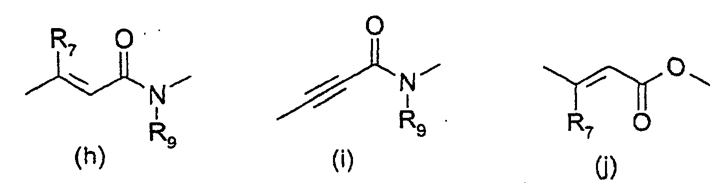 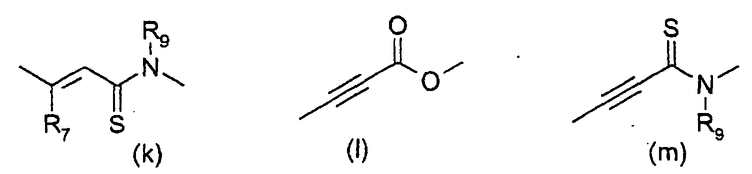 R₇,_{,}R₈ et R₉ ayant les significations données ci-après,
- Ar₂ est un radical choisi parmi les radicaux de formules (j) à (m) suivantes: 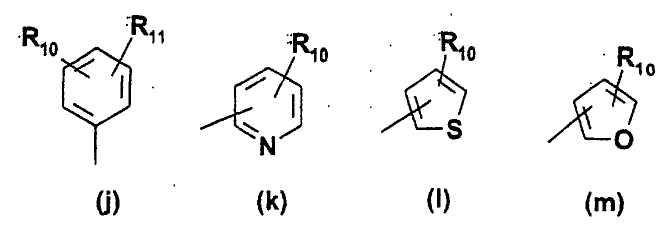 Ar₂ étant en position ortho ou méta sur le phényl par rapport la liaison X-Y
   R₁₀ et R₁₁ ayant les significations données ci-aprés,
- R₂ et R₃, identiques ou différents, représentent un atc me d'hydrogène, un radical alkyle linéaire ou ramifié ayant de 1 à 20 atomes de carbone, un atome d'halogène, un radical hydroxyle éventuellement protégé sous forme d'acétoxy, un radical alkoxy, un radical polyéther, un radical nitro, un radical amino événtuellement protégé sous forme d'acétamide ou substitué par un ou deux groupements alkyles, ayant de 1 à 6 atomes de carbone,
- R₄ représente un atome d'hydrogène, un radical alkyle ayant de 1 à 6 atomes de carbone ou un radical -CO-R₁₂,.
   R₁₂ ayant la signification donnée ci-après,
- R₅ représente :
   (a) un atome d'hydrogène
   (b) un radical alkyle ayant de 1 à 6 atomes de carbone
   (c) un radical de formule : 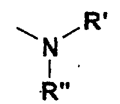 R' et R" ayant les significations données ci-après,
   (d) un radical -OR₁₃,
      R₁₃ ayant la signification donnée ci-après,
- R₆ représente un atome d'hydrogène, un atome d'halogène, un radical alkyle linéaire ou ramifié ayant de 1 à 20 atomes de carbone, un radical hydroxyle, un radical -OR₁₄ ou -OCOR₁₄, ou un radical polyéther,
   R₁₄ ayant la signification donnée ci-après,
- R₇ et R₈, identiques ou différents, représentent un atome d'hydrogène, un radical alkyle, ayant de 1 à 6 atomes de carbone,
- R₉ représente un atome d'hydrogène ou un radical alkyle ayant de 1 à 6 atomes de carbone,
- R₁₀ et R₁₁, identiques ou différents, représentent un atome d'hydrogène, un radical alkyle linéaire ou ramifié ayant de 1 à 20 atomes de carbone, un atome d'halogène, un radical hydroxyle éventuellement protégé sous forme d'acétoxy, un radical alkoxy, un radical polyéther, un radical nitro, un radical amino événtuellement protégé sous forme d'acétamide ou subst tué par un ou deux groupements alkyles ayant de 1 à 6 atomes de carbone, un radical CF₃, un radical alkényle ou un radical -(CH₂)ₙ-R₁₅,
   n et R₁₅ ayant les significations données ci-après
- R' et R", ayant de 1 à 6 atomes de carbone identiques ou différents, représentent un atome d'hydrogène, un radical alkyle, un radical mono ou polyhydroxyalkyle, un radical aryle éventuellement substitué ou un reste d'aminoacide ou de peptide où encore pris ensemble, forment un hétérocycle,
- R₁₂ représente un radical alkyle, ayant de 1 à 6 atomes de carbone,
- R₁₃ représente un atome d'hydrogène, un radical alkyle linéaire ou ramifié, ayant de 1 à 20 atomes de carbone, un radical alkényle; un radical mono ou polyhydroxyalkyle, un radical aryle ou aralkyle éventuellement substitué(s) ou un reste de sucre,
- R₁₄ représente un radical alkyle ayant de 1 à 6 atomes de carbone,
- R₁₅ représente un radical hydroxyle éventuellement protégé sous forme d'acétoxy, un radical alkoxy, ou un radical polyéther,
- n représente un nombre entier compris entre 1 et 6 inclus,
et les isomères optiques et géométriques desdits composés de formule (I) ainsi que leurs sels.

Ainsi, l'invention vise également les sels des composés de formule (I) lorsque R₁ représente une fonction acide carboxylique ou lorsque R₂, R₃, R₁₀ ou R₁₁ représente une fonction amine. Lorsque les composés selon l'invention se présentent sous forme de sels, par addition d'un acide, il s'agit de sels pharmaceutiquement ou cosmétiquement acceptables obtenus par addition d'un acide minéral ou organique, en particulier l'acide chlorhydrique, sulfurique, acétique, citrique, fumarique, hémisuccinique, maléique et mandélique. Lorsque les composés selon l'invention se présentent sous forme de sels par addition d'une base, il s'agit préférentiellement de sels d'un métal alcalin ou alcalino-terreux ou encore de zinc ou d'une amine organique.

Selon la présente invention, parmi les radicaux alkyle linéaire ou ramifié ayant de 1 à 20 atomes de carbone, on peut citer avantageusement les radicaux méthyle, éthyle, isopropyle, butyle, tertiobutyle, hexyle, octyle, nonyle, 2-éthyl-hexyl et dodécyle. De préférence, ces radicaux présentent de 1 à 12 atomes de carbone. Losqu'il est inférieur, le radical alkyle comprend généralement de 1 à 6 atomes de carbone. On peut citer, comme radical alkyle ayant de 1 à 6 atomes de carbone, les radicaux méthyle, éthyle, propyle, isopropyle, tertiobutyle, hexyle.

Parmi les radicaux alkyle linéaire ayant de 1 à 20 atomes de carbone, on peut citer notamment Jes radicaux méthyle, éthyle, propyle, 2-éthyl-hexyle, octyle, dodécyle, hexadécyle et octadécyle.

Parmi les radicaux alkyle ramifié ayant de 1 à 20 atomes de carbone, on peut citer notamment les radicaux 2-méthylbutyle, 2-méthylpentyle, 1-méthylhexyle, 3-méthylheptyle.

Par radical alkényle, on entend un radical ayant de 2 à 20 atomes de carbone linéaire ou ramifié comportant une ou plusieurs doubles liaisons.

Parmi les radicaux alkényle, on préfère un radical contenant de 2 à 5 atomes de carbone et présentant une ou plusieurs insaturations éthyléniques, tel que plus particulièrement le radical allyle.

Par radical monohydroxyalkyle ou polyhydroxyalkyle, on doit entendre un radical contenant de 1 à 6 atomes de carbone et de 1 à 5 groupes hydroxyles.

Parmi les radicaux monohydroxyalkyle, on préfère un radical contenant de préférence 1 ou 3 atomes de carbone, notamment les radicaux hydroxyméthyl, 2-hydroxyéthyl, 2 ou 3-hydroxypropyle.

Parmi les radicaux polyhydroxyalkyle, on préfère un radical présentant de 3 à 6 atomes de carbone et de 2 à 5 groupes hydroxyles, tels que les radicaux 2,3-dihydroxypropyle, 2,3,4-trihydroxybutyle, 2,3,4,5-tétrahydroxypentyle ou le reste du pentaérythritol.

Parmi les radicaux aryle, on préfère un radical phényle, thiophène ou pyridine, éventuellement substitué par au moins un atome d'halogène, un radical hydroxyle, un radical alkyle, une fonction nitro, un groupe méthoxy ou une fonction amine éventuellement substituée. On préfère le radical phényle éventuellement substitué.

Parmi les radicaux aralkyle, on préfère le radical benzyle ou phénéthyle éventuellement substitué par au moins un atome d'halogène, un radical hydroxyle, une fonction nitro ou un groupe méthoxy.

Par reste de sucre, on entend un reste dérivant notamment de glucose, de galactose ou de mannose, ou bien encore de l'acide glucuronique.

Par reste d'aminoacide, on entend notamment un reste dérivant de l'un des acides aminés tels que la lysine, la glycine ou l'acide aspartique, et par reste de peptide, on entend plus particulièrement un reste de dipeptide ou de tripeptide résultant de la combinaison d'acides aminés.

Par hétérocycle, on entend de préférence un radical pipéridino, morpholino, pyrrolidino ou pipérazino, éventuellement substitué en position 4 par un radical alkyle en C₁-C₆ ou polyhydroxyalkyle tels que définis ci-dessus.

Par radical polyéther, on entend un radical contenant de préférence 2 à 6 atomes de carbone, notamment les radicaux méthoxyméthoxy, méthoxyéthoxy, méthoxyéthoxyméthoxy, méthoxyméthoxyéthyl, méthoxyméthoxypropyl et méthoxyhexyloxy.

Parmi les radicaux alkoxy, on préfère un radical alkoxy contenant de 1 à 12 atomes de carbone, tels que notamment les radicaux méthoxy éthoxy, propyloxy, isopropyloxy, hexyloxy, heptyloxy, octyloxy et nonyloxy.

Lorsque les radicaux R₂, R₃, R₆, R₁₀, R₁₁ représentent un atome d'halogène, celui-ci est de préférence un atome de fluor, de brome ou de chlore.

Parmi les composés de formule (I) ci-dessus rentrant dans le cadre de la présente invention, on peut notamment citer les suivants:
Acide 4-[4-(biphényl-2-yl)but-3-en-1-ynyl)benzoïque
Acide 4-[4-(4'-méthylbiphényl-2-yl)but-3-en-1-ynyl]benzoïque.
Acide 4-[3-(4'-méthyl-biphényl-2-yl)-acryloylamino]-benzoïque.
Acide 4-[3-(4'-méthyl-biphényl-2-yl)-(E)-thioacryloylamino]-benzoïque.
Acide 4-[3-(4'-méthyl-biphényl-2-yl)-acryloyloxy]-benzoïque.
Acide 4-[4-(4'-méthyl-biphényl-2-yl)-buta-1(E),3(Z)-diènyl]-benzoïque.
Acide 4-[4-(4'-méthyl-biphényl-2-yl)-buta-1(E),3(E)-diènyl]-benzoïque.
Acide 4-[3-(4'-méthyl-biphényl-2-yl)-propynoyloxy]-benzoïque.
Acides 4-[4-(4'-méthyl-biphényl-2-yl)-(E)/(Z)-but-1-èn-3-ynyl]-benzoïques.
Acide 4-[4-(4'-méthyl-biphényl-2-yl)-buta-1,3-diynyl]-benzoïque.
Acide 4-[4-(3-fluoro-4'-méthyl-biphényl-2-yl)-but-3-èn-(E)-1-ynyl]-benzoïque.
Acide 4-[4-(4,4'-diméthyl-biphényl-2-yl)-but-3-èn-1-ynyl]-benzoïque.
Acide 4-[4-(5,4'-diméthyl-biphényl-2-yl)-but-3-èn-1-ynyl]-benzoïque.
Acide 4-[4-(6,4'-diméthyl-biphényl-2-yl )-but-3-èn-(E)-1-ynyl]-benzoïque.
Acide 4-[4-(4-hydroxy-4'-méthyl-biphényl-2-yl)-but-3-èn-(E)-1-ynyl]-benzoïque.
Acide 4-[4-(5-hydroxy-4'-méthyl-biphényl-2-yl)-but-3-èn-(E)-1-ynyl]-benzoïque.
Acide 4-[4-(6-hydroxy-4'-méthyl-biphényl-2-yl)-but-3-èn-(E)-1-ynyl]-benzoïque.
Acide 4-[4-(4'-méthyl-biphényl-2-yl)-pent-3-èn-(E)-1-ynyl]-benzoïque.
Acide 4-[3-(4'-méthyl-biphényl-2-yl)-propynoylamino]-benzoïque.
Acide 4-[3-(4'-méthyl-biphényl-2-yl)-propynethioylamino]-benzoïque.
Acide 4-[4-(3'-méthyl-biphényl-2-yl)-but-3-èn-1-ynyl]-benzoïque.
Acide 4-[4-(2'-méthyl-biphényl-2-yl)-but-3-èn-(E)-1-ynyl]-benzoïque.
Acide 4-[4-(4'-chloro-biphényl-2-yl)-but-3-èn-1-ynyl]-benzoïque.
Acide 4-[4-(3'-chloro-biphényl-2-yl)-but-3-èn-(E)-1-ynyl]-benzoïque.
Acide 4-[4-(4'-fluoro-biphényl-2-yl)-but-3-èn-(E)-1-ynyl]-benzoïque.
Acide 4-[4-(4'-propyl-biphényl-2-yl)-but-3-èn-(E)-1-ynyl]-benzoïque.
Acide 4-[4-(4'-vinyl-biphényl-2-yl)-but-3-èn-(E)-1 -ynyl]-benzoïque.
Acide 4-[4-(4'-méthoxyméthoxy-biphényl-2-yl)-but-3-èn-(E)-1-ynyl]-benzoïque.
Acide 4-[4-(3'-méthoxyméthoxy-biphényl-2-yl)-but-3-èn-(E)-1-ynyl]-benzoïque.
Acide 4-[4-(2-thiophène-3-yl-phényl)-but-3-èn-(E)-1-ynyl]-benzoïque.
Acide 4-[4-(2-thiophène-2-yl-phényl)-but-3-èn-(E)-1-ynyl]-benzoïque.
Acide 4-[4-(4'-hydroxy-biphényl-2-yl)-but-3-èn-(E)-1-ynyl]-benzoïque.
Acide 4-[4-(4'-méthoxy-biphényl-2-yl)-but-3-èn-(E)-1-ynyl]-benzoïque.
Acide 4-[4-(4'-propoxy-biphényl-2-yl)-but-3-èn-(E)-1-ynyl]-benzoïque.
Acide 4-[4-(3'-hydroxy-biphényl-2-yl)-but-3-èn-(E)-1-ynyl]-benzoïque.
Acide 4-[4-(3'-méthoxy-biphényl-2-yl)-but-3-èn-(E)-1-ynyl]-benzoïque.
Acide 4-[4-(3'-propoxy-biphényl-2-yl)-but-3-èn-(E)-1-ynyl]-benzoïque.
Acide 4-[4-(4'-méthyl-4-hydroxy-biphényl-2-yl )-but-3-èn-(E)-1-ynyl]-benzoïque.
Acide 4-[4-(4'-méthyl-4-méthoxy-biphényl-2-yl)-but-3-èn-(E)-1-ynyl]-benzoïque.
Acide 4-[4-(4'-méthyl-4-propoxy-biphényl-2-yl)-but-3-èn-(E)-1-ynyl]-benzoïque.
Acide 4-[4-(4'-méthyl-5-hydroxy-biphényl-2-yl)-but-3-èn-(E)-1 -ynyl]-benzoïque.
Acide 4-[4-(4'-méthyl-5-méthoxy-biphényl-2-yl)-but-3-èn-(E)-1-ynyl]-benzoïque.
Acide 4-[4-(4'-méthyl-5-propoxy-biphényl-2-yl)-but-3-èn-(E)-1-ynyl]-benzoïque.
Acide 4-[4-(4'-méthyl-6-hydroxy-biphényl-2-yl)-but-3-èn-(E)-1-ynyl]-benzoïque.
Acide 4-[4-(4'-méthyl-6-méthoxy-biphényl-2-yl)-but-3-èn-(E)-1-ynyl]-benzoïque.
Acide 4-[4-(4'-trifuorométhyl-biphényl-2-yl)-but-3-èn(E)-1-ynyl]-benzoïque.
Acide 4-[4-(4'-hydroxyméthyl-biphényl-2-yl)-but-3-èn-(E)-1-ynyl]-benzoïque.
Acide 4-{4-[4'-(2-hydroxy-éthyl)-biphényl-2-yl]-but-3-èn-(E)-1-ynyl}-benzoïque.
Acide 4-[4-(3'-méthyl-biphényl-3-yl)-but-3-èn-(E)-1-ynyl]-benzoïque.
Acide 4-[4-(2-pyridin-4-yl-phényl)-but-3-èn-(E)-1-ynyl]-benzoïque.
Acide 4-[4-(2-pyridin-3-yl-phényl)-but-3-èn-(E)-1-ynyl]-benzoïque.
Acide 4-[4-(3-méthoxyméthoxy-4'-méthyl-biphényl-2-yl)-but-3-èn-(E)-1-ynyl]-benzoïque.
Acide 4-[4-(3-hydroxy-4'-méthyl-biphényl-2-yl)-but-3-èn-(E)-1-ynyl]-benzoïque.
Acide 4-[4-(3-méthoxy-4'-méthyl-biphényl-2-yl)-but-3-èn-(E)-1-ynyl]-benzoïque.
Acide 4-[4-(4'-éthoxyméthoxyméthyl-biphényl-2-yl)-but-3-èn-(E )-1-ynyl]-benzoïque.
Acide 4-[4-(4'-éthoxyméthoxyéthyl-biphényl-2-yl)-but-3-èn-(E)-1-ynyl]-benzoate de méthyle.
Acide 2-méthyl-4-[4-(4'-méthyl-biphényl-2-yl)-but-3-èn-(E)-1-ynyl]-benzoïque.
Acide 2-hydroxy-4-[4-(4'-méthyl-biphényl-2-yl)-but-3-èn-(E)-1-ynyl]-benzoïque.
Acide 6-[4-(4'-méthyl-biphényl-2-yl)-but-3-èn-(E)-1-ynyl]-pyridine-3-carboxylique.
Acide 5-[4-(4'-méthyl-biphényl-2-yl)-but-3-èn-(E)-1-ynyl]-pyridine-2-carboxylique.
Acide 3-méthoxyméthoxy-4-[4-(4'-méthyl-biphényl-2-yl)-but-3-èn-(E)-1-ynyl]-benzoïque.
Acide 3-hydroxy-4-[4-(4'-méthyl-biphényl-2-yl)-but-3-èn-(E)-1-ynyl]-benzoïque.
Acide 3-méthoxy-4-[4-(4'-méthyl-biphényl-2-yl)-but-3-èn-(E)-1-ynyl]-benzoïque.
{4-[4-(4'-Méthyl-biphényl-2-yl)-but-3-èn-(E)-1-ynyl]-phényl}-méthanol.
4-[4-(4'-Méthyl-biphényl-2-yl)-but-3-èn-(E)-1-ynyl]-benzaldéhyde.
4-[4-(4'-Méthyl-biphényl-2-yl)-but-3-èn-(E)-1-ynyl]-phénol.
4-[4-(4'-Méthyl-biphényl-2-yl)-but-3-èn-(E)-1-ynyl)-benzamide.
N-Ethyl-4-[4-(4'-méthyl-biphényl-2-yl)-but-3-èn-(E)-1-ynyl]-benzamide.
{4-[4-(4'-Méthyl-biphényl-2-yl)-but-3-èn-(E)-1-ynyl]-phényl}-morpholin-4-yl-méthanone.
N-(4-Hydroxy-phényl)-4-[4-(4'-méthyl-biphényl-2-yl)-but-3-èn-(E)-1-ynyl]-benzamide.

Selon la présente invention, les composés de formule (I) plus particulièrement préférés sont ceux pour lesquels l'une au moins et de préférence toutes les conditions ci-dessous sont respectées :
- R₁ est le radical -CO-R₅,
- Ar₁ représente les radicaux de formules (a) ou (b),
- X-Y représente les liaisons de formule (e), (f), ou (h).
- Ar₂ représente le radical de formule (j),

De préférence, les composés présentent la formule (I) dans laquelle R₁ est le radical -CO-OR₁₃, avec R₁₃ représentant un atome d'hydrogène ou un radical alkyle linéaire ou ramifié ayant de 1 à 20 atomes de carbone, tel que défini ci-dessus.

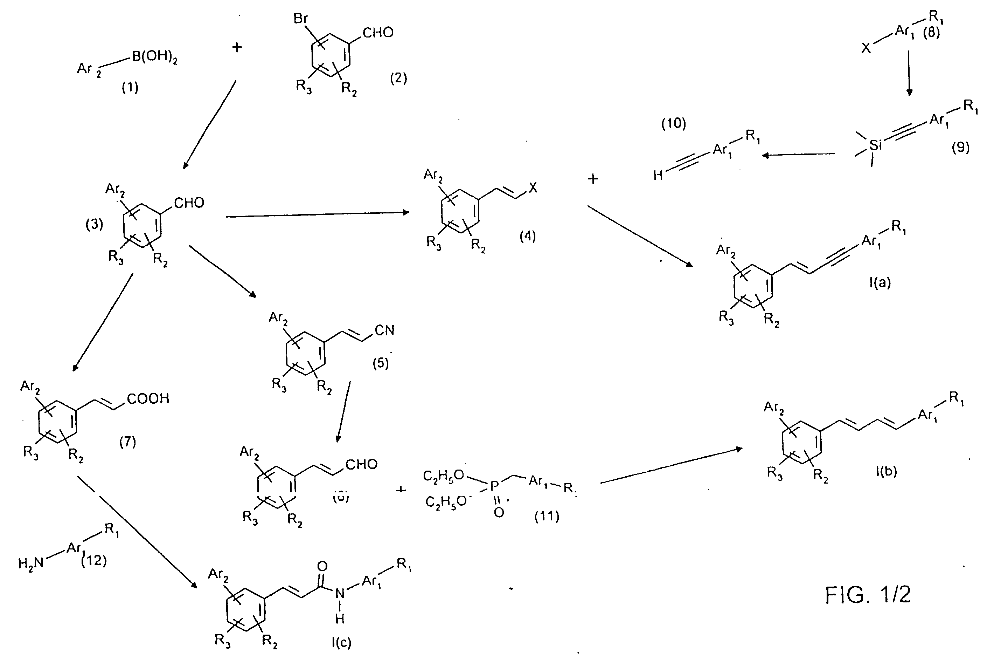

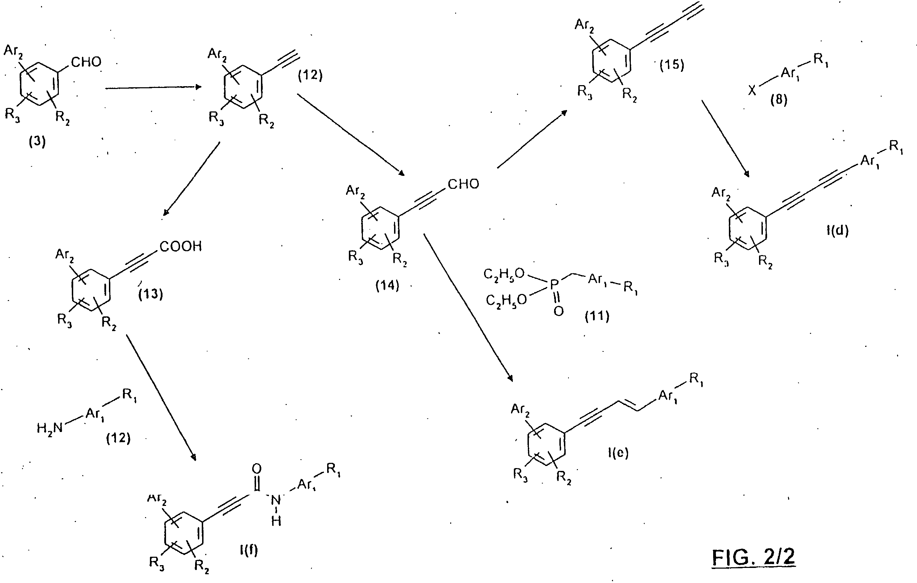

La présente invention a également pour objet les procédés de préparation des. composés de formule (I), en particulier selon les schémas réactionnels donnés aux figures 1 et 2.

Ainsi, les composés de formule I(a) peuvent être obtenus (figure 1) par une réaction de couplage entre les dérivés acétyléniques (10) et les dérivés halogénés (de préférence iodés) (4) dans un solvant tel que le DMF en présence de PPh₃, Cul et K₂CO₃ (selon K. Okuro Tetrahedron Letters, 1992, Vol 33, No 37, 5363-4). Les dérivés halogénés (4) peuvent être obtenus à partir des aldéhydes (3) soit par réaction de type Wittig avec le bromure de bromométhylènetriphénylphosphonium (D. R. Williams Tétrahedron Letters, 1981, Vol 22, No 38, 3745-8) ou par réaction avec l'iodoform en présence de CrCl₂ (K. Takai J. Am. Chem. Soc. 1986, *108*, 7408-10). Les dérivés aldéhydiques (3) étant obtenus par une réaction de couplage de type Suzuki entre les dérivés boroniques (1) et les dérivés halogénés (2) en présence d'un catalyseur tel le Pd(PPh₃)₄.

Ainsi, les composés de formule I(b) peuvent être obtenus (figure 1) par une réaction de Horner-Emmons entre des dérivés aldéhydiques (6) et des dérivés phosphonates (11). Les dérivés (6) peuvent être obtenus par réduction des dérivés nitriles (5) à l'aide de l'hydrure de diisobutylaluminium. Les dérivés (5) étant obtenus par une réaction de Horner-Emmons entre des dérivés aldéhydiques (3) et le cyanométhylphosphonate de diéthyle en présence d'une base, telle que l'hydroxyde de potassium.

Ainsi, les composés de formule I(c) peuvent être obtenus (figure 1) en faisant réagir en milieu anhydre, dans un solvant organique de préférence le THF et en présence d'une amine tertiaire (par exemple triéthylamine ou pyridine) une forme activée d'un dérivé cinnamique (7) par exemple un chlorure d'acide sur un dérivé d'aniline de formule (12). Les dérivés cinnamiques (7) pouvant être obtenus par une réaction de type Knoevenagel à partir des dérivés aldéhydiques (3) par réaction avec l'acide malonique ou le malonate de diéthyle.

Ainsi, les composés de formule I(d) peuvent être obtenus (figure 2) par une réaction de couplage entre les dérivés di-acétyléniques (15) et les dérivés halogénés (de préférence iodés) (8) dans un solvant tel que le DMF en présence de PPh₃, Cul et K₂CO₃ Les dérivés (15) étant obtenus par une réaction de Corey-Fuchs à partir des aldéhydes (14) eux-mêmes obtenus à partir des dérivés acétyléniques (12) par lithiation puis réaction avec le DMF. Les dérivés (12) étant obtenus à partir des dérivés aldéhydiques (3) par la réaction de Corey-Fuchs.

Ainsi, les composés de formule I(e) peuvent être obtenus (figure 2) par une réaction de Horner-Emmons entre des dérivés aldéhydiques (14) et des dérivés phosphonates (11). Les dérivés (14) pouvant être obtenus par lithiation des dérivés acétyléniques (12) avec le butyllithium puis réaction avec le DMF.

Ainsi les composés de formule I(f) peuvent être obtenus (figure 2) en faisant réagir en milieu anhydre, dans un solvant organique de préférence le THF et en présence d'une amine tertiaire (par exemple triéthylamine ou pyridine) une forme activée d'un acide phénylpropiolique (13) par exemple un chlorure d'acide (3) sur un dérivé d'aniline de formule (12). Les dérivés (13) peuvent être obtenus par lithiation des dérivés acétyléniques (12) avec le butyllithium puis réaction avec CO₂.

Lorsque R₁ représente le radical -COOH, les composés sont préparés :
- soit en protégeant R₁ par un groupe protecteur de type alkyle, allylique, benzylique ou tert-butylique.

Le passage à la forme libre peut être effectué :
- dans le cas d'un groupe protecteur alkyle, au moyen de soude ou d'hydroxyde de lithium dans un solvant alcoolique tel le méthanol ou dans le THF.
- dans le cas d'un groupe protecteur allylique, au moyen d'un catalyseur tel certains complexes de métaux de transition en présence d'une amine secondaire telle la morpholine.
- dans le cas d'un groupement protecteur benzylique, par débenzylation en présence d'hydrogène au moyen d'un catalyseur tel que le palladium sur charbon.
- dans le cas d'un groupement protecteur de type tert-butylique au moyen d'iodure de triméthylsilane.
- soit à partir du phénol correspondant par transformation en dérivé triflate puis carbonylation en présence de catalyseur au palladium.

Lorsque R₁ représente une fonction alcool les composés peuvent être obtenus :
- à partir des dérivés aldéhydiques correspondants par action d'un hydrure alcalin, tel le borohydrure de sodium, dans un solvant alcoolique (par exemple le méthanol).
- à partir des dérivés acides par réduction avec l'hydrure double de lithium et d'aluminium.

Lorsque R₁ représente une fonction aldéhyde les composés peuvent être obtenus à partir des dérivés alcools par oxydation en présence d'oxyde de manganèse, de pyridinium dichromate ou du réactif de Swem.

Lorsque R₁ représente une fonction amide les composés peuvent être obtenus à partir des dérivés carboxyliques correspondants par réaction avec des amines aliphatiques, aromatiques, hétérocycliques soit par l'intermédiaire d'un chlorure d'acide ou en présence de dicyclohexylcarbodiimide ou de carbonyldiimidazole.

Les produits de formule générale (I) ainsi obtenus peuvent servir de produits de départ pour la fabrication d'autres composés de formule (I) selon l'invention. Ces composés sont obtenus selon les méthodes classiques de synthèse employées en chimie, telles que celles décrites dans "Advanced Organic Chemistry" de J. March; John Willey and Sons, 1985.

Par exemple, on peut procéder aux modifications fonctionnelles du groupe R₁ comme indiqué ci-dessous:

| | |
|---|---|
| acide carboxylique | → ester |
| ester | → acide carboxylique |
| acide | → chlorure d' acide |
| chlorure d' acide | → amide |
| acide | → amide |
| acide | → alcool |
| alcool | → aldéhyde |
| amide | → amine |

Les composés selon l'invention présentent une activité dans le test de différenciation des cellules (F9) de tératocarcinome embryonnaire de souris (Cancer Research 43, p. 5268, 1983) et/ou dans le test d'inhibition de l'ornithine décarboxylase après induction par le TPA chez la souris (Cancer Research 38, p. 793-801, 1978). Ces tests montrent les activités de ces composés respectivement dans les domaines de la différenciation et de la prolifération cellulaire. Dans le test de différenciation des cellules (F9), il est possible d'évaluer une activité agoniste, comme une activité antagoniste aux récepteurs de l'acide rétinoïque. En effet, un antagoniste est inactif lorsqu'il est seul dans ce test, mais inhibe partiellement ou totalement l'effet produit par un rétinoïde agoniste sur la morphologie et sur la sécrétion de l'activateur plasminogène. Ces composés présentent donc également une activité dans un test qui consiste à identifier des molécules antagonistes des RARs, tel que décrit dans la demande de brevet français n° 95-07302 déposée le 19 juin 1995 par la Demanderesse. Ce test comprend les étapes suivantes : (i) on applique topiquement sur une partie de la peau d'un mammifère une quantité suffisante d'une molécule agoniste des RARs, (ii) on administre par voie systémique ou topique sur ce même mammifère ou sur cette même partie de la peau du mammifère, avant, pendant ou après l'étape (i), une molécule susceptible de présenter une activité antagoniste des RARs et (iii) on évalue la réponse sur la partie de la peau ainsi traitée du mammifère. Ainsi, la réponse à une application topique sur l'oreille d'un mammifère d'une molécule agoniste des RARs qui correspond à une augmentation de l'épaisseur de cette oreille peut être inhibée par l'administration par voie systémique ou topique d'une molécule antagoniste des RARs.

La présente invention a également pour objet à titre de médicament les composés de formule (I) telle que définie ci-dessus.

Les composés selon l'invention conviennent particulièrement bien dans les domaines de traitement suivants :
1) pour traiter les affections dermatologiques liées à un désordre de la kératinisation portant sur la différenciation et sur la prolifération notamment pour traiter les acnées vulgaires, comédoniennes, polymorphes, rosacées, les acnées nodulokystiques, conglobata, les acnés séniles, les acnés secondaires telles que l'acné solaire, médicamenteuse ou professionnelle,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ; les composés peuvent également être utilisés dans certaines affections inflammatoires ne présentant pas de trouble de la kératinisation,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires,
5) pour traiter d'autres désordres dermatologiques tels que les dermatoses bulleuses et les maladies du collagène,
6) pour traiter certains troubles ophtalmologiques, notamment les cornéopathies,
7) pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique,
8) pour prévenir ou guérir les stigmates de l'atrophie épidermique et/ou dermique induite par les corticostéroïdes locaux ou systémiques, ou toute autre forme d'atrophie cutanée,
9) pour prévenir ou traiter les troubles de la cicatrisation ou pour prévenir ou pour réparer les vergetures,
10) pour lutter contre les troubles de la fonction sébacée tels que l'hyperséborrhée de l'acné ou la séborrhée simple,
11) dans le traitement ou la prévention des états cancéreux ou précancéreux, plus particulièrement les leucémies promyélocytaires;
12) dans le traitement d'affections inflammatoires telles que l'arthrite,
13) dans le traitement de toute affection d'origine virale au niveau cutané, telle que le syndrome de Kaposis, ou général,
14) dans la prévention ou le traitement de l'alopécie,
15) dans le traitement d'affections dermatologiques ou générales à composante immunologique,
16) dans le traitement d'affections du système cardiovasculaire telles que l'artériosclérose,l'hypertension, le diabète non-insulino dépendant, ainsi que l'obésité,
17) dans le traitement de désordres cutanés dus à une exposition aux rayonnements U.V..

Dans les domaines thérapeutiques mentionnés ci-dessus, les composés selon l'invention peuvent être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques. Par vitamines D ou leurs dérivés, on entend par exemple les dérivés de la vitamine D₂ ou D₃ et en particulier la 1,25-dihydroxyvitamine D₃. Par anti-radicaux libres, on entend par exemple l'α-tocophérol, la Super Oxyde Dismutate, l'Ubiquinol ou certains chélatants de métaux. Par α-hydroxy ou α-céto acides ou leurs dérivés, on entend par exemple les acides lactique, malique, citrique, glycolique, mandélique, tartrique, glycérique ou ascorbique ou leurs sels, amides ou esters. Enfin, par bloqueurs de canaux ioniques, on entend par exemple le Minoxidil (2,4-diamino-6-pipéridino-pyrimidine-3-oxyde) et ses dérivés.

La présente invention a également pour objet des compositions médicamenteuses contenant au moins un composé de formule (I) telle que définie ci-dessus, l'un de ses isomères optiques ou géométriques ou un de ses sels.

La présente invention a donc ainsi pour objet une nouvelle composition médicamenteuse destinée notamment au traitement des affections susmentionnées, et qui est caractérisée par le fait qu'elle comprend, dans un support pharmaceutiquement acceptable et compatible avec le mode d'administration retenu pour cette dernière, au moins un composé de formule (I), l'un de ses isomères optiques ou géométriques ou un de ses sels.

L'administration des composés selon l'invention peut être effectuée par voie entérale, parentérale, topique ou oculaire.

Par voie entérale, les médicaments peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, les compositions peuvent se présenter sous forme de solutions ou de suspensions pour perfusion ou pour injection.

Les composés selon l'invention sont généralement administrés à une dose journalière d'environ 0,01 mg/kg à 100 mg/Kg en poids corporel, et ceci à raison de 1 à 3 prises.

Par voie topique, les compositions pharmaceutiques à base de composés selon l'invention sont plus particulièrement destinées au traitement de la peau et des muqueuses et peuvent alors se présenter sous forme d'onguents, de crèmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent par ailleurs se présenter soit sous forme anhydre, soit sous une forme aqueuse, selon l'indication clinique.

Par voie oculaire, ce sont principalement des collyres.

Ces compositions à usage topique ou oculaire contiennent au moins un composé de formule (I) telle que définie ci-dessus, ou l'un de ses isomères optiques ou géométriques ou encore l'un de ses sels, à une concentration de préférence comprise entre 0,001% et 5% en poids par rapport au poids total de la composition.

Les composés de formule (I) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photo-induit ou chronologique.

Dans le domaine cosmétique, les composés selon l'invention peuvent par ailleurs être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides où leurs dérivés, ou bien encore avec des bloqueurs de canaux ioniques, tous ces différents produits étant tels que définis ci-avant.

La présente invention vise donc également une composition cosmétique qui est caractérisée par le fait qu'elle comprend, dans un support cosmétiquement acceptable et convenant à une application topique, au moins un composé de formule (I) telle que définie ci-dessus ou l'un de ses isomères optiques ou géométriques ou l'un de ses sels, cette composition cosmétique pouvant notamment se présenter sous la forme d'une crème, d'un lait, d'une lotion, d'un gel, de microsphères ou nanosphères ou vésicules lipidiques ou polymériques, d'un savon ou d'un shampooing.

La concentration en composé de formule (I) dans les compositions cosmétiques selon l'invention est avantageusement comprise entre 0,001 % et 3% en poids par rapport à l'ensemble de la composition.

Les compositions médicamenteuses et cosmétiques selon l'invention peuvent en outre contenir des additifs inertes ou même pharmacodynamiquement ou cosmétiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des antibiotiques comme l'érythromycine et ses esters, la néomycine, la clindamycine et ses esters, les tétracyclines; des agents antifongiques tels que le kétokonazole ou les polyméthylène-4,5 isothiazolidones-3; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (5,4-diphényl-imidazolidine 2,4-dione); des agents anti-inflammatoires non stéroïdiens; des caroténoïdes et, notamment, le β-carotène; des agents anti-psoriatiques tels que l'anthraline et ses dérivés; et enfin les acides eicosa-5,8,11,14-tétraynoïque et eicosa-5,8,11-trynoïque, leurs esters et amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.

On va maintenant donner, à titre d'illustration et sans aucun caractère limitatif, plusieurs exemples d'obtention de composés actifs de formule (I) selon l'invention, ainsi que diverses formulations concrètes à base de tels composés.

### A. EXEMPLES DE COMPOSES

### EXEMPLE 1

### Acide 4-(4-biphényl-2-yl-but-3-èn-(E)-1-ynyl)-benzoïque.

### (a) 4-triméthylsilanyléthynyl-benzoate d'éthyle.

Dans un tricol et sous courant d'azote, on introduit 21,50 g (100,0 mmoles) de 4-bromobenzoate de méthyle, 300 ml de triéthylamine et un mélange composé de 200 mg d'acétate de palladium et de 400 mg de triphénylphosphine. On ajoute ensuite 20,00 g (204 mmoles) de triméthylsilylacétylène, chauffe progressivement 90°C pendant une heure et maintient cette température pendant cinq heures. On refroidit le milieu réactionnel, filtre le sel et évapore. On reprend le résidu avec 200 ml d'acide chlorhydrique (5%) et 400 ml d'éther éthylique. On décante la phase éthérée, lave à l'eau, sèche sur sulfate de magnésium, évapore. Le résidu obtenu est purifié par chromatographie sur colonne de silice, élué par du dichlorométhane. Après évaporation des solvants, on recueille 23,00 g (100%) du composé attendu, sous la forme d'une huile incolore.
¹H NMR (CDCl₃) δ 0,36 (s, 9H), 1,20 (t, 3H, *J* = 7,1 Hz), 4,19 (q, 2H, *J* = 7,1 Hz), 7,60 (dd, 2H, *J* = 6,8 / 1,5 Hz), 8,06 (dd, 2H, *J* = 6,6 / 1,6 Hz).

### (b) 4-éthynyl-benzoate de méthyle.

Dans un ballon, on introduit 45,90 g (197 mmoles) de 4-triméthylsilyléthynyl benzoate d'éthyle, 300 ml de méthanol, et ajoute 500 mg de carbonate de potassium. On agite à température ambiante pendant douze heures et évapore à sec le milieu réactionnel. Le résidu obtenu est purifié par chromatographie sur colonne de silice, élué par du dichlorométhane. Après évaporation des solvants, on recueille 32,00 g (100%) du composé attendu, sous la forme d'un solide jaune clair de point de fusion 86-88°C.
¹H NMR (CDCl₃) δ 3,23 (s, 1H), 3,92 (s, 3H), 7,55 (d, 2H, *J* = 8,3 Hz), 7,99 (d, 2H, *J* = 8,2 Hz).

### (c) biphényl-2-carboxaldéhyde.

Dans un tricol et sous courant d'azote, on introduit 11,43 g (49 mmoles) de 2-bromobiphényle et 50 ml de THF. On ajoute goutte à goutte à -78°C, 21,6 ml (54 mmoles) d'une solution de n.butyllithium (2,5M dans l'hexane) et agite une heure à cette même température. On ajoute ensuite goutte à goutte 4,17 ml (53,9 mmoles) de DMF et laisse remonter à la température ambiante. On acidifie le milieu réactionnel avec de l'acide chlorhydrique (1N), extrait avec de l'éther éthylique, décante la phase organique, sèche sur sulfate de magnésium, évapore. Le résidu obtenu est purifié par chromatographie sur colonné de silice élué par un mélange composé de 60% d'hexane et de 40% de dichlorométhane. On recueille 6,50 g (73%) du composé attendu, sous la forme d'une huile jaune.
¹H NMR (CDCl₃) δ 7,36 à 7,67 (m, 7H), 7,62 (dd, 1H, *J* = 7,5 / 1,4 Hz), 8,03 (dd, 1H, *J* = 7,7 / 1,2 Hz).

### (d) 2-((Z)/(E)-2-iodovinyl)-biphényle.

Dans un tricol et sous argon, on introduit 11,90 g (97 mmoles) de chlorure de chrome(II) et 195 ml de THF sec. On refroidit à 0°C et ajoute goutte à goutte, en maintenant à cette température, une solution composée de 3,54 g (19,4 mmoles) de biphényl-2-carboxaldéhyde, 15,30 g (38,8 mmoles) d'iodoforme et 97 ml de THF sec. On agite à 0°C pendant trois heures, verse le milieu réactionnel dans l'eau, extrait par de l'éther éthylique, décante la phase organique, sèche sur sulfate de magnésium, évapore. Le résidu obtenu est purifié par chromatographie sur colonne de silice, élué par de l'heptane. On recueille 4,70 g (79%) du composé attendu sous la forme d'une huile légèrement jaune, sous la forme d'un mélange composé de 70% de l'isomère (E) et de 30% de l'isomère (Z) (détermination par RMN).
¹H NMR (CDCl₃) (Isomère (E)): δ 6,74 (d, 1H, *J* = 14,8 Hz), 7,25 à 7,49 (m, 10H).

### (e) 4-(4-biphényl-2-yl-but-3-èn-(E)-1-ynyl)-benzoate de méthyle.

Dans un ballon, on introduit successivement 4,22 g (13,8 mmoles) de 2-(2-iodovinyl)biphényle, 2,21 g (13,8 mmoles) de 4-éthynylbenzoate de méthyle, 131 mg de Cul, 362 mg (1,38 mmole) de triphénylphosphine, 2,86 g (20,7 mmoles) de carbonate de potassium et 30 ml de DMF. On chauffe à 120°C pendant douze heures, refroidit, verse le milieu réactionnel dans l'eau, extrait par de l'acétate d'éthyle, décante la phase organique, sèche sur sulfate de magnésium, évapore. Le résidu obtenu est purifié par chromatographie sur colonne de silice, élué par un mélange composé de 70% d'heptane et de 30% de dichlorométhane. Après évaporation des solvants, on recueille 2,25 g (48%) du composé attendu, sous la forme d'une poudre blanche de point de fusion 97-100°C.
¹H NMR (CDCl₃) δ 3,91 (s, 3H), 6,35 (d, 1H, *J* = 16,2 Hz), 7,09 (d, 1H, *J* = 16,2 Hz), 7,30 à 7,49 (m, 10H), 7,63 à 7,68 (m, 1H), 7,97 (dd, 2H, *J* = 6,7 / 1,7 Hz).

### (f) acide 4-(4-biphényl-2-yl-but-3-èn-(E)-1-ynyl)-benzoïque.

Dans un ballon, on introduit 1,99 g (5,9 mmoles) de l'ester obtenu à l'exemple 1(e), 40 ml de méthanol et 40 ml de THF. On ajoute 11,8 ml (59 mmoles) d'une solution de soude méthanolique (5N) et chauffe à reflux pendant une heure. On évapore à sec, reprend le résidu par un mélange d'éther éthylique et d'acide chlorhydrique (4N), décante la phase organique, sèche sur sulfate de magnésium, évapore. On triture le résidu obtenu dans un mélange composé de 20% d'éther éthylique et de 80% d'heptane, filtre, sèche. On recueille 1,70 g (90%) d'acide 4-(4-biphényl-2-yl-but-3-èn-(E)-1-ynyl)-benzoïque, sous la forme d'une poudre blanche de point de fusion 228-229°C.
¹H NMR (CDCl₃) δ 6,36 (d, 1H, *J* = 16,2 Hz), 7,07 (d, 1H, *J* = 16,2 Hz), 7,29 à 7,48 (m, 10H), 7,65 à 7,68 (m, 1H), 7,98 (d, 2H, *J* = 8,4 Hz).

### EXEMPLE 2

### Acide 4-[4-(4'-méthyl-biphényl-2-yl)-but-3-èn(E)-1-ynyl]-benzoïque.

### (a) acide 4-méthyl-phényl boronique.

Dans un tricol et sous courant d'azote, on introduit 17,1 g (0,1 mole) de 4-bromotoluène et 70 ml de THF. On ajoute goutte à goutte à -78°C, 48 ml (120 mmoles) d'une solution de n.butyllithium (2,5M dans l'hexane) et agite une heure à cette même température. On ajoute ensuite goutte à goutte 34,6 ml (150 mmoles) de borate de triisopropyle, laisse remonter à température ambiante, et agite pendant seize heures. On ajoute lentement 450 ml d'acide chlorhydrique (1N), et agite une heure à la température ambiante. On extrait le milieu réactionnel par du dichlorométhane, décante la phase organique, sèche sur sulfate de magnésium, évapore. On recueile 11,10 g (81%) du composé attendu, sous la forme d'un solide blanc cassé de point de fusion 250-252°C. ¹H NMR (CDCl₃) δ 2,42 (s, 3H), 7,29 (d, 2H, *J* = 7,6 Hz), 8,10 (d, 2H, *J* = 7,9 Hz).

### (b) 2-bromo-4'-méthyl-biphényle.

Dans un tricol, on introduit 5,34 g (39,3 mmoles) d'acide 4-méthylphényl boronique, 10,0 g (35,3 mmoles) de 1-bromo-2-iodobenzène, 100 ml de toluène et 42 ml d'une solution aqueuse de carbonate de potassium (2M). On dégaze le milieu réactionnel par barbotage d'argon et ajoute 1,23 g (1,06 mmole) de tétrakistriphénylphosphinepalladium(0) et chauffe à 90°C pendant vingt heures. On verse le milieu réactionnel dans l'eau, extrait par de l'acétate d'éthyle, décante la phase organique, sèche sur sulfate de magnésium, évapore. Le résidu obtenu est purifié par chromatographie sur colonne de silice, élué par de l'hexane. Après évaporation des solvants, on recueille 6,23 g (71%) du composé attendu, sous la forme d'une huile incolore.
¹H NMR (CDCl₃) δ 2,40 (s, 3H), 7,13 à 7,36 (m, 7H), 7,64 (d, 1H, *J* = 7,9 Hz).

### (c) 4'-méthyl-biphényl-2-carboxaldéhyde.

De manière analogue à l'exemple 1(c), à partir de 5,90 g (23,9 mmoles) de 4'-méthyl-2-bromobiphényle, on obtient 3,92 g (84%) du composé attendu, sous la forme d'une huile incolore.
¹H NMR (CDCl₃) δ 2,43 (s, 3H), 7,28 (s, 4H), 7,42 à 7,50 (m, 2H), 7,61 (dd, 1H, *J* = 7,5 / 1,4 Hz), 8,02 (dd, 1H, *J =* 7,6 / 1,2 Hz), 9,99 (s, 1H).

### (d) 2-((E)-2-iodovinyl)-4'-méthyl-biphényle.

De manière analogue à l'exemple 1(d), à partir de 3,92 g (20,0 mmoles) de 4'-méthyl-biphényl-2-carboxaldéhyde, on obtient 4,98 g (78%) du composé attendu, sous la forme d'une huile jaune.
¹H NMR (CDCl₃): δ 2,42 (s, 3H), 6,73 (d, 1H, *J* = 14,8 Hz), 7,22 à 7,74 (m, 10H).

### (e) 4-[4-(4'-méthyl-biphényl-2-yl)-but-3-èn-(E)-1-ynyl]-benzoate de méthyle.

De manière analogue à l'exemple 1(e), par réaction de 4,12 g (12,9 mmoles) de 2-(2-iodovinyl)-4'-méthylbiphényle avec 2,06 g (12,9 mmoles) de 4-éthynyl benzoate de méthyle, on obtient 1,67 g (37%) du composé attendu, sous la forme d'un solide blanc de point de fusion 117-118°C.
¹H NMR (CDCl₃) δ 2,43 (s, 3H), 3,91 (s, 3H), 6,34 (d, 1H, *J* = 16,2 Hz), 7,11 (d, 1H, *J* = 16,2 Hz), 7,26 à 7,37 (m, 7H), 7,47 (d, 2H, *J* = 8,4 Hz), 7,63 à 7,66 (m, 1H), 7,97 (d, 2H, *J* = 8,4 Hz).

### (f) acide 4-[4-(4'-méthyl-biphényl-2-yl)-but-3-èn-(E)-1-ynyl]-benzoïque.

De manière analogue à l'exemple 1(f), à partir de 1,46 g (4,14 mmoles) de l'ester méthylique obtenu à l'exemple 2(e), on obtient 1,31 g (93%) d'acide 4-[4-(4'-méthyl-biphényl-2-yl)-but-3-èn-(E)-1-ynyl]-benzoïque, sous la forme d'un solide blanc de point de fusion 241-242°C.
¹H NMR(CDCl₃) δ 2,36 (s, 3H), 6,29 (d, 1H, *J* = 16,2 Hz), 7,02 (d, 1H, *J* = 16,2 Hz), 7,15 à 7,31 (m, 7H), 7,39 (d, 2H, *J* = 8,4 Hz), 7,57 à 7,61 (m, 1H), 7,91 d, 2H, *J* = 8,4 Hz).

### EXEMPLE 3

### Acide 4-[3-(4'-méthyl-biphényl-2-yl)-acryloylamino]-benzoïque.

### (a) 3-(4'-méthyl-biphényl-2-yl)-(E)-acrylate d'éthyle.

Dans un tricol et sous courant d'azote, on introduit 20,75 g (92,0 mmoles) de diéthylphosphonoacétate d'éthyle et 40 ml de THF. On refroidit à 0°C, ajoute 3,45 g (115,0 mmoles) d'hydrure de sodium à 80% par petites fractions, et agite le milieu réactionnel pendant quinze minutes à 0°C. On ajoute goutte à goutte une solution de 15,00 g (76,4 mmoles) de l'aldéhyde obtenu à l'exemple 2(c), en solution dans 40 ml de THF. On agite le milieu réactionnel pendant dix minutes à la température ambiante, acidifie à pH 1 à l'aide d'acide chlorhydrique 2 N et extrait à l'éther éthylique. La phase organique est lavée à l'eau jusqu'à pH neutre, séchée sur sulfate de magnésium, filtrée, et les solvants évaporés. Le résidu obtenu est purifié par chromatographie sur colonne de silice, élué par un mélange composé de 50% d'acétate d'éthyle et 50% d'heptane. Après évaporation des solvants, on recueille 19,40 g (95%) du composé attendu, sous la forme d'une huile jaune.
¹H NMR (CDCl₃) δ 1,29 (t, 3H, *J* = 7,1 Hz), 2,41 (s, 3H), 4,21 (q, 2H, *J* = 7,2 Hz), 6,39 (d, 1H, *J* = 15,9 Hz), 7,18 à 7,26 (m, 4H), 7,32 à 7,45 (m, 3H), 7,67 à 7,71 (m, 1H), 7,75 (d, 1H, *J* = 16,0 Hz).

### (b) acide 3-(4'-méthyl-biphényl-2-yl)-(E)-acrylique.

Dans un tricot et sous courant d'azote, on introduit 19,00 g (71,0 mmoles) de l'ester obtenu à l'exemple 3(a), 70 ml de soude aqueuse 10 N, et 150 ml de THF. On chauffe le milieu réactionnel pendant quatre heures à 67°C, refroidit, acidifie à pH 1 à l'aide d'acide chlorhydrique 2 N et extrait à l'acétate d'éthyle. La phase organique est lavée à l'eau jusqu'à pH neutre, séchée sur sulfate de magnésium, filtrée, et les solvants évaporés. On recueille 16,92 g (99%) du composé attendu, sous la forme d'un solide cristallin blanc cotonneux, de point de fusion 214-216°C.
¹H NMR (CDCl₃) δ 2,41 (s, 3H), 6,38 (d, 1H, *J* = 16,0 Hz), 7,18 à 7,26 (m, 4H), 7,32 à 7,45 (m, 3H), 7,68 à 7,71 (m, 1H), 7,70 (d, 1H, *J* = 15,9 Hz).

### (c) 4-[3-(4'-méthyl-biphényl-2-yl)-(E)-acryloylamino]-benzoate de méthyle.

Dans un tricol et sous courant d'azote, on introduit 17,50 g (73,4 mmoles) de l'acide obtenu à l'exemple 3(b) et 350 ml de dichlorométhane. On coule goutte à goutte 14,9 ml (74,9 mmoles) de dicyclohexylamine et agite la solution obtenue pendant une heure à la température ambiante. Le milieu réactionnel est évaporé à sec, les cristaux obtenus sont triturés dans l'éther éthylique, filtrés et séchés à l'étuve. On recueille 29,67 g (96%) d'une poudre blanche.
20,00 g de ce sel (47,6 mmoles) sont solubilisés dans 400 ml de dichlorométhane. On coule goutte à goutte 3,8 ml (52,0 mmoles) de chlorure de thionyle et agite la solution obtenue pendant une heure à la température ambiante. Le milieu réactionnel est filtré et le filtrat évaporé à sec. Le résidu obtenu est solubilisé dans 200 ml de THF et la solution ainsi obtenue est coulée goutte à goutte sur une solution composée de 7,20 g (47,6 mmoles) de 4-aminobenzoate de méthyle, 7,24 ml (52,0 mmoles) de triéthylamine, et 150 ml de THF. Le milieu réactionnel est agité pendant une heure et quinze minutes à la température ambiante, acidifié à pH 1 à l'aide d'acide chlorhydrique 2 N et extrait à l'éther éthylique. La phase organique est lavée à l'eau jusqu'à pH neutre, séchée sur sulfate de magnésium, filtrée, et les solvants évaporés. Le résidu obtenu est purifié par chromatographie sur colonne de silice, élué par un mélange composé de 30% d'acétate d'éthyle et 70% d'heptane. Après évaporation des solvants, on recueille 6,00 g (33%) du composé attendu, sous la forme d'un solide rosé de point de fusion 207°C.
¹H NMR (CDCl₃) δ 2,38 (s, 3H), 3,88 (s, 3H), 6,70 (d, 1H, *J* = 15,5 Hz), 7,18 à 7,25 (m, 4H), 7,31 à 7,44 (m, 3H), 7,68 (d, 1H, *J* = 8,0 Hz), 7,78 (d, 2H, *J* = 8 8 Hz), 7,80 (d, 1H, *J* = 15,7 Hz), 7,97 (d, 2H, *J* = 8,8 Hz), 9,48 (s, 1H).

### (d) acide 4-[3-(4'-méthyl-biphényl-2-yl )-(E)-acryloylamino]-benzoïque.

Dans un ballon de 250 ml et sous courant d'azote, on introduit 2,10 g (5,6 mmoles) du composé obtenu à l'exemple 3(d) et 100 ml de THF. On coule rapidement 5,6 ml (56,0 mmoles) de soude aqueuse 10 N, et chauffe le milieu réactionnel pendant six heures à reflux suivi de seize heures à la température ambiante. Le milieu réactionnel est refroidi, acidifié à pH 1 à l'aide d'acide chlorhydrique 2N et extrait à l'éther éthylique. La phase organique est lavée à l'eau jusqu'à pH neutre, séchée sur sulfate de magnésium, filtrée, et les solvants évaporés. On recueille 1,82 g (90%) d'acide 4-[3-(4'-méthyl-biphényl-2-yl)-(E)-acryloylamino]-benzoïque, sous la forme d'une poudre cristalline blanche de point de fusion 265°C.
¹H NMR (CDCl₃) δ 2,39 (s, 3H), 6,78 (d, 1H, *J* = 15,6 Hz), 7,18 à 7,25 (m, 4H), 7,32 à 7,44 (m, 3H), 7,70 (d, 1H, *J* = 15,7 Hz), 7,70 à 7,74 (m, 1H), 7,77 (d, 2H, *J* = 8,7 Hz), 7,93 (d, 2H, *J* = 8,7 Hz), 10,14 (s, 1H).

### EXEMPLE 4

### Acide 4-[3-(4'-méthyl-biphényl-2-yl)-(E)-thioacryloylamino]-benzoïque.

### (a) 4-[3-(4'-méthyl-biphényl-2-yl)-(E)-thioacryloylamino]-benzoate de méthyle.

Dans un ballon de 250 ml et sous courant d'azote, on introduit 2,50 g (6,7 mmoles) du composé obtenu à l'exemple 3(d), 1,36 g (3,36 mmoles) de réactif de Lawesson, et 60 ml de toluène. Le milieu réactionnel est chauffé à reflux pendant une heure, refroidi et évaporé à sec. Le résidu obtenu est trituré dans un mélange composé de 50% de dichlorométhane et 50% d'heptane, filtré et séché. On recueille 1,92 g (73%) du composé attendu, sous la forme d'une poudre orange vif de point de fusion 183°C.
¹H NMR (CDCl₃) δ 2,42 (s, 3H), 3,91 (s, 3H), 7,17 à 7,26 (m, 5H), 7,37 à 7,49 (m, 3H), 7,71 (d, 1H, *J* = 7,2 Hz), 7,95 à 8,04 (m, 6H), 11,28 (s, 1H).

### (b) acide 4-[3-(4'-méthyl-biphényl-2-yl)-(E)-thioacryloylamino]-benzoïque.

De manière analogue à l'exemple 1(f), à partir de 1,50 g (3,9 mmoles) du composé obtenu à l'exemple 4(a), on obtient 1,25 g (86%) d'acide 4-[3-(4'-méthyl-biphényl-2-yl)-thioacryloylamino]-(E)-benzoïque, sous la forme d'une poudre orange de point de fusion 220°C.
¹H NMR (DMSO D₆) δ 2.39 (s, 3H), 7,23 à 7,33 (m, 4H), 7,39 à 7,42 (m, 2H), 7,48 à 7,51 (m, 2H), 7,80 à 7,83 (m, 1H), 7,88 (d, 1H, *J* = 15,1 Hz), 8,00 (d, 2H, *J* = 8,5 Hz), 8,12 (m, 2H), 11,91 (s, 1H), 12,99 (s, 1H).

### EXEMPLE 5

### Acide 4-[3-(4'-méthyl-biphényl-2-yl)-acryloyloxy]-benzoïque.

### (a) 4-[3-(4'-méthyl-biphényl-2-yl)-(E)-acryloyloxy]-benzoate d'allyle

Dans un ballon de un litre et sous courant d'azote, on introduit 9,30 g (22,0 mmoles) du sel de dicyclohexylamine obtenu dans la première partie de l'exemple 3(c) et 100 ml de dichlorométhane. On coule goutte à goutte 1,77 ml (24 0 mmoles) de chlorure de thionyle et agite la solution obtenue pendant une heure et trente minutes à la température ambiante. Le milieu réactionnel est filtré et le filtrat évaporé à sec. Le résidu est solubilisé dans 200 ml de THF et la solution ainsi obtenue est coulée goutte à goutte sur une solution composée de 7,20 g (47,6 mmoles) de 4-hydroxybenzoate d'allyle, 3,34 ml (24,0 mmoles) de triéthylamine et 130 ml de THF. Le milieu réactionnel est agité pendant 24 heures à la température ambiante, acidifié à pH 1 à l'aide d'acide chlorhydrique 2 N et extrait à l'éther éthylique. La phase organique est lavée à l'eau jusqu'à pH neutre, séchée sur sulfate de magnésium, filtrée, et les solvants évaporés. Le résidu obtenu est purifié par chromatographie sur colonne de silice, élué par un mélange composé de 15% d'acétate d'éthyle et 85% d'heptane. Après évaporation des solvants, on recueille 4,95 g (55%) du composé attendu, sous la forme d'une poudre blanche de point de fusion 68°C.
¹H NMR (CDCl₃) δ 2,41 (s, 3H), 4,81 à 4,84 (m, 2H), 5,29 (dd, 1H, *J* = 10,4 / 1,3 Hz), 5,41 (dd, 1H, *J* = 17,2 /1,5 Hz), 5,96 à 6,12 (m, 1H), 6,59 (d, 1H, *J* = 15,9 Hz), 7,21 à 7,28 (m, 6H), 7,39 à 7,51 (m, 3H), 7,77 (d, 1H, *J* = 7,2 Hz), 7,95 (d, 1H, *J* = 15,9 Hz), 8,10 (d, 2H, *J* = 8,7 Hz).

### (b) acide 4-[3-(4'-méthyl-biphényl-2-yl)-(E)-acryloyloxy]-benzoïque.

Dans un tricot et sous courant d'azote, on introduit 843 mg (5,25 mmoles) de malonate de diéthyle, 10 ml de THF, et ajoute 170 mg (5,65 mmoles) d'hydrure de sodium à 80%, par petites fractions. Le milieu réactionnel est agité à la température ambiante pendant vingt minutes, puis transvasé dans une ampoule de coulée. Cette solution est additionnée lentement sur un mélange composé de 2,00 g (5,0 mmoles) du composé obtenu à l'exemple 3(a), 290 mg de tétrakistriphénylphosphinepalladium(0) et de 75 ml de THF. Le milieu réactionnel est agité pendant vingt minutes à la température ambiante, acidifié à pH 1 à l'aide d'acide chlorhydrique 2 N et extrait à l'éther éthylique. La phase organique est lavée à l'eau jusqu'à pH neutre, séchée sur sulfate de magnésium, filtrée, et les solvants évaporés. On recueille, après trituration dans le minimum d'acétate d'éthyle, 1,50 g (83%) d'acide 4-[3-(4'-méthyl-biphényl-2-yl)-(E)-acryloyloxy]-benzoïque, sous la forme de paillettes beige clair, de point de fusion 234-236°C.
¹H NMR (DMSO D₆) à 2.37 (s, 3H), 6,90 (d, 1H, *J* = 15,9 Hz), 7,23 a 7,33 (m, 6H), 7,39 à 7,59 (m, 3H), 7,77 (d, 1H, *J* = 15,9 Hz), 7,99 à 8,07 (m, 3H), 13,09 (s, 1H).

### EXEMPLE 6

### Acide 4-[4-(4'-méthyl-biphényl-2-yl)-buta-1(E),3(Z)-diènyl]-benzoïque.

### (a) (Z)/(E) 3-(4'-méthyl-biphényl-2-yl)-acrylonitrile.

Dans un ballon de 250 ml et sous courant d'azote, on introduit 5,40 g (30,5 mmoles) de cyanométhylphosphonate de diéthyle et 20 ml de THF. La solution obtenue est refroidie à 0°C, puis on ajoute 1,15 g (38,0 mmoles) d'hydrure de sodium à 80%, par petites fractions. Le milieu réactionnel est agité pendant dix minutes à 0°C, puis on coule goutte à goutte une solution composée de 5,00 g (25,0 mmoles) de l'aldéhyde obtenu à l'exemple 2(c), en solution dans 20 ml de THF. On agite le milieu réactionnel pendant quinze minutes à la température ambiante, acidifie à pH 1 à l'aide d'acide chlorhydrique 2N et extrait à l'éther éthylique. La phase organique est lavée à l'eau jusqu'à pH neutre, séchée sur sulfate de magnésium, filtrée, et les solvants évaporés. Le résidu obtenu est purifié par chromatographie sur colonne de silice, élué par un mélange composé de 2% d'acétate d'éthyle et 98% d'heptane. Après évaporation des solvants, on recueille 1,40 g (25%) de l'isomère (Z), sous la forme de cristaux légèrement jaunes de point de fusion 80-82°C, et 2,87 g (52%) de l'isomère (E), sous la forme de cristaux blancs de point de fusion 95-97°C.
¹H NMR (CDCl₃) (Isomère (E)): δ 2,43 (s, 3H), 5,83 (d, 1H, *J* = 16,7 Hz), 7,16 (d, 2H, *J* = 8,1 Hz), 7,27 (d, 2H, *J* = 7,1 Hz), 7,35 à 7,50 (m, 4H), 7,59 (d, 1H, *J* = 7,6 Hz).
¹H NMR (CDCl₃) (Isomère (Z)): δ 2,41 (s, 3H), 5,41 (d, 1H, *J* = 12,0 Hz), 7,12 (d, 1H, *J* = 12,1 Hz), 7,07 à 7,26 (m, 4H), 7,37 à 7,52 (m, 3H), 8,14 (d, 1H, *J* = 6,7 Hz).

### (b) 3-(4'-méthyl-biphényl-2-yl)-propèn-(E)/(Z)-al.

Dans un tricol de 100 ml et sous courant d'azote, on introduit 1,35 g (6,1 mmoles) de l'isomère (Z) obtenu à l'exemple 6(a) et 15 ml de toluène. La solution obtenue est refroidie à -78°C, et on coule goutte à goutte 8,0 ml (8,0 mmoles d'une solution (1M dans le toluène) d'hydrure de diisobutylaluminium. Le milieu réactionnel est agité pendant une heure à -78°C, hydrolysé à l'aide d'une solution saturée de tartrate double de sodium et de potassium, et filtré. La phase organique est lavée à l'eau jusqu'à pH neutre, séchée sur sulfate de magnésium, filtrée, et les solvants évaporés. Le résidu obtenu est purifié par chromatographie sur colonne de silice, élué par un mélange composé de 5% d'acétate d'éthyle et 95% d'heptane. Après évaporation des solvants, on recueille 1,00 g (74%) du composé attendu, contenant 16% de l'isomère (E).
¹H NMR (CDCl₃) (Isomère (Z)): δ 2,41 (s, 3H), 6,15 (dd, 1H, *J* = 11,5 / 8,2 Hz), 7,20 à 7,26 (m, 4H), 7,36 à 7,60 (m, 5H), 10,05 (d, 1H, *J* = 8,2 Hz).

### (c) 4-[4-(4'-méthyl-biphényl-2-yl)-buta-1(E),3(Z)-diènyl]-benzoate d'éthyle.

Dans un tricol de 100 ml, et sous courant d'azote, on introduit 2,01 g (6,7 mmoles) de 4-(diéthylphosphonométhyl)-benzoate d'éthyle et 10 ml de THF. On ajoute 270 mg (9,0 mmoles) d'hydrure de sodium à 80% par petites fractions, et agite le milieu réactionnel pendant dix minutes à la température ambiante. On ajoute goutte à goutte une solution composée de 1,00 g (4,5 mmoles) de l'aldéhyde obtenu à l'exemple 6(b), en solution dans 15 ml de THF. On agite le milieu réactionnel pendant vingt minutes à la température ambiante, ajoute 4 ml de DMPU et poursuit l'agitation pendant quinze minutes supplémentaires. Le milieu réactionnel est acidifié à pH 1 à l'aide d'acide chlorhydrique 2 N et extrait à l'éther éthylique. La phase organique est lavée à l'eau jusqu'à pH neutre, séchée sur sulfate de magnésium, filtrée, et les solvants évaporés. Le résidu obtenu est purifié par chromatographie sur colonne de silice, élué par un mélange composé de 1% d'acétate d'éthyle et 99% d'heptane. Après évaporation des solvants, on recueille 440 mg (26%) du composé attendu, sous la forme d'une huile incolore.
¹H NMR (CDCl₃) δ 1,39 (t, 3H, *J* = 7,1 Hz), 2,39 (s, 3H), 4,37 (q, 2H, *J* = 7,1 Hz), 6,34 à 6,46 (m, 2H), 6,70 (d, 1H, *J* = 15,6 Hz), 7,20 (d, 2H, *J* = 8,0 Hz), 7 29 (d, 2H, *J* = 8,1 Hz), 7,35 à 7,51 (m, 7H), 7,98 (d, 2H, *J* = 8,4 Hz).

### (d) acide 4-[4-(4'-méthyl-biphényl-2-yl)-buta-1(E),3(Z)-diènyl]-benzoïque.

De manière analogue à l'exemple 1(f), à partir de 440 mg (1,2 mmole) du composé obtenu à l'exemple 6(c), on obtient 370 mg (90%) d'acide 4-[4-(4'-méthyl-biphényl-2-yl)-buta-1(E),3(Z)-diènyl]-benzoïque, sous la forme d'une poudre jaune clair de point de fusion 180-182°C.
¹H NMR (DMSO D₆) δ 2,32 (s, 3H), 6,33 à 6,49 (m, 2H), 6,85 (d, 1H, *J* = 15,6 Hz), 7,20 à 7,27 (m, 4H), 7,32 à 7,49 (m, 5H), 7,53 (d, 2H, *J* = 8,3 Hz), 7,89 (d, 2H, *J* = 8,3 Hz).

### EXEMPLE 7

### Acide 4-[4-(4'-méthyl-biphényl-2-yl)-buta-1(E),3(E)-diènyl]-benzoïque.

### (a) (E) 3-(4'-méthyl-biphényl-2-yl)-propènal.

De manière analogue à l'exemple 6(b), à partir de 800 mg (3,65 mmoles) de l'isomère (E) obtenu à l'exemple 6(a), on obtient 420 mg (51%) du composé attendu, sous la forme d'une huile jaune.
¹H NMR (CDCl₃) δ 2,44 (s, 3H), 6,69 (dd, 1H, *J =* 15,9 / 7,8 Hz), 7,20 à 7,29 (m, 4H), 7,37 à 7,52 (m, 3H), 7,56 (d, 1H, *J* = 15,9 Hz), 7,73 (d, 1H, *J* = 7,8 Hz), 9,55 (d, 1H, *J* = 7,8 Hz).

### (b) 4-[4-(4'-méthyl-biphényl-2-yl)-buta-1(E),3(E)-diènyl]-benzoate d'éthyle.

De manière analogue à l'exemple 6(c), par réaction de 700 mg (3,15 mmoles) de l'isomère (E) obtenu à l'exemple 7(a) et de 1,32 g (4,4 mmoles) de 4-(diéthylphosphonométhyl)-benzoate d'éthyle, on obtient 860 mg (74%) du composé attendu, sous la forme d'une poudre jaune de point de fusion 136 °C.
¹H NMR (CDCl₃) δ 1,39 (t, 3H, *J* = 7,1 Hz), 2,44 (s, 3H), 4,37 (q, 2H, *J* = 7,1 Hz), 6,62 à 6,79 (m, 2H), 6,87 à 6,97 (m, 2H), 7,26 à 7,35 (m, 7H), 7,43 (d, 2H, *J* = 8,3 Hz), 7,71 (d, 1H, *J* = 6,0 Hz), 7,97 (d, 2H, *J* = 8,3 Hz).

### (c) acide 4-[4-(4'-méthyl-biphényl-2-yl)-buta-1(E),3(E)-diènyl]-benzoïque.

De manière analogue à l'exemple 1(f), à partir de 860 mg (2,3 mmoles) du composé obtenu à l'exemple 7(b), on obtient 500 mg (64%) d'acide 4-[4-(4'-méthyl-biphényl-2-yl)-buta-1(E),3(E)-diènyl]-benzoïque, sous la forme d'une poudre jaune de point de fusion 244-246°C.
¹H NMR (DMSO D₆) δ 2,37 (s, 3H), 6,69 à 6,80 (m, 2H), 7,04 à 7,41 (m, 9H), 7,59 (d, 2H, *J* = 8,4 Hz), 7,80 (d, 1H, *J* = 6,9 Hz), 7,86 (d, 2H, *J* = 8,3 Hz).

### EXEMPLE 8

### Acide 4-[3-(4'-méthyl-biphényl-2-yl)-propynoyloxy]-benzoïque.

### (a) 2-(2,2-dibromo-vinyl)-4'-méthyl-biphényle.

Dans un tricol de 100 ml, et sous courant d'azote, on introduit 5,35 g (20,4 mmoles) de triphénylphosphine, 3,38 g (10,2 mmoles) de tétrabromure de carbone et 30 ml de dichlorométhane. La solution est agitée pendant cinq minutes à la température ambiante, puis on coule goutte à goutte une solution composée de 1,00 g (5,1 mmoles) de l'aldéhyde obtenu à l'exemple 2(c), en solution dans 10 ml de dichlorométhane. On agite le milieu réactionnel pendant deux heures à la température ambiante, ajoute une solution diluée de carbonate de potassium, de façon à amener le pH vers 9-10 et extrait au dichlorométhane. La phase organique est lavée à l'eau jusqu'à pH neutre, séchée sur sulfate de magnésium, filtrée, et les solvants évaporés. Le residu obtenu est purifié par chromatographie sur colonne de silice, élué par un mélange composé de 3% d'acétate d'éthyle et 97% d'heptane. Après évaporation des solvants, on recueille 1,60 g (88%) du composé attendu, sous la forme d'une huile incolore.
¹H NMR (CDCl₃) δ 2,41 (s, 3H), 7,21 à 7,23 (m, 5H), 7,31 à 7,43 (m, 3H), 7,65 à 7,69 (m, 1H).

### (b) 2-éthynyl-4'-méthyl-biphényle.

Dans un tricol de 500 ml, et sous courant d'azote, on introduit 20,00 g (56,8 mmoles) du composé obtenu à l'exemple 8(a), et 150 ml de THF. La solution obtenue est refroidie à -78°C, et on ajoute goutte à goutte 48 ml (119,0 mmoles) de n.Butyllithium (2,5 N dans l'hexane), en maintenant la température inférieure à -68°C. Le milieu réactionnel est agité pendant quinze minutes à -78°C, puis on ajoute rapidement de l'acide chlorhydrique 2 N, de façon à amener le pH vers 1. Le produit est extrait par de l'éther éthylique, la phase organique lavée à l'eau jusqu'à pH neutre, séchée sur sulfate de magnésium, filtrée, et les solvants évaporés. Le résidu obtenu est purifié par chromatographie sur colonne de silice, élué par de l'heptane. Après évaporation du solvant, on recueille 9,05 g (81%) du composé attendu, sous la forme d'une huile incolore.
¹H NMR (CDCl₃) δ 2,39 (s, 3H), 3,03 (s, 1H), 7,20 à 7,29 (m, 3H), 7,31 à 7,41 (m, 2H), 7,48 (d, 2H, *J* = 8,1 Hz), 7,60 (d, 1H, *J* = 7,5 Hz).

### (c) (4'-méthyl-biphényl-2-yl)-propynoate de méthyle.

Dans un tricol de 250 ml, et sous courant d'azote, on introduit 5,00 g (25,5 mmoles) du composé obtenu à l'exemple 8(b), et 60 ml de THF. La solution obtenue est refroidie à -78°C, et on ajoute goutte à goutte 11,2 ml (29,3 mmoles) de n.Butyllithium (2,5 N dans l'hexane), en maintenant la température inférieure à -70°C. Le milieu réactionnel est agité pendant quinze minutes à -78°C, puis on coule goutte à goutte une solution composée de 2,26 ml (29,3 mmoles) de chloroformiate de méthyle en solution dans 10 ml de THF. On agite une heure et trente minutes à -78°C et ajoute rapidement de l'acide chlorhydrique 2 N, de façon à amener le pH vers 1. Le produit est extrait par de l'éther éthylique, la phase organique lavée à l'eau jusqu'à pH neutre, séchée sur sulfate de magnésium, filtrée, et les solvants évaporés. Le résidu obtenu est purifié par chromatographie sur colonne de silice, élué par de l'heptane. Après évaporation du solvant, on recueille 5,97 g (93%) du composé attendu, sous la forme d'une huile jaune.
¹H NMR (CDCl₃) δ 2,41 (s, 3H), 3,77 (s, 3H), 7,25 à 7,36 (m, 3H), 7,41 à 7,53 (m, 4H), 7,68 (dd, 1H, *J* = 7,7 / 1,0 Hz).

### (d) acide (4'-méthyl-biphényl-2-yl)-propynoïque.

Dans un ballon de 250 ml, on introduit 5,83 g (23,3 mmoles) du composé obtenu à l'exemple 8(c), 10 ml de THF, 5 ml de méthanol, et 11,6 ml (116,5 mmoles) de soude aqueuse 10 N. Le milieu réactionnel est agité pendant une heure et trente minutes à la température ambiante, puis on ajoute rapidement de l'acide chlorhydrique 2 N, de façon à amener le pH vers 1. Le produit est extrait par de l'éther éthylique, la phase organique lavée à l'eau jusqu'à pH neutre, séchée sur sulfate de magnésium, filtrée, et les solvants évaporés. Le résidu obtenu est trituré dans l'heptane, filtré et séché. On recueille 5,35 g (97%) du composé attendu, sous forme d'une poudre beige de point de fusion 114°C.
¹H NMR (CDCl₃) δ 2,37 (s, 3H), 7,29 (d, 2H, *J* = 7,9 Hz), 7,42 à 7 50 (m, 4H), 7,59 (d, 1H, *J* = 7,1 Hz), 7,72 (d, 1H, *J* = 7,4 Hz), 13,68 (s, 1H).

### (e) 4-[3-(4'-méthyl-biphényl-2-yl)-propynoyloxy]-benzoate d'allyle.

Dans un ballon de 250 ml, et sous courant d'azote, on introduit 1,80 g (7,6 mmoles) du composé obtenu à l'exemple 8(d), 1,45 g (8,0 mmoles) de dicyclohexylamine, 20 ml de dichlorométhane et, goutte à goutte, 0,58 ml (8,0 mmoles) de chlorure de thionyle. Le milieu réactionnel est agité pendant cinq minutes à la température ambiante, puis le précipité est filtré, lavé par du dichlorométhane, le filtrat évaporé à sec et repris par 15 ml de THF. La solution ainsi obtenue est coulée goutte à goutte sur un mélange composé de 1,35 g (7,6 mmoles) de 4-hydroxybenzoate d'allyle, 1,17 ml (8,4 mmoles) de triéthylamine, et 20 ml de THF. Après quatre heures d'agitation, on ajoute rapidement de l'acide chlorhydrique 2 N, de façon à amener le pH vers 1. Le produit est extrait par de l'éther éthylique, la phase organique lavée à l'eau jusqu'à pH neutre, séchée sur sulfate de magnésium, filtrée, et les solvants évaporés. Le résidu obtenu est purifié par chromatographie sur colonne de silice, élué par un mélange composé de 99% d'heptane et 1% d'acétate d'éthyle. Après évaporation des solvants, on recueille 1,50 g (50%) du composé attendu, sous la forme d'une huile jaune.
¹H NMR (CDCl₃) δ 2,42 (s, 3H), 4,81 à 4,84 (m, 2H), 5,30 (dd, H, *J* = 10,4 1,3 Hz), 5,41 (dd, 1H, *J* = 17,2 / 1,4 Hz), 5,96 à 6,11 (m, 1H), 7,21 à 7,40 (m, 5H), 7,45 à 7,54 (m, 4H), 7,73 (dd, 1H, *J* = 7,7 / 1,0 Hz), 8,10 (d, 2H, *J* = 11,2 Hz).

### (f) acide 4-[3-(4'-méthyl-biphényl-2-yl)-propynoyloxy]-benzoïque.

Dans un tricol de 100 ml, et sous courant d'azote, on introduit 638 mg (3,97 mmoles) de malonate de diéthyle, 3 ml de THF et 128 mg (4,27 mmoles) d'hydrure de sodium à 80%, par petites fractions. Le milieu réactionnel est agité pendant quinze minutes à la température ambiante, puis la solution obtenue est coulée goutte à goutte sur un mélange composé de 1,50 g (3,78 mmoles) du composé obtenu à l'exemple 8(e), 218 mg (0,19 mmole) de tétrakistriphénylphosphinepalladium(0) et de 10 ml de THF. Le milieu réactionnel est agité pendant trente minutes à la température ambiante, acidifié à pH 1 à l'aide d'acide chlorhydrique 2 N et extrait à l'éther éthylique. La phase organique est lavée à l'eau jusqu'à pH neutre, séchée sur sulfate de magnésium, filtrée, et les solvants évaporés. Le résidu obtenu est purifié par chromatographie sur colonne de silice, élué par un mélange composé de 70% d'heptane et 30% d'acétate d'éthyle. Après évaporation des solvants, on recueille 200 mg (15%) d'acide 4-[3-(4'-méthyl-biphényl-2-yl)-propynoyloxy]-benzoïque, sous la forme d'une poudre beige de point de fusion 178°C.
¹H NMR (DMSO D₆) δ 2,37 (s, 3H), 7,31 (d, 2H, *J* = 8,0 Hz), 7,38 (d, 2H, *J* = 8,7 Hz), 7,47 à 7,56 (m, 4H), 7,65 à 7,71 (m, 1H), 7,82 (d, 1H, *J* = 7,7 Hz), 8,01 (d, 1H, *J* = 8,7 Hz), 13,14 (s, 1H).

### EXEMPLE 9

### Acides 4-[4-(4'-méthyl-biphényl-2-yl)-(E)/(Z)-but-1-èn-3-ynyl]-benzoïques.

### (a) (4'-méthyl-biphényl-2-yl)-propynal.

Dans un tricol de 250 ml, et sous courant d'azote, on introduit 3,83 g (19,5 mmoles) du composé obtenu à l'exemple 8(b), et 40 ml de THF. La solution obtenue est refroidie à -78°C, et on ajoute goutte à goutte 8,6 ml (21,5 mmoles) de n.butyllithium (2,5 N dans l'hexane), en maintenant la température inférieure à -65°C. Le milieu réactionnel est agité pendant vingt minutes à -78°C, puis on coule goutte à goutte 2,26 ml (29,3 mmoles) de N,N-diméthylformamide. On agite deux heures à -78°C, laisse la température remonter à -30°C, et ajoute rapidement de l'acide chlorhydrique 2 N, de façon à amener le pH vers 1. Le produit est extrait par de l'éther éthylique, la phase aqueuse est rendue basique par addition de soude 10 N, et extraite par de l'acétate d'éthyle. Les phases organiques sont réunies, lavées à l'eau jusqu'à pH neutre, séchées sur sulfate de magnésium, filtrées, et les solvants évaporés. Le résidu obtenu est purifié par chromatographie sur colonne de silice, élué par un mélange composé de 95% d'heptane et 5% d'acétate d'éthyle. Après évaporation des solvants, on recueille 3,65 g (83%) du composé attendu, sous la forme d'une huile jaune.
¹H NMR (CDCl₃) δ 2,42 (s, 3H), 7,27 (d, 1H, *J* = 8,2 Hz), 7,32 à 7,56 (m, 6H), 7,70 (dd, 1H, *J =* 7,7 / 1,2 Hz), 9,28 (s, 1H).

### (b) 4-[4-(4'-méthyl-biphényl-2-yl)-but-1-èn-(E)/(Z)-3-ynyl]-benzoate d'éthyle.

De manière analogue à l'exemple 6(c), par réaction de 1,60 g (7,1 mmoles) de l'aldéhyde obtenu à l'exemple 9(a) et de 2,80 g (9,3 mmoles) de 4-(diéthylphosphonométhyl)-benzoate d'éthyle, on obtient 2,12 g (80%) du composé attendu sous la forme d'une huile jaune contenant 78% de l'isomère (E) et 22% de l'isomère (Z).
¹H NMR (CDCl₃) (Isomère (E)): δ 1,41 (t, 3H, *J* = 7,0 Hz), 2,43 (s 3H), 4,37 (q, 2H, *J* = 7,0 Hz), 6,38 (d, 1H, *J* = 16,2 Hz), 6,86 (d, 1H, *J =* 16,2 Hz, 7,20 à 7,62 (m, 10H), 7.99 (d, 2H, *J* = 8,4 Hz).
¹H NMR (CDCl₃) (Isomère (Z)): δ 1,40 (t, 3H, *J* = 7,0 Hz), 2,38 (s 3H), 4,36 (q, 2H, *J* = 7,0 Hz), 5,96 (d, 1H, *J* = 12,0 Hz), 6,60 (d, 1H, *J* = 12,0 Hz), 7,20 à 7,62 (m, 8H), 7,68 (d, 2H, *J* = 8,4 Hz), 7,83 (d, 2H, *J* = 8,4 Hz).

### (c) acides 4-[4-(4'-méthyl-biphényl-2-yl)-(E)/(Z)-but-1-èn-3-ynyl]-benzoïques.

De manière analogue à l'exemple 1(f), à partir de 2,00 g (5,4 mmoles) de produit obtenu à l'exemple 9(b), et après une heure et trente minutes de chauffage, on obtient une poudre jaune, identifiée comme étant un mélange des isomères (Z) et (E). Ce mélange est séparé par chromatographie sur colonne de silice, élué par un gradient de solvant passant de l'heptane pur à un mélange composé de 50% d'heptane et 50% d'acétate d'éthyle. Après évaporation des solvants, on recueille 1,03 g (55%) d'acide 4-[4-(4'-méthyl-biphényl-2-yl)-(E)-but-1-èn-3-ynyl]-benzoïque de point de fusion 225°C, et 0,30 g (16%) d'acide 4-[4-(4'-méthyl-biphényl-2-yl)-(Z)-but-1-èn-3-ynyl]-benzoïque de point de fusion 177-179°C.
¹H NMR (DMSO D₆) (Isomère (E)): δ 2,38 (s, 3H), 6,70 (d, 1H, *J* = 16,3 Hz), 6,99 (d, 1H, *J* = 16,3 Hz), 7,31 (d, 1H, *J* = 8,0 Hz), 7,36 à 7,63 (m, 7H), 7,66 (d 2H, *J* = 8,4 Hz), 7,91 (d, 2H, J = 8,3 Hz), 13,03 (s, 1H).
¹H NMR (DMSO D₆) (Isomère (Z)): δ 2,34 (s, 3H), 6,11 (d, 1H, *J* = 12,0 Hz), 6,79 (d, 1H, *J* = 12,1 Hz), 7,25 (d, 2H, *J* = 7,9 Hz), 7,40 à 7,53 (m, 5H), 7,64 à 7,95 (m, 5H).

### EXEMPLE 10

### Acide 4-[4-(4'-méthyl-biphényl-2-yl)-buta-1,3-diynyl]-benzoïque.

### (a) 2-bromoéthynyl-4'-méthyl-biphényle.

Dans un ballon de 250 ml, et sous courant d'azote, on introduit 1,45 g (7,39 mmoles) du composé obtenu à l'exemple 8(b), 7,35 g (22,2 mmoles) de tétrabromure de carbone et 50 ml de dichlorométhane. La solution obtenue est refroidie à 0°C, puis on ajoute 17,44 g (66,5 mmoles) de triphénylphosphine, et agite quatre heures à la température ambiante. On ajoute de la silice, évapore à sec et purifie le produit par chromatographie sur colonne de silice, élué par de l'heptane. Après évaporation des solvants, on recueille 750 mg (37%) du composé attendu, sous la forme d'une huile jaune.
¹H NMR (CDCl₃) δ 2,41 (s, 3H), 7,23 à 7,32 (m, 3H), 7,35 à 7,42 (m 2H), 7,48 (d, 2H, *J* = 8,1 Hz), 7,56 (d, 1H, *J =* 7,5 Hz).

### (b) 4-[4-(4'-méthyl-biphényl-2-yl)-buta-1,3-diynyl]-benzoate de méthyle.

Dans un tricot de 50 ml, et sous courant d'azote, on introduit 128 mg (1,84 mmole) de chlorhydrate d'hydroxylamine, 45 mg (0,46 mmole) de chlorure cuivreux (CuCI), 1 ml (15 mmoles) d'éthylamine, 20 ml de méthanol et 128 µl d'eau. On ajoute, tout en agitant, 370 mg (2,3 mmoles) du composé obtenu à l'exemple 1(b), et chauffe à 45°C pendant une heure sous vive agitation. On ajoute alors goutte à goutte 750 mg du composé obtenu à l'exemple 10(a) en solution dans 10 ml de méthanol. Le milieu réactionnel est agité pendant 24 heures à 30°C, refroidi et on ajoute rapidement de l'acide chlorhydrique 2 N, de façon à amener le pH vers 1. Le produit est extrait par de l'éther éthylique, la phase organique lavée à l'eau jusqu'à pH neutre, séchée sur sulfate de magnésium, filtrée, et les solvants évaporés. Le résidu obtenu est repris par de l'éther éthylique, on ajoute de la silice et évapore à sec. Le produit est purifié par chromatographie sur colonne de silice, élué par de l'heptane. Après évaporation des solvants, on recueille 380 mg (47%) du composé attendu, sous la forme d'une poudre beige de point de fusion 100-102°C.
¹H NMR (CDCl₃) δ 2,42 (s, 3H), 3,92 (s, 3H), 7,26 à 7,34 (m, 2H), 7,39 à 7,47 (m, 2H), 7,50 à 7,58 (m, 5H), 7,66 (d, 1H, *J* = 7,4 Hz), 7,99 (d, 2H, *J* = 8,5 Hz).

### (c) acide 4-[4-(4'-méthyl-biphényl-2-yl)-buta-1,3-diynyl]-benzoïque.

De manière analogue à l'exemple 1(f), à partir de 340 mg (0,97 mmole) de produit obtenu à l'exemple 10(b), on obtient 300 mg (93%) d'acide 4-[4-(4 -méthyl-biphényl-2-yl)-buta-1,3-diynyl]-benzoïque, sous la forme d'une poudre blanche de point de fusion 217-219°C.
¹H NMR (CDCl₃) δ 2,43 (s, 3H), 7,30 à 7,34 (m, 2H), 7,39 à 7,44 (m, 2H), 7,50 à 7,55 (m, 5H), 7,66 (d, 1H, *J* = 7,4 Hz), 8,00 (d, 2H, *J* = 8,4 Hz).

### EXEMPLE 11

### Acide 4-[4-(3-fluoro-4'-méthyl-biphényl-2-yl)-but-3-èn-(E)-1-ynyl]-benzoïque.

### (a) 2-fluoro-6-iodo-benzoate de méthyle.

Dans un ballon de 100 ml, et sous courant d'azote, on introduit 5,00 g (18,8 mmoles) d'acide 2-fluoro-6-iodo-benzoïque et 30 ml de DMF. On refroidit à 0°C et ajoute par petites fractions 62 mg (20,7 mmoles) d'hydrure de sodium à 80%. Le milieu réactionnel est agité pendant une heure à la température ambiante, puis on ajoute goutte à goutte 1,76 ml (28,2 mmoles) d'iodure de méthyle. Le milieu réactionnel est agité pendant une heure à la température ambiante, puis on ajoute de l'eau et de l'éther éthylique. Le produit est extrait par de l'éther éthylique, la phase organique lavée à l'eau jusqu'à pH neutre, séchée sur sulfate de magnésium, filtrée, et les solvants évaporés. Le résidu obtenu est purifié par chromatographie sur colonne de silice, élué par un mélange composé de 50% d'heptane et 50% de dichlorométhane. Après évaporation des solvants, on recueille 5,08 g (97%) du composé attendu, sous la forme d'un liquide incolore.
¹H NMR (CDCl₃) δ 3,98 (s, 3H), 7,09 à 7,16 (m, 2H), 7,61 à 7,68 (m, 1H).

### (b) 3-fluoro-4'-méthyl-biphényl-2-carboxylate de méthyle.

De manière analogue à l'exemple 2(b), à partir de 3,21 g (23,6 mmoles) d'acide 4-méthylphényl boronique, 5,08 g (18,1 mmoles) de 2-fluoro-6-iodo-benzoate de méthyle obtenu à l'exemple 11(a), 629 mg (0,54 mmole) de tétrakistriphénylphosphine palladium(0) et 23,6 ml d'une solution aqueuse 2M de carbonate de potassium, on obtient 4,14 g (93%) du composé attendu, sous la forme d'une poudre blanche de point de fusion 62-65°C.
¹H NMR (CDCl₃) δ 2,38 (s, 3H), 3,70 (s, 3H), 7,05 à 7,28 (m, 6H), 7,38 à 7,47 (m, 1H).

### (c) (3-fluoro-4'-méthyl-biphényl-2-yl)-méthanol.

De manière analogue à l'exemple 6(b), à partir de 3,34 g (13,7 mmoles) du fluoroester obtenu à l'exemple 11(b), on obtient 2,95 g (100%) du composé attendu, sous la forme d'une poudre blanche de point de fusion 76-78°C.
¹H NMR (CDCl₃) δ 1,82 (dt, 1H, *J* = 6,3 /1,3 Hz), 2,41 (s, 3H), 4,65 (dd, 2H, *J* = 6,3 / 1,7 Hz), 7,04 à 7,12 (m, 2H), 7,23 à 7,37 (m, 5H).

### (d) 3-fluoro-4'-méthyl-biphényl-2-carboxaldéhyde.

Dans un ballon de 500 ml, on mélange 2,16 g (10,0 mmoles) du fluoroalcool obtenu à l'exemple 11(c), 17,39 g d'oxyde de manganèse et 150 ml de dichlorométhane. Le milieu réactionnel est agité pendant vingt heures à la température ambiante, puis on filtre l'oxyde de manganèse et évapore le dichlorométhane. Le résidu obtenu est purifié par chromatographie sur colonne de silice, élué par un mélange composé de 30% d'heptane et 70% de dichlorométhane. Après évaporation des solvants, on recueille 1,47 g (69%) du composé attendu, sous la forme d'un liquide incolore.
¹H NMR (CDCl₃) δ 2,42 (s, 3H), 7,11 à 7,29 (m, 5H), 7,52 à 7,61 (m, 2H), 9,94 (s, 1H).

### (e) 2-((E)-2-iodovinyl)3-fluoro-4'-méthyl-biphényle.

De manière analogue à l'exemple 1(d), à partir de 1,47 g (6,26 mmoles) 3-fluoro-4'-méthyl-biphényl-2-carboxaldéhyde obtenu à l'exemple 11(d), on obtient 770 mg (36%) du composé attendu, sous la forme d'une huile jaune.
¹H NMR (CDCl₃) δ 2,42 (s, 3H), 6,95 (d, 1H, *J* = 15,1 Hz), 7,01 a 7,17 (m, 2H), 7,21 à 7,31 (m, 7H).

### (f) 4-[4-(3-fluoro-4'-méthyl-biphényl-2-yl)-but-3-èn-(E)-1-ynyl]-benzoate de méthyle.

Dans un ballon, on introduit successivement 770 mg (2,28 mmoles) 2-((E)-2-iodovinyl)3-fluoro-4'-méthyl-biphényle et 50 ml de pipéridine. On ajoute goutte à goutte une solution composée de 548 mg (3,4 mmoles) de 4-éthynylbenzoate de méthyle et de 50 ml de pipéridine. On ajoute 132 mg (0,11 mmole) de tétrakistriphénylphosphinepalladium (0) et agite le milieu réactionnel pendant seize heures à la température ambiante. On verse le milieu réactionnel dans l'eau, extrait par de l'acétate d'éthyle, décante la phase organique, sèche sur sulfate de magnésium, évapore. Le résidu obtenu est purifié par chromatographie sur colonne de silice, élué par un mélange composé de 90% d'heptane et de 10% d'acétate d'éthyle. Après évaporation des solvants, on recueille 490 mg (58%) du composé attendu, sous la forme d'une solide cristallisé beige de point de fusion 92°C.
¹H NMR (CDCl₃) δ 2,43 (s, 3H), 3,91 (s, 3H), 6,53 (d, 1H, *J* = 16,6 Hz), 6,85 (d, 1H, *J* = 16,6 Hz), 7,05 à 7,13 (m, 2H), 7,21 à 7,28 (m, 5H), 7,47 (d, 2H, *J* = 3,4 Hz), 7,97 (d, 2H, *J* = 8,4 Hz).

### (g) acide 4-[4-(3-fluoro-4'-méthyl-biphényl-2-yl)-but-3-èn-(E)-1-ynyl]-benzoïque.

De manière analogue à l'exemple 1(f), à partir de 490 mg (1,3 mmole) de l'ester méthylique obtenu à l'exemple 11(f), on obtient 450 mg (95%) d'acide 4-[4-(3-fluoro-4'-méthyl-biphényl-2-yl)-but-3-èn-(E)-1-ynyl]-benzoïque, sous la forme d'une solide cristallisé beige de point de fusion 202°C.
¹H NMR (CDCl₃ + 2 gouttes de DMSO D₆) δ 2,43 (s, 3H), 6,52 (d, 1H, *J* = 16,6 Hz), 6,83 (d, 1H, *J* = 16,6 Hz), 7,05 à 7,13 (m, 2H), 7,21 à 7,31 (m, 5H), 7,46 (d, 2H, *J* = 8,3 Hz), 7,98 (d, 2H, *J* = 8,3 Hz).

### EXEMPLE 12

### Acide 4-[4-(4,4'-diméthyl-biphényl-2-yl)-but-3-èn-1-ynyl]-benzoïque.

### (a) 2-hydroxy-5-méthyl-benzoate de méthyle.

Dans un ballon de un litre, on solubilise 20,60 g (135,4 mmoles) d'acide 2-hydroxy-5-méthyl-benzoïque dans 670 ml de méthanol. On ajoute goutte à goutte 6,7 ml d'acide sulfurique et chauffe le milieu réactionnel à reflux pendant 48 heures. Après refroidissement, le méthanol est évaporé, puis on ajoute de la glace et extrait par de l'éther éthylique. La phase organique est lavée à l'eau jusqu'à pH neutre, séchée sur sulfate de magnésium, filtrée, et les solvants évaporés. Le résidu obtenu est purifié par chromatographie sur colonne de silice, élué par du dichlorométhane. Après évaporation des solvants, on recueille 20,87 g (93%) du composé attendu, sous la forme d'un liquide incolore.
¹H NMR (CDCl₃) δ 2,28 (s, 3H), 3,93 (s, 3H), 6,88 (d, 1H, *J* = 8,5 Hz), 7,26 (dd, 1H, *J* = 8,5 / 2,2 Hz), 7,62 (d, 1H, *J* = 1,8 Hz).

### (b) 5-méthyl-2-trifluorométhanesulfonyloxy-benzoate de méthyle.

Dans un ballon de 500 ml, et sous courant d'azote, on introduit 13,48 g (81,13 mmoles) de 2-hydroxy-5-méthyl-benzoate de méthyle obtenu à l'exemple 12(a), 22,42 g (162,3 mmoles) de carbonate de potassium et 135 ml de DMF. On refroidit à 0°C et ajoute goutte à goutte 22,0 g de 4-nitro-trifluorométhanesulfonyloxybenzène en solution dans 80 ml de DMF. Le milieu réactionnel est agité pendant deux heures et trente minutes à la température ambiante, puis on ajoute de l'eau et de l'éther éthylique. Le produit est extrait par de l'éther éthylique, la phase organique lavée à l'eau jusqu'à pH neutre, séchée sur sulfate de magnésium et filtrée. Après évaporation des solvants, on recueille 23,89 g (99%) du composé attendu, sous la forme d'un liquide incolore.
¹H NMR (CDCl₃) δ 2,43 (s, 3H), 3,96 (s, 3H), 7,18 (d, 1H, *J* = 8,4 Hz), 7,41 (dd, 1H, *J* = 8,4 / 2,3 Hz), 7,89 (d, 1H, *J* = 2,1 Hz).

### (c) 4,4'-diméthyl-biphényl-2-carboxylate de méthyle.

Dans un tricol, on introduit 12,37 g (91,0 mmoles) d'acide 4-méthylphényl boronique, 22,61 g (75,8 mmoles) du triflate obtenu à l'exemple 12(b), 6,43 g (151,6 mmoles) de chlorure de lithium, 91 ml d'une solution aqueuse de carbonate de potassium (2M) et 680 ml de toluène. On dégaze le milieu réactionnel par barbotage d'argon, ajoute 2,63 g (2,27 mmoles) de tétrakistriphénylphosphinepalladium(0) et chauffe à 90°C pendant cinq heures. On verse le milieu réactionnel dans l'eau, extrait par de l'éther éthylique, décante la phase organique, sèche sur sulfate de magnésium, évapore. Le résidu obtenu est purifié par chromatographie sur colonne de silice, élué par un mélange composé de 20% de dichlorométhane et 80% d'heptane. Après évaporation des solvants, on recueille 18,26 g (96%) du composé attendu, sous la forme d'une huile incolore.
¹H NMR (CDCl₃) δ 2,39 (s, 3H), 2,41 (s, 3H), 3,65 (s, 3H), 7,19 à 7,34 (m, 6H), 7,61 (s, 1H).

### (d) (4,4'-diméthyl-biphényl-2-yl)-méthanol.

Dans un tricol de un litre, on introduit 160 ml d'éther éthylique et 2,81 g (74,1 mmoles) d'hydrure double de lithium et d'aluminium. Le milieu réactionnel est refroidi à 0°C, puis on introduit goutte à goutte 17,81 g (74,1 mmoles) de l'ester obtenu à l'exemple 12(c), en solution dans 160 ml d'éther éthylique. Le milieu réactionnel est agité pendant deux heures à 0°C, on ajoute 1,40 g d'hydrure double de lithium et d'aluminium et agite deux heures à la température ambiante. Le milieu est refroidi à 0°C, puis on ajoute goutte à goutte une solution saturée de chlorure de sodium, filtre sur Célite® , ajoute de l'eau et de l'éther éthylique. Le produit est extrait par de l'éther éthylique, la phase organique lavée à l'eau jusqu'à pH neutre, séchée sur sulfate de magnésium, filtrée, et les solvants évaporés. On recueille 15,48 g (98%) du composé attendu, sous la forme d'une huile jaune.
¹H NMR (CDCl₃) δ 1,59 (t, 1H, *J* = 5,9 Hz), 2,39 (s, 3H), 2,40 (s, 3H), 4,59 (d, 2H, *J* = 5,8 Hz), 7,13 à 7,27 (m, 6H), 7,35 (s, 1H).

### (e) 4,4'-diméthyl-biphényl-2-carboxaldéhyde.

De manière analogue à l'exemple 11(d), à partir de 14,80 g (69,7 mmoles) de l'alcool obtenu à l'exemple 12(d), on obtient 14,27 g (97%) du composé attendu, sous la forme d'une poudre blanche de point de fusion 61-67°C.
¹H NMR (CDCl₃) δ 2,43 (s, 3H), 2,45 (s, 3H), 7,18 à 7,26 (m, 4H), 7,33 (d, 1H, *J* = 7,8 Hz), 7,44 (dd, 1H, *J* = 7,9 / 1,6 Hz), 7,82 (s, 1H), 9,97 (s, 1H).

### (f) 2-((E)-2-iodovinyl)-4,4'-diméthyl-biphényle.

De manière analogue à l'exemple 1(d), à partir de 3,00 g (14,3 mmoles) de 4,4'-diméthyl-biphényl-2-carboxaldéhyde obtenu à l'exemple 12(e), on obtient 4,47 g (93%) du composé attendu, sous la forme d'une huile orange.
¹H NMR (CDCl₃) δ 2,40 (s, 3H), 2,43 (s, 3H), 6,72 (d, 1H, *J* = 14,7 Hz), 7,18 à 7,32 (m, 8H).

### (g) 4-[4-(4,4'-diméthyl-biphényl-2-yl)-but-3-èn-(E)-1-ynyl]-benzoate de méthyle.

De manière analogue à l'exemple 1(e), par réaction de 4,00 g (12,0 mmoles) de 2-((E)-2-iodovinyl)-4,4'-diméthyl-biphényle obtenu à l'exemple 12(f) avec 1,74 g (10,9 mmoles) de 4-éthynyl benzoate de méthyle, on obtient 400 mg (10%) du composé attendu, sous la forme de paillettes jaunes de point de fusion 95-97°C.
¹H NMR (CDCl₃) δ 2,41 (s, 3H), 2,42 (s, 3H), 3,91 (s, 3H), 6,34 (d, 1H, *J* = 16,2 Hz), 7,09 (d, 1H, *J* = 16,2 Hz), 7,13 à 7,26 (m, 6H), 7,45 (s, 1H), 7,47 (d 2H, *J* = 8,3 Hz), 7,97 (d, 2H, *J* = 8,4 Hz).

### (h) acide 4-[4-(4,4'-diméthyl-biphényl-2-yl)-but-3-èn-(E)-1-ynyl]-benzoïque.

De manière analogue à l'exemple 1(f), à partir de 360 g (1,0 mmole) de l'ester méthylique obtenu à l'exemple 12(g), on obtient 240 mg (74%) d'acide 4-[4-(4,4'-diméthyl-biphényl-2-yl)-but-3-èn-(E)-1-ynyl]-benzoïque, sous la forme d'une poudre jaune clair de point de fusion 217°C.
¹H NMR (DMSO D₆) δ 2,25 (s, 6H), 6,50 (d, 1H, *J* = 16,2 Hz), 6,83 (d, 1H, *J* = 16,3 Hz), 7,05 à 7,18 (m, 6H), 7,42 (d, 2H, *J* = 8,3 Hz), 7,55 (s, 1H), 7,79 (d, 2H, *J* = 8, 32 Hz).

### EXEMPLE 13

### Acide 4-[4-(5,4'-dimèthyl-biphényl-2-yl)-but-3-èn-1-ynyl]-benzoïque.

### (a) 2-hydroxy-4-méthyl-benzoate de méthyle.

De manière analogue à l'exemple 12(a), à partir de 30,43 g (200,0 mmoles) d'acide 2-hydroxy-4-méthyl-benzoïque, on obtient, après 72 heures de reflux, 29,49 g (89%) du composé attendu, sous la forme d'un liquide incolore qui cristallise lentement, de point de fusion 20-25°C.
¹H NMR (CDCl₃) δ 2,34 (s, 3H), 3,92 (s, 3H), 6,69 (d, 1H, *J* = 8,2 Hz), 6,79 (s, 1H), 7,70 (d, 1H, *J* = 8,1 Hz).

### (b) 4-méthyl-2-trifluorométhanesulfonyloxy-benzoate de méthyle.

De manière analogue à l'exemple 12(b), à partir de 9,97 g (60,0 mmoles) de 2-hydroxy-4-méthyl-benzoate de méthyle obtenu à l'exemple 13(a), on obtient 13,0 g (73%) du composé attendu, sous la forme d'un liquide incolore.
¹H NMR (CDCl₃) δ 2,45 (s, 3H), 3,95 (s, 3H), 7,09 (s, 1H), 7,27 (dd, 1H, *J* = 7,9 / 2,9 Hz), 7,98 (d, 1H, *J* = 8,0 Hz).

### (c) 5,4'-diméthyl-biphényl-2-carboxylate de méthyle.

De manière analogue à l'exemple 12(c), à partir de 7,11 g (52,3 mmoles) d'acide 4-méthylphényl boronique et 13,0 g (43,6 mmoles) du triflate obtenu à l'exemple 13(b), on obtient 10,15 g (97%) du composé attendu, sous la forme de cristaux blancs de point de fusion 86-88°C.
¹H NMR (CDCl₃) δ 2,39 (s, 3H), 2,40 (s, 3H), 3,65 (s, 3H), 7,16 à 7,24 (m, 6H), 7,74 (d, 1H, *J* = 7,7 Hz).

### (d) (5,4'-diméthyl-biphényl-2-yl)-méthanol.

De manière analogue à l'exemple 12(d), à partir de 10,08 g (42,0 mmoles) d'ester obtenu à l'exemple 13(d), on obtient 8,21 g (92%) du composé attendu, sous la forme de cristaux blancs de point de fusion 71-73°C.
¹H NMR (CDCl₃) δ 1,66 (t, 1H, *J* = 5,9 Hz), 2,37 (s, 3H), 2,39 (s, 3H), 4,56 (d, 2H, *J* = 5,9 Hz), 7,09 (s, 1H), 7,15 à 7,27 (m, 5H), 7,39 (d, 1H, *J* = 7,8 Hz).

### (e) 5,4'-diméthyl-biphényl-2-carboxaldéhyde.

De manière analogue à l'exemple 11(d), à partir de 8,17 g (38,5 mmoles) de l'alcool obtenu à l'exemple 13(d), on obtient 7,62 g (94%) du composé attendu, sous la forme d'une huile jaune clair.
¹H NMR (CDCl₃) δ 2,42 (s, 3H), 2,45 (s, 3H), 7,23 à 7,29 (m, 6H), 7,93 (d, 1H, *J* = 7,9 Hz), 9,94 (d, 1H, *J* = 0,7 Hz).

### (f) 2-((E)-2-iodovinyl)-5,4'-diméthyl-biphényle.

De manière analogue à l'exemple 1(d), à partir de 3,00 g 14,3 mmoles) de 5,4'-diméthyl-biphényl-2-carboxaldéhyde obtenu à l'exemple 13(e), on obtient 3,69 g (77%) du composé attendu, sous la forme d'une huile orange qui sera utilisée directement pour l'étape suivante.

### (g) 4-[4-(5,4'-diméthyl-biphényl-2-yl)-but-3-èn-(E)-1-ynyl]-benzoate de méthyle.

De manière analogue à l'exemple 1(e), par réaction de 3,69 g (11,0 mmoles) de 2-((E)-2-iodovinyl)-5,4'-diméthyl-biphényle obtenu à l'exemple 13(f) avec 1,60 g (10.0 mmoles) de 4-éthynyl benzoate de méthyle, on obtient 1,00 g (27%) du composé attendu, sous la forme d'une poudre blanche de point de fusion 123-125°C.
¹H NMR (CDCl₃) δ 2,40 (s, 3H), 2,44 (s, 3H), 3,92 (s, 3H), 6,30 (d, 1H, *J* = 16,2 Hz), 7,10 (d, 1H, *J =* 16,3 Hz), 7,13 à 7,26 (m, 6H), 7,47 (d, 2H, *J =* 8,5 Hz) 7,61 (m, 1H), 7,97 (d, 2H, *J* = 8,4 Hz).

### (h) acide 4-[4-(5,4'-diméthyl-biphényl-2-yl)-but-3-èn-(E)-1-ynyl]-ber zoïque.

De manière analogue à l'exemple 1(f), à partir de 1,00 g (2,7 mmoles) de l'ester méthylique obtenu à l'exemple 13(g), on obtient 920 mg (97%) d'acide 4-[4-(5,4'-diméthyl-biphényl-2-yl)-but-3-èn-(E)-1-ynyl]-benzoïque, sous la forme d'une poudre jaune de point de fusion 269-271°C.
¹H NMR (DMSO D₆) δ 2,14 (s, 3H), 2,17 (s, 3H), 6,37 (d, 1H, *J* = 16,2 Hz), 6,73 (d, 1H, *J* = 16,3 Hz), 6,90 (s, 1H), 6,98 à 7,01 (m, 1H), 6,99 (d, 2H, *J* = 8,0 Hz), 7,32 (d, 2H, *J* = 8,3 Hz), 7,54 (d, 1H, *J* = 8,1 Hz), 7,70 (d, 2H, *J* = 8,3 Hz), 12,94 (br s, 1H).

### EXEMPLE 14

### Acide 4-[4-(6,4'-diméthyl-biphényl-2-yl)-but-3-èn-(E)-1-ynyl]-benzoïque.

### (a) 2-hydroxy-3-méthyl-benzoate de méthyle.

De manière analogue à l'exemple 12(a), à partir de 30,43 g (200,0 mmoles) d'acide 2-hydroxy-3-méthyl-benzoïque, on obtient, après six jours de reflux, 27,71 g (83%) du composé attendu, sous la forme d'un liquide incolore.
¹H NMR (CDCl₃) δ 2,34 (s, 3H), 3,92 (s, 3H), 6,69 (dd, 1H, *J* = 8,2 / 1,1 Hz), 6,79 (s, 1H), 7,70 (d, 1H, *J* = 8,1 Hz), 10,70 (s, 1H).

### (b) 3-méthyl-2-trifluorométhanesulfonyloxy-benzoate de méthyle.

De manière analogue à l'exemple 12(b), à partir de 13,29 g 80,0 mmoles) de 2-hydroxy-3-méthyl-benzoate de méthyle obtenu à l'exemple 14(a), on obtient 11,69 g (49%) du composé attendu, sous la forme d'un liquide incolore.
¹H NMR (CDCl₃) δ 2,43 (s, 3H), 3,94 (s, 3H), 7,34 (t, 1H, *J* = 7,7 Hz), 7,48 (dd, 1H, *J* = 7,7 / 1,2 Hz), 7,81 (dd, 1H, *J* = 7,7 / 1,8 Hz).

### (c) 6,4'-diméthyl-biphényl-2-carboxylate de méthyle.

De manière analogue à l'exemple 12(c), à partir de 6,39 g (47,0 mmoles) d'acide 4-méthylphényl boronique et 11,69 g (39,2 mmoles) du triflate obtenu à l'exemple 14(b), on obtient 8,60 g (91%) du composé attendu, sous la forme d'une huile incolore.
¹H NMR (CDCl₃) δ 2,10 (s, 3H), 2,40 (s, 3H), 3,57 (s, 3H), 7,04 (d, 2H, *J* = 7,8 Hz), 7,20 (d, 2H, *J* = 7,8 Hz), 7,29 (t, 1H, *J* = 7,6 Hz), 7,39 (dd, 1H, *J* = 7,5 / 0,7 Hz), 7,66 (dd, 1H, *J* = 7,6 / 0,9 Hz).

### (d) (6,4'-diméthyl-biphényl-2-yl)-méthanol.

De manière analogue à l'exemple 12(d), à partir de 8,60 g (35,8 mmoles) d'ester obtenu à l'exemple 14(c), on obtient 7,59 g (100%) du composé attendu, sous la forme d'une huile jaune.
¹H NMR (CDCl₃) δ 1,47 (br s, 1H), 2,04 (s, 3H), 2,41 (s, 3H), 4,37 (d, 2H, *J* = 3,8 Hz), 7,06 (d, 2H, *J* = 7,9 Hz), 7,19 à 7,36 (m, 7H).

### (e) 6,4'-diméthyl-biphényl-2-carboxaldéhyde.

De manière analogue à l'exemple 11(d), à partir de 7,59 g (35,75 mmoles) de l'alcool obtenu à l'exemple 14(d), on obtient 7,09 g (94%) du composé attendu, sous la forme d'une huile jaune.
¹H NMR (CDCl₃) δ 2,14 (s, 3H), 2,52 (s, 3H), 7,12 (d, 2H, *J* = 8,0 Hz), 7,26 (d, 2H, *J* = 7,8 Hz), 7,37 t, 1H, *J* = 7,6 Hz), 7,48 (dd, 1H, *J* = 7,4 / 0,5 Hz), 7,84 (d, 1H, *J* = 7,7 Hz), 9,71 (s, 1H).

### (f) 2-((E)-2-iodovinyl)-6,4'-diméthyl-biphényle.

De manière analogue à l'exemple 1(d), à partir de 3,00 g (14,3 mmoles) de 6,4'-diméthyl-biphényl-2-carboxaldéhyde obtenu à l'exemple 14(e), on obtient 3,06 g (64%) du composé attendu, sous la forme d'une huile jaune.
¹H NMR (CDCl₃) δ 2,11 (s, 3H), 2,39 (s, 3H), 6,60 (d, 1H, *J* = 14,8 Hz), 6,99 à 7,05 (m, 3H), 7,24 à 7,35 (m, 4H), 7,52 à 7,59 (m, 1H).

### (g) 4-[4-(6,4'-diméthyl-biphényl-2-yl)-but-3-èn-(E)-1-ynyl]-benzoate de méthyle.

De manière analogue à l'exemple 1(e), par réaction de 3,06 g (9,2 mmoles) de 2-((E)-2-iodovinyl)-6,4'-diméthyl-biphényle obtenu à l'exemple 14(f) avec 2,20 g (13,7 mmoles) de 4-éthynyl benzoate de méthyle, on obtient 2,40 g (71%) du composé attendu, sous la forme de cristaux jaunes de point de fusion 67°C.
¹H NMR (CDCl₃) δ 2,06 (s, 3H), 2,44 (s, 3H), 3,92 (s, 3H), 6,25 (d, 1H, *J* = 16,2 Hz), 6,79 (d, 1H, *J* = 16,3 Hz), 7,05 (d, 2H, *J* = 8,0 Hz), 7,23 à 7,26 (m, 4H), 7,44 (d, 2H, *J* = 8,3 Hz), 7,51 à 7,57 (m, 1H), 7,95 (d, 2H, *J* = 8,4 Hz).

### (h) acide 4-[4-(6,4'-diméthyl-biphényl-2-yl)-but-3-èn-(E)-1-ynyl]-benzoïque.

De manière analogue à l'exemple 1(f), à partir de 700 mg (1,9 mmole) de l'ester méthylique obtenu à l'exemple 14(g), on obtient 580 mg (86%) d'acide 4-[4-(6,4'-diméthyl-biphényl-2-yl)-but-3-èn-(E)-1-ynyl]-benzoïque, sous la forme de cristaux jaunes de point de fusion 238°C.
¹H NMR (CDCl₃ + 2 gouttes de DMSO D₆) δ 2,06 (s, 3H), 2,44 (s, 3H), 6,25 (d, 1H, *J* = 16,2 Hz), 6,79 (d, 1H, *J* = 16,3 Hz), 7,05 (d, 2H, *J* = 7,9 Hz), 7,24 à 7,28 (m, 4H), 7,45 (d. 2H, *J* = 8,3 Hz), 7,49 à 7,52 (m, 1H), 7,99 (d, 2H, *J* = 8,3 Hz).

### EXEMPLE 15

### Acide 4-[4-(4-hydroxy-4'-méthyl-biphényl-2-yl)-but-3-én-(E)-1-ynyl]-benzoïque.

### (a) 2-hydroxy-5-méthoxyméthoxy-benzaldéhyde.

Dans un tricol, on introduit 15,00 g (108,6 mmoles) de 2,5-dihydroxybenzaldéhyde et 180 ml de dichlorométhane. On refroidit la solution obtenue à 0°C, ajoute 20,8 ml de diisopropyléthylamine goutte à goutte et agite à 0°C pendant quinze minutes. On ajoute alors goutte à goutte 9,0 ml (119,0 mmoles) d'éther méthylique de chlorométhyle et agite le milieu réactionnel pendant seize heures à la température ambiante. On verse le milieu réactionnel dans un mélange HCI 1N / éther éthylique, extrait par de l'éther éthylique, décante la phase organique, sèche sur sulfate de magnésium, évapore. Le résidu obtenu est purifié par chromatographie sur colonne de silice, élué par un mélange composé de 10% d'éther éthylique et 90% d'heptane. Après évaporation des solvants, on recueille 4,42 g (22%) du composé attendu, sous la forme d'un solide blanc de point de fusion 36-41°C.
¹H NMR (CDCl₃) δ 3,50 (s, 3H), 5,15 (s, 2H), 6,91 à 6,95 (m, 1H), 7,22 à 7,27 (m, 2H), 9,85 (d, 1H, *J* = 0,35 Hz), 10,72 (s, 1H).

### (b) trifluorométhanesulfonate de 2-formyl-4-méthoxyméthoxyphényle.

De manière analogue à l'exemple 12(b), à partir de 3,70 g (20,3 mmoles) de 2-hydroxy-5-méthoxyméthoxy-benzaldéhyde obtenu à l'exemple 15(a), on obtient 2,65 g (41%) du composé attendu, sous la forme d'un liquide incolore qui sera utilisée directement pour l'étape suivante.

### (c) 4-méthoxyméthoxy-4'-méthyl-biphényl-2-carboxaldéhyde.

De manière analogue à l'exemple 12(c), à partir de 1,37 g (10,1 mmoles) d'acide 4-méthylphényl boronique et 2,65 g (8,4 mmoles) du triflate obtenu à l'exemple 15(b), on obtient 1,92 g (89%) du composé attendu, sous la forme d'une poudre beige de point de fusion 58-63°C.
¹H NMR (CDCl₃) δ 2,42 (s, 3H), 3,51 (s, 3H), 5,26 (s, 2H), 7,21 à 7,25 (m, 4H), 7,30 (dd, 1H, *J* = 8,5 / 2,6 Hz), 7,38 (d, 1H, *J* = 8,4 Hz), 7,65 (d, 1H, *J =* 2,6 Hz), 9,94 (s, 1H).

### (d) 2-((E)-2-iodovinyl)-4-méthoxyméthoxy-4'-méthyl-biphényle.

De manière analogue à l'exemple 1(d), à partir de 1,92 g (7,5 mmoles) de 4-méthoxyméthoxy-4'-méthyl-biphényl-2-carboxaldéhyde obtenu à 'exemple 15(c), on obtient 2,00 g (70%) du composé attendu, sous la forme d'une huile jaune.
¹H NMR (CDCl₃) δ 2,41 (s, 3H), 3,51 (s, 3H), 5,22 (s, 2H), 6,75 (d, 1H, *J* = 14,8 Hz), 6,98 à 7,29 (m, 7H), 7,35 (d, 1H, *J* = 14,8 Hz).

### (e) 4-[4-(4-méthoxyméthoxy-4'-méthyl-biphényl-2-yl)-but-3-èn-(E)-1-ynyl]-benzoate de méthyle.

De manière analogue à l'exemple 1(e), par réaction de 2,00 g (5,3 mmoles) de 2-((E)-2-iodovinyl)-4-méthoxyméthoxy-4'-méthyl-biphényle obtenu à l'exemple 15(d) avec 766 mg (4,8 mmoles) de 4-éthynyl benzoate de méthyle, on obtient 880 mg (44%) du composé attendu, sous la forme d'une poudre jaune clair de point de fusion 89-91°C.
¹H NMR (CDCl₃) δ 2,42 (s, 3H), 3,53 (s, 3H), 3,91 (s, 3H), 5,24 (s, 2H), 6,34 (d, 1H, *J* = 16,2 Hz), 7,05 à 7,09 (m, 1H), 7,07 (d, 1H, *J* = 16,1 Hz), 7,19 à 7,27 (m, 5H), 7,32 (d, 1H, *J* = 2,4 Hz), 7,47 (d, 2H, *J* = 8,4 Hz), 7,97 (d, 2H, *J* = 8,4 Hz).

### (f) acide 4-[4-(4-méthoxyméthoxy-4'-méthyl-biphényl-2-yl)-but-3-èn-(E)-1-ynyl]-benzoïque.

De manière analogue à l'exemple 1(f), à partir de 170 mg (0,41 mmole) de l'ester méthylique obtenu à l'exemple 15(e), on obtient 110 mg (69%) d'acide 4-[4-(4-méthoxyméthoxy-4'-méthyl-biphényl-2-yl)-but-3-èn-(E)-1-ynyl]-benzoïque, sous la forme d'une poudre jaune de point de fusion 216°C.
¹H NMR (CDCl₃ + 2 gouttes de DMSO D₆) δ 2,42 (s, 3H), 3,43 (s, 3H), 5,15 (s, 2H), 6,27 (d, 1H, *J* = 16,2 Hz), 6,95 (d, 1H, *J* = 16,2 Hz), 6,97 (dd, 1H, *J* = 8,7 / 2,4 Hz), 7,10 à 7,19 (m, 5H), 7,22 (d, 1H, *J* = 2,4 Hz), 7,37 (d, 2H, *J* = 8,3 Hz), 7,88 (d, 2H, *J* = 8,3 Hz).

### EXEMPLE 16

### Acide 4-[4-(5-hydroxy-4'-méthyl-biphényl-2-yl)-but-3-èn-(E)-1-ynyl]-benzoïque.

### (a) 2-hydroxy-4-méthoxyméthoxy-benzaldéhyde.

De manière analogue à l'exemple 15(a), à partir de 20,00 g (144,8 mmoles) de 2,4-dihydroxybenzaldéhyde, on obtient 21,05 g (80%) du composé attendu, sous la forme d'un solide blanc de point de fusion 58-64°C.
¹H NMR (CDCl₃) δ 3,49 (s, 3H), 5,23 (s, 2H), 6,60 à 6,68 (m, 2H), 7,46 (d, 1H, *J* = 8,6 Hz), 9,74 (s, 1H), 11,38 (s, 1H).

### (b) trifluorométhanesulfonate de 2-formyl-5-méthoxyméthoxyphényle.

De manière analogue à l'exemple 12(b), à partir de 6,67 g (36,6 mmoles) de 2-hydroxy-4-méthoxyméthoxy-benzaldéhyde obtenu à l'exemple 16(a), on obtient 8,03 g (70%) du composé attendu, sous la forme d'un liquide incolore.
¹H NMR (CDCl₃) δ 3,51 (s, 3H), 5,27 (s, 2H), 7,05 (d, 1H, *J* = 2,2 Hz), 7,18 (dd, 1H, *J* = 8,8 / 2,1 Hz), 7,94 (d, 1H, *J =* 8,7 Hz), 10,14 (s, 1H).

### (c) 5-méthoxyméthoxy-4'-méthyl-biphényl-2-carboxaldéhyde.

De manière analogue à l'exemple 12(c), à partir de 4,17 g (30,7 mmoles) d'acide 4-méthylphényl boronique et 8,03 g (25,6 mmoles) du triflate obtenu à l'exemple 16(b), on obtient 6,19 g (95 %) du composé attendu, sous la forme d'une huile incolore.
¹H NMR (CDCl₃) δ 2,43 (s, 3H), 3,50 (s, 3H), 5,26 (s, 2H), 7,03 (d, 1H, *J* = 2,4 Hz), 7,11 (dd, 1H, *J* = 8,7 / 2,4 Hz), 7,26 à 7,28 (m, 4H), 8,01 (d, 1H, *J* = 8,7 Hz), 9,86 (d, 1H, *J* = 0,5 Hz).

### (d) 2-((E)-2-iodovinyl)-5-méthoxyméthoxy-4'-méthyl-biphényle.

De manière analogue à l'exemple 1(d), à partir de 6,20 g (24,2 mmoles) de 5-méthoxyméthoxy-4'-méthyl-biphényl-2-carboxaldéhyde obtenu à l'exemple 16(c), on obtient 5,60 g (60%) du composé attendu, sous la forme d'une huile rouge.
¹H NMR (CDCl₃) δ 2,34 (s, 3H), 3,41 (s, 3H), 5,12 (s, 2H), 6,51 (d, 1H, *J* = 14,8 Hz), 6,87 à 7,01 (m, 2H), 7,12 à 7,18 (m, 5H), 7,23 (d, 1H, *J* = 14,9 Hz).

### (e) 4-[4-(5-méthoxyméthoxy-4'-méthyl-biphényl-2-yl)-but-3-èn-(E)-1-ynyl]-benzoate de méthyle.

De manière analogue à l'exemple 1(e), par réaction de 5,60 g (14,7 mmoles) de 2-((E)-2-iodovinyl)-5-méthoxyméthoxy-4'-méthyl-biphényle obtenu à l'exemple 16(d) avec 2,14 g (13,4 mmoles) de 4-éthynyl benzoate de méthyle, on obtient 2,51 g (45%) du composé attendu, sous la forme d'une poudre beige de point de fusion 96-98°C.
¹H NMR (CDCl₃) δ 2,43 (s, 3H), 3,49 (s, 3H), 3,91 (s, 3H), 5,22 (s, 2H), 6,24 (d, 1H, *J* = 16,2 Hz), 6,98 à 7,07 (m, 3H), 7,26 (s, 4H), 7,46 (d, 2H, *J* = 8,4 Hz), 7,60 (d, 1H, *J* = 8,6 Hz), 7,96 (d, 2H, *J* = 8,5 Hz).

### (f) acide 4-[4-(5-méthoxyméthoxy-4'-méthyl-biphényl-2-yl)-but-3-èn-(E)-1-ynyl]-benzoïque.

De manière analogue à l'exemple 1(f), à partir de 500 mg (1,2 mmole) de l'ester méthylique obtenu à l'exemple 16(e), on obtient 400 mg (83%) d'acide 4-[4-(5-méthoxyméthoxy-4'-méthyl-biphényl-2-yl)-but-3-èn-(E)-1-ynyl]-benzoique, sous la forme d'une poudre jaune de point de fusion 235°C.
¹H NMR (DMSO D₆) δ 2,38 (s, 3H), 3,40 (s, 3H), 5,27 (s, 2H), 6,52 d, 1H, *J* = 16,2 Hz), 6,90 (d, 1H, *J* = 16,1 Hz), 6,92 (d, 1H, *J* = 2,6 Hz), 7,07 (dd, 1H, *J* = 8,7 / 2,4 Hz), 7,23 (d, 2H, *J* = 8,0 Hz), 7,31 (d, 2H, *J* = 8,0 Hz), 7,53 (d, 2H, *J* = 8,3 Hz) 7,80 (d, 1H, *J* = 8,8 Hz), 7,91 (d, 2H, *J* = 8,3 Hz), 13,13 (br s, 1H).

### EXEMPLE 17

### Acide 4-[4-(6-hydroxy-4'-méthyl-biphényl-2-yl)-but-3-èn-(E)-1-ynyl]-benzoïque.

### (a) trifluorométhanesulfonate de 2-formyl-6-méthoxyphényle.

De manière analogue à l'exemple 12(b), à partir de 1,40 g (9,2 mmoles) de 2-hydroxy-3-méthoxy-benzaldéhyde, on obtient 1,70 g (65%) du composé attendu, sous la forme d'une huile orange.
¹H NMR (CDCl₃) δ 3,97 (s, 3H), 7,32 (dd, 1H, *J* = 7,8 1,7 Hz), 7,44 à 7,55 (m, 2H), 10,25 (s, 1H).

### (b) 6-méthoxy-4'-méthyl-biphényl-2-carboxaldéhyde.

De manière analogue à l'exemple 12(c), à partir de 6,34 g (46,7 mmoles) d'acide 4-méthylphényl boronique et 11,00 g (38,7 mmoles) du triflate obtenu à l'exemple 17(a), on obtient 7,75 g (88%) du composé attendu, sous la forme de cristaux blancs de point de fusion 63-65°C.
¹H NMR (CDCl₃) δ 2,42 (s, 3H), 3,79 (s, 3H), 7,16 à 7,23 (m, 1H), 7,21 (d, 2H, *J* = 8,0 Hz), 7,27 (d, 2H, *J* = 8,1 Hz), 7,43 (t, 1H, *J* = 7,9 Hz), 7,61 (dd, 1H, *J* = 7,9 / 1,1 Hz), 9,74 (s, 1H).

### (c) 6-hydroxy-4'-méthyl-biphényl-2-carboxaldéhyde.

Dans un ballon, on introduit 950 mg (4,2 mmoles) de 6-méthoxy-4'-méthyl-biphényl-2-carboxaldéhyde obtenu à l'exemple 17(b), 1,41 g (16,8 mmoles) d'éthanethiolate de sodium et 20 ml de DMF. Le milieu est chauffé à 150°C pendant deux heures, refroidi, versé dans un mélange HCI 1N / éther éthyl que et extrait par de l'éther éthylique. On décante la phase organique, sèche sur sulfate de magnésium, évapore. Le résidu obtenu est purifié par trituration dans un mélange composé de 10% d'éther éthylique et 90% d'heptane. Après séchage à l'étuve à vide, on recueille 550 mg (61%) du composé attendu, sous la forme d'une poudre orangée de point de fusion 170-171°C.
¹H NMR (CDCl₃) δ 2,45 (s, 3H), 5,14 (br s, 1H), 7,22 à 7,27 (m, 3H), 7,35 à 7,43 (m, 3H), 7,60 (dd, 1H, *J* = 7,7 / 1,2 Hz), 9,74 (s, 1H).

### (d) 6-méthoxyméthoxy-4'-méthyl-biphényl-2-carboxaldéhyde.

De manière analogue à l'exemple 15(a), à partir de 4,35 g (20,5 mmoles) de 6-hydroxy-4'-méthyl-biphényl-2-carboxaldéhyde obtenu à l'exemple 17(c), on obtient 4,31 g (82%) du composé attendu, sous la forme d'une huile jaune.
¹H NMR (CDCl₃) δ 2,43 (s, 3H), 3,35 (s, 3H), 5,09 (s, 2H), 7,22 (d 2H, *J* = 8,3 Hz), 7,27 (d, 2H, *J* = 8,2 Hz), 7,38 à 7,45 (m, 2H), 7,64 à 7,71 (m, 1H), 9,74 (s, 1H).

### (e) 2-((E)-2-iodovinyl)-6-méthoxyméthoxy-4'-méthyl-biphényle.

De manière analogue à l'exemple 1(d), à partir de 4,30 g (16,8 mmoles) de 6-méthoxyméthoxy-4'-méthyl-biphényl-2-carboxaldéhyde obtenu à l'exemple 17(d), on obtient 3,74 g (58%) du composé attendu, sous la forme d'une huile orange.
¹H NMR (CDCl₃) δ 2,42 (s, 3H), 3,31 (s, 3H), 5,03 (s, 2H), 6,67 (d, 1H, *J* = 14,8 Hz), 7,09 à 7,41 (m, 8H).

### (f) 4-[4-(6-méthoxyméthoxy-4'-méthyl-biphényl-2-yl)-but-3-èn-(E )1-ynyl]-benzoate de méthyle.

De manière analogue à l'exemple 1(e), par réaction de 3,74 g (9,8 mmoles) de 2-((E)-2-iodovinyl)-6-méthoxyméthoxy-4'-méthyl-biphényle obtenu à l'exemple 17(e), avec 1,89 g (11,8 mmoles) de 4-éthynyl benzoate de méthyle, on obtient 900 mg (22%) du composé attendu, sous la forme d'une poudre orange clair de point de fusion 98-100°C.
¹H NMR (CDCl₃) δ 2,43 (s, 3H), 3,32 (s, 3H), 3,91 (s, 3H), 5,04 s, 2H), 6,28 (d, 1H, *J* = 16,2 Hz), 6,83 (d, 1H, *J* = 16,2 Hz), 7,15 (d, 2H, *J* = 8,0 Hz), 7,16 (d, 1H, *J* = 8,3 Hz), 7,27 (d, 2H, *J* = 8,3 Hz), 7,32 à 7,36 (m, 2H), 7,45 (d, 2H, *J* = 8,3 Hz), 7,96 (d, 2H, *J* = 8,2 Hz).

### (g) acide 4-[4-(6-méthoxyméthoxy-4'-méthyl-biphényl-2-yl)-but-3-èn-(E)1-ynyl]-benzoïque.

De manière analogue à l'exemple 1(f), à partir de 200 mg (0,48 mmole) de l'ester méthylique obtenu à l'exemple 17(f), on obtient 165 mg (87%) d'acide 4-[4-(6-méthoxyméthoxy-4'-méthyl-biphényl-2-yl)-but-3-èn-(E)1-ynyl]-berzoïque, sous la forme d'une poudre blanche de point de fusion 185-187°C.
¹H NMR (DMSO D₆) δ 2,38 (s, 3H), 3,20 (s, 3H), 5,07 (s, 2H), 6,58 (d, 1H, *J* = 16,3 Hz), 6,68 (d, 1H, *J* = 16,3 Hz), 7,10 (d, 2H, *J* = 7,9 Hz), 7,18 (d, 1H, *J =* 8,1 Hz), 7,28 (d, 2H, *J* = 7,9 Hz), 7,35 (t, 1H, *J* = 8,0 Hz), 7,52 (d, 1H, *J* = 8,0 Hz), 7,53 d, 2H, *J* = 8,3 Hz), 7,90 (d, 2H, *J* = 8,3 Hz), 13,13 (br s, 1H).

### EXEMPLE 18

### Acide 4-[4-(4'-méthyl-biphényl-2-yl)-pent-3-èn-(E)-1-ynyl]-benzoïque.

### (a) 1-(4'-méthyl-biphényl-2-yl)-éthanone.

De manière analogue à l'exemple 2(b), à partir de 15,00 g (75,0 mmoles) de 2-bromoacétophénone et de 13,3 g (98,0 mmoles) d'acide 4-inéthylphényl boronique obtenu à l'exemple 2(a), on obtient 15,10 g (95%) du composé attendu, sous la forme d'une huile jaune.
¹H NMR (CDCl₃) δ 2,02 (s, 3H), 2,41 (s, 3H), 7,24 (s, 4H), 7,37 à 7,56 (m, 4H).

### (b) 3-(4'-méthyl-biphényl-2-yl)-but-2-ène-(E)-nitrile.

De manière analogue à l'exemple 6(a), à partir de 13,00 g (61,8 mmoles) de la cétone obtenue à l'exemple 18(a), 16,40 g (93,0 mmoles) de cyanométhylphosphonate de diéthyle, 3,15 g (105,0 mmoles) d'hydrure de sodium à 80%, et 160 ml de THF, on obtient 14,40 g (100%) du composé attendu, brut, sous la forme d'une huile brune.
¹H NMR (CDCl₃) δ 1,97 (d, 3H, *J* = 1,0 Hz), 2,40 (s, 3H), 5,35 (d 1H, *J* = 1,1 Hz), 7,16 à 7,46 (m, 8H).

### (c) 3-(4'-méthyl-biphényl-2-yl)-but-2-èn-(E)-al.

De manière analogue à l'exemple 6(b) à partir de 14,4 g (61,7 mmoles) du nitrile obtenu à l'exemple 18(b), on obtient 16,00 g (75% net) du produit attendu, brut, sous la forme d'une huile jaune, contenant environ 25% de produit de départ.
¹H NMR (CDCl₃) δ 2,04 (d, 3H, *J* = 1,2 Hz), 2,39 (s, 3H), 6,16 (dd, 1H, *J* = 8,1/1,3 Hz), 7,17 à 7,45 (m, 8H), 10,01 (d, 1H, *J* = 8,1 Hz).

### (d) 2-(4,4-dibromo-1-méthyl-buta-1-(E),3-diènyl)-4'-méthyl-biphényle.

De manière analogue à l'exemple 8(a), à partir de 11,00 g (46,5 mmoles) de l'aldéhyde obtenu à l'exemple 18(c), 48,80 g (186,0 mmoles) de triphénylphosphine, 30,87 g (93,1 mmoles) de tétrabromure de carbone et 300 ml de dichlorométhane, on obtient 16,70 g (91%) du composé attendu, sous la forme d'une huile jaune qui cristallise lentement.
¹H NMR (CDCl₃) δ 1,62 (d, 3H, *J* = 1,3 Hz), 2,39 (s, 3H), 6,18 (dd, 1H, *J* = 10,7 / 1,4 Hz), 6,75 (d, 1H, *J* = 13,7 Hz), 7,12 à 7,37 (m, 8H).

### (e) 4'-méthyl-2-(1-méthyl-but-1-èn-(E)-3-ynyl)-biphényle.

De manière analogue à l'exemple 8(b), à partir de 5,00 g (12,7 mmoles) du composé dibromé obtenu à l'exemple 18(d) et 10,7 ml de n.butyllithium (2,5 N dans l'hexane), on obtient 3,25 g (100%) du composé attendu, sous la forme d'une huile orangée.
¹H NMR (CDCl₃) δ 1,80 (s, 3H), 2,39 (s, 3H), 3,18 (d, 1H, *J* = 2,3 Hz), 5,56 à 5,57 (m, 1H), 7,10 à 7,38 (m, 8H).

### (f) 4-[4-(4'-méthyl-biphényl-2-yl)-pent-3-èn-(E)-1-ynyl]-benzoate de méthyle.

Dans un ballon, on introduit successivement 3,00 g (13,0 mmoles) du composé obtenu à l'exemple 18(e), 3,40 g (13,0 mmoles) de 4-iodobenzoate de méthyle et 40 ml de triéthylamine. Le milieu réactionnel est dégazé à l'azote pendant vingt minutes, puis on ajoute 250 mg de Cul, et 360 mg de chlorure de bis(triphénylphosphine)palladium(ll). On agite à la température ambiante pendant trois heures, verse le milieu réactionnel dans l'eau, acidifie par de l'acide chlorhydrique 1N, extrait par de l'éther éthylique, décante la phase organique, sèche sur sulfate de magnésium, évapore. Le résidu obtenu est purifié par chromatographie sur colonne de silice élué par un mélange composé de 90% d'acétate d'éthyle et de 10% d'heptane. Après évaporation des solvants, on recueille 2,43 g (50%) du composé attendu, sous la forme d'une huile orangée.
¹H NMR (CDCl₃) δ 1,88 (d, 3H, *J* = 1,0 Hz), 2,38 (s, 3H), 3,92 (s, 3H), 5,82 (d, 1H, *J* = 1,1 Hz), 7,14 à 7,40 (m, 8H), 7,49 (d, 2H, *J* = 8,4 Hz), 7,99 (d, 2H, *J* = 8,4 Hz).

### (g) acide 4-[4-(4'-méthyl-biphényl-2-yl)-pent-3-èn-(E)-1-ynyl]-benzoïque.

De manière analogue à l'exemple 1(f), à partir de 2,40 g (6,5 mmoles) de composé obtenu à l'exemple 18(f), on obtient 1,20 g (52%) d'acide 4-[4-(4'-méthyl-biphényl-2-yl)-pent-3-èn-(E)-1-ynyl]-benzoïque, sous la forme d'une poudre beige de point de fusion 261-265°C.
¹H NMR (DMSO D₆) δ 1,88 (s, 3H), 2,34 (s, 3H), 5,82 (d, 1H, *J* = 0 Hz), 7,25 à 7,43 (m, 8H), 7,56 (d, 2H, *J* = 8,3 Hz), 7,93 (d, 2H, *J* = 8,3 Hz).

### EXEMPLE 19

### Acide 4-(3-(4'-méthyl-biphényl-2-yl)-propynoylamino]-benzoïque.

### (a) 4-[3-(4'-méthyl-biphényl-2-yl)-propynoylamino]-benzoate d'allyle.

De manière analogue à l'exemple 8(e), à partir de 3,30 g (14,0 mmoles) du composé obtenu à l'exemple 8(d) et 1,98 g (11,0 mmoles) de 4-aminobenzoate d'allyle, on obtient 900 mg (20%) du composé attendu, sous la forme d'une huile brune.
¹H NMR (CDCl₃) δ 2,41 (s, 3H), 4,79 à 4,81 (m, 2H), 5,28 (dd, 1H, *J* = 10,4 / 1,2 Hz), 5,39 (dd, 1H, *J* = 17,2 / 1,5 Hz), 5,95 à 6,10 (m, 1H), 7,25 à 7,33 (m, 3H), 7,39 à 7,62 (m, 6H), 7,73 (s, 1H), 8,00 (d, 2H, J = 8,6 Hz).

### (b) acide 4-[3-(4'-méthyl-biphényl-2-yl)-propynoylamino]-benzoïque.

De manière analogue à l'exemple 3(d), à partir de 500 mg (1,3 mmole) du composé obtenu à l'exemple 19(a), on obtient 350 mg (77%) d'acide 4-[3-(4'-méthyl-biphényl-2-yl)-propynoylamino]-benzoïque, sous la forme d'une poudre beige de point de fusion 184-188°C.
¹H NMR (DMSO D₆) δ 2,37 (s, 3H), 7,30 (d, 2H, *J* = 7,9 Hz), 7,45 à 7,64 (m, 5H), 7,71 à 7,79 (m, 3H), 7,92 (d, 2H, *J* = 8,6 Hz), 11,06 (s, 1H), 12,83 (br s, 1H).

### EXEMPLE 20

### Acide 4-[3-(4'-méthyl-biphényl-2-yl)-propynethioylamino]-benzoïque.

### (a) 4-[3-(4'-méthyl-biphényl-2-yl)-propynethioylamino]-benzoate d allyle.

De manière analogue à l'exemple 4(a), à partir de 310 mg (0,8 mmole) du composé obtenu à l'exemple 19(a), on obtient 160 mg (50%) du composé attendu, sous la forme d'une huile rouge.
¹H NMR (CDCl₃) δ 2,40 (s, 3H), 4,82 (d, 2H, *J* = 5,6 Hz), 5,29 (d, 1H, *J* = 10,4 Hz), 5,42 (dd, 1H, *J* = 17,2 / 1,5 Hz), 5,99 à 6,10 (m, 1H), 6,60 (s, 1H), 7,12 (d, 2H, *J* = 8,5 Hz), 7,19 à 7,26 (m, 4H), 7,42 à 7,44 (m, 2H), 7,49 à 7,57 (m, 2H), 8,08 (d, 2H, *J* = 8,5 Hz).

### (b) acide 4-[3-(4'-méthyl-biphényl-2-yl)-propynethioylamino]-benzoïque.

De manière analogue à l'exemple 1(f), à partir de 100 mg (0,24 mmole) du composé obtenu à l'exemple 20(a), on obtient 90 mg (90%) d'acide 4-[3-(4'-méthyl-biphényl-2-yl)-propynethioylamino]-benzoïque, sous la forme d'une poudre jaune orangée de point de fusion 170°C (décomposition).
¹H NMR (DMSO D₆) δ 2,36 (s, 3H), 7,14 (s, 1H), 7,28 (br s, 6H), 7,46 à 7,68 (m, 4H), 8,00 (d, 2H, *J* = 8,2 Hz).

### EXEMPLE 21

### Acide 4-[4-(3'-méthyl-biphényl-2-yl)-but-3-èn-1-ynyl]-benzoïque.

### (a) 3'-méthyl-biphényl-2-carboxaldéhyde.

Dans un tricol, on introduit 3,85 g (28,0 mmoles) d'acide 3-méthylbenzène boronique, 3,50 g (18,9 mmoles) de 2-bromobenzaldéhyde, 18,9 ml (37,8 mmoles) d'une solution aqueuse de carbonate de potassium (2M) et 75 ml de diméthoxyéthane. Le mélange est dégazé à la température ambiante par barbotage d'argon pendant une heure, puis on ajoute 1,09 g (0,95 mmole) de tétrakistriphénylphosphinepalladium(0) et chauffe à reflux pendant deux heures et quarante minutes. On refroidit, verse le milieu réactionnel dans l'eau, extrait par de l'acétate d'éthyle, décante la phase organique, sèche sur sulfate de magnésium, évapore. Le résidu obtenu est purifié par chromatographie sur colonne de silice, élué par un mélange composé de 5% d'acétate d'éthyle et de 95% d'heptane. Après évaporation des solvants, on recueille 3,70 g (100%) du composé attendu, sous la forme d'une huile jaune pâle.
¹H NMR (CDCl₃) δ 2,43 (s, 3H), 7,17 à 7,20 (m, 1H), 7,24 à 7,27 (m, 1H), 7,35 (d, 1H, *J* = 7,7 Hz), 7,39 à 7,67 (m, 3H), 7,62 (dd, 1H, *J* = 7,4 / 1,5 Hz), 8,02 (dd, 1H, *J* = 7,6 / 1,4 Hz), 9,99 (s, 1H).

### (b) 2-((E)-2-iodovinyl)-3'-méthyl-biphényle.

De manière analogue à l'exemple 1(d), à partir de 1,45 g (7,38 mmoles) de 3'-méthyl-biphényl-2-carboxaldéhyde obtenu à l'exemple 21(a), on obtient 2,10 g (89%) du composé attendu, sous la forme d'une huile jaune.
¹H NMR (CDCl₃) δ 2,34 (s, 3H), 6,65 (d, 1H, *J* = 14,8 Hz), 6,91 à 7,38 (m, 9H).

### (c) 4-[4-(3'-méthyl-biphényl-2-yl)-but-3-èn-(E)-1-ynyl]-benzoate de méthyle.

De manière analogue à l'exemple 1(e), par réaction de 2,10 g (6,55 mmoles) de 2-(2-iodovinyl)-3'-méthyl-biphényle obtenu à l'exemple 21(b) avec 1,05 g (6,55 mmoles) de 4-éthynyl benzoate de méthyle, on obtient 520 mg (22%) du composé attendu, sous la forme d'une huile jaune.
¹H NMR (CDCl₃) δ 2,43 (s, 3H), 3,91 (s, 3H), 6,35 (d, 1H, *J* = 16,2 Hz), 7,09 (d, 1H, *J* = 16,3 Hz), 7,17 à 7,38 (m, 7H), 7,47 (d, 2H, *J* = 8,4 Hz), 7,64 à 7,68 (m, 1H), 7,97 (d, 2H, *J* = 8,4 Hz).

### (d) acide 4-[4-(3'-méthyl-biphényl-2-yl)-but-3-èn-(E)-1-ynyl]-benzoïque.

De manière analogue à l'exemple 1(f), à partir de 520 mg (1,47 mmole) de l'ester méthylique obtenu à l'exemple 21(c), on obtient 370 mg (74%) d'acide 4-[4-(3'-méthyl-biphényl-2-yl)-but-3-èn-(E)-1-ynyl]-benzoïque, sous la forme d'une poudre beige de point de fusion 211°C.
¹H NMR (CDCl₃ + 2 gouttes de DMSO D₆) δ 2,43 (s, 3H), 6,36 (d, 1H, *J* = 16,2 Hz), 7,07 (d, 1H, *J* = 16,3 Hz), 7,12 à 7,23 (m, 3H), 7,29 à 7,48 (m, 4H), 7,46 (d, 2H, *J* = 8,3 Hz), 7,65 à 7,69 (m, 1H), 7,98 (d, 2H, *J* = 8,3 Hz).

### EXEMPLE 22

### Acide 4-[4-(2'-méthyl-biphényl-2-yl)-but-3-èn-(E)-1-ynyl]-benzoïque.

### (a) 2'-méthyl-biphényl-2-carboxaldéhyde.

De manière analogue à l'exemple 21(a), à partir de 3,86 g (28,4 mmoles) d'acide 2-méthylbenzène boronique et 3,50 g (18,9 mmoles) de 2-bromobenzaldéhyde, on obtient 2,50 g (67%) du composé attendu, sous la forme d'une huile legèrement jaune.
¹H NMR (CDCl₃) δ 2,10 (s, 3H), 7,17 à 7,38 (m, 5H), 7,47 à 7,53 (m, 1H), 7,63 (dd, 1H, *J* = 7,5 / 1,5 Hz), 8,03 (dd, 1H, *J* = 7,7 / 1,3 Hz), 9,75 (d, 1H, *J* = 0,6 Hz).

### (b) 2-((E)-2-iodovinyl)-2'-méthyl-biphényle.

De manière analogue à l'exemple 1(d), à partir de 2,00 g (10,2 mmoles) de 2'-méthyl-biphényl-2-carboxaldéhyde obtenu à l'exemple 22(a), on obtient 3,26 g (100%) du composé attendu, sous la forme d'une huile jaune.
¹H NMR (CDCl₃) δ 2,04 (s, 3H), 6,70 (d, 1H, *J* = 14,8 Hz), 7,10 (d, 1H, *J* = 14,8 Hz), 7,09 à 7,49 (m, 8H).

### (c) 4-[4-(2'-méthyl-biphényl-2-yl)-but-3-èn-(E)-1-ynyl]-benzoate de méthyle.

De manière analogue à l'exemple 1(e), par réaction de 3,26 g (10,2 mmoles) de 2-(2-iodovinyl)-2'-méthyl-biphényle obtenu à l'exemple 22(b) avec 1,63 g (10,2 mmoles) de 4-éthynyl benzoate de méthyle, on obtient 360 mg (10%) du composé attendu, sous la forme d'une huile orangée.
¹H NMR (CDCl₃) δ 2,07 (s, 3H), 3,91 (s, 3H), 6,32 (d, 1H, *J* = 16,3 Hz), 6,77 (d, 1H, *J* = 16,3 Hz), 7,13 à 7,57 (m, 7H), 7,45 (d, 2H, *J =* 8,4 Hz), 7,66 à 7,70 (m, 1H), 7,95 (d, 2H, *J* = 8,5 Hz).

### (d) acide 4-[4-(2'-méthyl-biphényl-2-yl)-but-3-èn-(E)-1-ynyl]-benzoïque.

De manière analogue à l'exemple 1(f), à partir de 360 mg (1,02 mmole) de l'ester méthylique obtenu à l'exemple 22(c), on obtient 222 mg (64%) d'acide 4-[4-(2'-méthyl-biphényl-2-yl)-but-3-èn-(E)-1-ynyl]-benzoïque, sous la forme d'une poudre jaune de point de fusion 250°C.
¹H NMR (CDCl₃ + 2 gouttes de DMSO D₆) δ 2,07 (s, 3H), 6,33 (d, 1H, *J* = 16,3 Hz), 6,75 (d, 1H, *J* = 16,3 Hz), 7,12 à 7,19 (m, 2H), 7,27 à 7,46 (m, 5H), 7,44 (d, 2H, *J* = 8,3 Hz), 7,67 à 7,71 (m, 1H), 7,96 (d, 2H, J = 8,3 Hz).

### EXEMPLE 23

### Acide 4-[4-(4'-chloro-biphényl-2-yl)-but-3-èn-1-ynyl]-benzoïque.

### (a) 4'-chloro-biphényl-2-carboxaldéhyde.

De manière analogue à l'exemple 21(a), à partir de 5,00 g (31,9 mmoles) d'acide 4-chlorobenzène boronique et 3,94 g (21,3 mmoles) de 2-bromobenzaldéhyde, on obtient 4,40 g (95%) du composé attendu, sous la forme d'une huile jaune.
¹H NMR (CDCl₃) δ 7,32 (d, 2H, *J* = 8,5 Hz), 7,42 à 7,55 (m, 2H), 7,45 (d, 2H, *J* = 8,5 Hz), 7,64 (dd, 1H, *J* = 7,5 / 1,5 Hz), 8,03 (dd, 1H, *J* = 7,7 / 1,3 Hz), 9,97 (s, 1H).

### (b) 2-((E)-2-iodovinyl)-4'-chloro-biphényle.

De manière analogue à l'exemple 1(d), à partir de 2,50 g (11,5 mmoles) de 4'-chloro-biphényl-2-carboxaldéhyde obtenu à l'exemple 23(a), on obtient 2,40 g (61%) du composé attendu, sous la forme d'une huile orangée.
¹H NMR (CDCl₃) δ 6,76 (d, 1H, *J* = 14,8 Hz), 7,24 à 7,47 (m, 9H).

### (c) 4-[4-(4'-chloro-biphényl-2-yl)-but-3-èn-(E)-1-ynyl]-benzoate de méthyle.

De manière analogue à l'exemple 1(e), par réaction de 2,40 g (7,0 mmoles) de 2-((E)-2-iodovinyl)-4'-chloro-biphényle obtenu à l'exemple 23(b) avec 1,03 g (6,4 mmoles) de 4-éthynyl benzoate de méthyle, on obtient 970 mg (41%) du composé attendu, sous la forme d'un solide jaune clair de point de fusion 135°C.
¹H NMR (CDCl₃) δ 3,91 (s, 3H), 6,35 (d, 1H, *J* = 16,2 Hz), 7,01 (d, 1H, *J =* 16,2 Hz), 7,26 à 7,45 (m, 7H), 7,49 (d, 2H, *J* = 8,4 Hz), 7,63 à 7,67 (m, 1H), 7,97 (d, 2H, *J* = 8,4 Hz).

### (d) acide 4-[4-(4'-chloro-biphényl-2-yl)-but-3-èn-(E)-1-ynyl]-benzoïque.

De manière analogue à l'exemple 1(f), à partir de 0,97 g (26 01 mmoles) de l'ester méthylique obtenu à l'exemple 23(c), on obtient 880 mg (94%) d'acide 4-[4-(4'-chloro-biphényl-2-yl)-but-3-èn-(E)-1-ynyl]-benzoïque, sous la forme d'un solide jaune clair de point de fusion 273°C.
¹H NMR (CDCl₃ + 2 gouttes de DMSO D₆) δ 6,15 (d, 1H, *J* = 16,2 Hz), 6,76 (d, 1H, *J* = 16,2 Hz), 7,05 à 7,18 (m, 7H), 7,21 (d, 2H, *J* = 8,2 Hz), 7,43 à 7,46 (m, 1H), 7,73 (d, 2H, *J* = 8,3 Hz).

### EXEMPLE 24

### Acide 4-[4-(3'-chloro-biphényl-2-yl)-but-3-èn-(E)-1-ynyl]-benzoïque.

### (a) 3'-chloro-biphényl-2-carboxaldéhyde.

De manière analogue à l'exemple 21(a), à partir de 5,00 g (31,9 mmoles) d'acide 3-chlorobenzène boronique et 3,94 g (21,3 mmoles) de 2-bromobenzaldéhyde, on obtient 4,39 g (95%) du composé attendu, sous la forme d'une huile jaune.
¹H NMR (CDCl₃) δ 7,23 à 7,27 (m, 1H), 7,37 à 7,45 (m, 4H), 7,5C à 7,56 (m, 1H), 7,64 (dd, 1H, *J =* 7,5 / 1,5 Hz), 8,04 (dd, 1H, *J* = 7,7 / 1,4 Hz), 9,98 (d, 1H, *J* = 0,6 Hz).

### (b) 2-((E)-2-iodovinyl)-3'-chloro-biphényle.

De manière analogue à l'exemple 1(d), à partir de 2,50 g (11,5 mmoles) de 3'-chloro-biphényl-2-carboxaldéhyde obtenu à l'exemple 24(a), on obtient 1,85 g (47%) du composé attendu, sous la forme d'une huile jaune.
¹H NMR (CDCl₃) δ 6,77 (d, 1H, *J* = 14,9 Hz), 7,19 à 7,47 (m, 9H).

### (c) 4-[4-(3'-chloro-biphényl-2-yl)-but-3-èn-(E)-1-ynyl]-benzoate de méthyle.

De manière analogue à l'exemple 1(e), par réaction de 1,85 g (5,4 mmoles) de 2-((E)-2-iodovinyl)-3'-chloro-biphényle obtenu à l'exemple 24(b) avec 791 mg (4,9 mmoles) de 4-éthynyl benzoate de méthyle, on obtient 900 mg (49%) du composé attendu, sous la forme d'une poudre beige de point de fusion 120-125°C.
¹H NMR (CDCl₃) δ 3,91 (s, 3H), 6,36 (d, 1H, *J* = 16,2 Hz), 7,01 (d, 1H, *J* = 16,2 Hz), 7,20 à 7,30 (m, 2H), 7,35 à 7,42 (m, 5H), 7,48 (d, 2H, *J* = 8,4 Hz), 7,64 à 7,68 (m, 1H), 7,97 (d, 2H, *J* = 8,5 Hz).

### (d) acide 4-[4-(3'-chloro-biphényl-2-yl)-but-3-èn-(E)-1-ynyl]-benzoïque.

De manière analogue à l'exemple 1(f), à partir de 900 mg (24,1 mmoles) de l'ester méthylique obtenu à l'exemple 24(c), on obtient 800 mg (92%) d'acide 4-[4-(3'-chloro-biphényl-2-yl)-but-3-èn-(E)-1-ynyl]-benzoïque, sous la forme d'un solide jaune clair de point de fusion 212°C.
¹H NMR (CDCl₃ + 2 gouttes de DMSO D₆) δ 6,38 *(d,* 1H, *J* = 16,2 Hz), 6,99 (d, 1H, *J* = 16,2 Hz), 7,22 à 7,51 (m, 7H), 7,48 (d, 2H, *J* = 8,3 Hz), 7,66 à 7,70 (m, 1H), 7,97 (d, 2H, *J* = 8,3 Hz).

### EXEMPLE 25

### Acide 4-[4-(4'-fluoro-biphényl-2-yl)-but-3-èn-(E)-1-ynyl]-benzoïque.

### (a) 4'-fluoro-biphényl-2-carboxaldéhyde.

De manière analogue à l'exemple 21(a), à partir de 4,36 g (31,1 mmoles) d'acide 4-fluorobenzène boronique et 3,84 g (20,7 mmoles) de 2-bromobenzaldéhyde, on obtient 4,09 g (98%) du composé attendu, sous la forme d'une huile jaune.
¹H NMR (CDCl₃) δ 7,15 (d, 2H, *J* = 8,7 Hz), 7,17 à 7,54 (m, 4H), 7,63 (dd, 1H, *J* = 7,5 / 1,5 Hz), 8,02 (dd, 1H, *J* = 7,7 / 1,3 Hz), 9,97 (d, 1H, *J* = 0,6 Hz).

### (b) 2-((E)-2-iodovinyl)-4'-fluoro-biphényle.

De manière analogue à l'exemple 1(d) à partir de 2,50 g (12,4 mmoles) de 4'-fluoro-biphényl-2-carboxaldéhyde obtenu à l'exemple 25(a), on obtient 2,69 g (66%) du composé attendu, sous la forme d'une huile orangée qui sera utilisée directement pour l'étape suivante.

### (c) 4-[4-(4'-fluoro-biphényl-2-yl)-but-3-èn-(E)-1-ynyl]-benzoate de méthyle.

De manière analogue à l'exemple 1(e), par réaction de 2,69 g (8,3 mmoles) de 2-((E)-2-iodovinyl)-4'-fluoro-biphényle obtenu à l'exemple 25(b) avec 1,20 g (7,5 mmoles) de 4-éthynyl benzoate de méthyle, on obtient 1,20 g (45%) du composé attendu, sous la forme d'une poudre jaune de point de fusion 113°C.
¹H NMR (CDCl₃) δ 3,91 (s, 3H), 6,35 (d, 1H, *J* = 16,2 Hz), 7,02 (d, 1H, *J* = 16,2 Hz), 7,11 à 7,19 (m, 2H), 7,25 à 7,40 (m, 5H), 7,48 (d, 2H, *J* = 8,4 Hz), 7,62 à 7,68 (m, 1H), 7,97 (d, 2H, *J* = 8,4 Hz).

### (d) acide 4-[4-(4'-fluoro-biphényl-2-yl)-but-3-èn-(E)-1-ynyl]-benzoïque.

De manière analogue à l'exemple 1(f), à partir de 1,20 g (33,7 mmoles) de l'ester méthylique obtenu à l'exemple 25(c), on obtient 1,12 g (97%) d'acide 4-[4-(4'-fluoro-biphényl-2-yl)-but-3-èn-(E)-1-ynyl]-benzoïque, sous la forme d'un solide jaune clair de point de fusion 220°C.
¹H NMR (CDCl₃ + 2 gouttes de DMSO D₆) δ 6,36 (d, 1H, *J* = 16,2 Hz), 7,01 (d, 1H, *J* = 16,2 Hz), 7,12 à 7,19 (m, 2H), 7,27 à 7,40 (m, 5H), 7,47 (d, 2H, *J* = 8,4 Hz), 7,64 à 7,68 (m, 1H), 7,99 (d, 2H, *J* = 8,4 Hz).

### EXEMPLE 26

### Acide 4-[4-(4'-propyl-biphényl-2-yl)-but-3-èn-(E)-1-ynyl]-benzoïque.

### (a) 4'-propyl-biphényl-2-carboxaldéhyde.

De manière analogue à l'exemple 21(a), à partir de 3,40 g (20,7 mmoles) d'acide 4-propylbenzène boronique et 2,56 g (13,8 mmoles) de 2-bromobenzaldéhyde, on obtient 2,07 g (67%) du composé attendu, sous la forme d'une huile légèrement jaune.
¹H NMR (CDCl₃) δ 0,99 (t, 3H, *J* = 7,4 Hz), 1,70 (m, 2H, *J* = 7,4 Hz), 2,66 (t, 2H, *J* = 7,4 Hz), 7,29 à 7,32 (m, 4H), 7,44 à 7,66 (m, 2H), 7,61 (dd, 1H, *J* = 7,5 / 1,5 Hz), 8,01 (dd, 1H, *J =* 6,5 / 1,3 Hz), 9,99 (s, 1H).

### (b) 2-((E)-2-iodovinyl)-4'-propyl-biphényle.

De manière analogue à l'exemple 1(d), à partir de 2,06 g (9,1 mmoles) de 4'-propyl-biphényl-2-carboxaldéhyde obtenu à l'exemple 26(a), on obtient 2,14 g (67%) du composé attendu, sous la forme d'une huile jaune.
'H NMR (CDCl₃) δ 1,00 (t, 3H, *J* = 7,5 Hz), 1,69 (m, 2H, *J* = 7,6 Hz), 2,65 (t, 2H, *J* = 7,3 Hz), 6,73 (d, 1H, *J* = 14,8 Hz), 7,21 à 7,48 (m, 9H).

### (c) 4-[4-(4'-propyl-biphényl-2-yl)-but-3-èn-(E)-1-ynyl]-benzoate de méthyle.

De manière analogue à l'exemple 1(e), par réaction de 2,14 g (6,1 mmoles) de 2-(2-iodovinyl)-4'-propyl-biphényle obtenu à l'exemple 26(b) avec 980 mg (6,1 mmoles) de 4-éthynyl benzoate de méthyle, on obtient 760 mg (32%) du composé attendu, sous la forme d'une huile incolore.
¹H NMR (CDCl₃) δ 1,01 (t, 3H, *J* = 7,2 Hz), 1,72 (m, 2H, *J* = 7,4 Hz), 2,66 (t, 2H, *J* = 7,5 Hz), 3,91 (s, 3H), 6,36 (d, 1H, *J* = 16,2 Hz), 7,12 (d, 1H, *J* = 16,2 Hz), 7,26 à 7,31 (m, 4H), 7,33 à 7,38 (m, 3H), 7,48 (d, 2H, J = 8,4 Hz), 7,64 à 7,67 (m, 1H), 7,97 (d, 2H, *J* = 8,4 Hz).

### (d) acide 4-[4-(4'-propyl-biphényl-2-yl)-but-3-èn-(E)-1-ynyl]-benzoïque.

De manière analogue à l'exemple 1(f), à partir de 760 mg (2,0 mmoles) de l'ester méthylique obtenu à l'exemple 26(c), on obtient 670 mg (91%) d'acide 4-[4-(4'-propyl-biphényl-2-yl)-but-3-èn-(E)-1-ynyl]-benzoïque,sous la forme d'une poudre verdâtre de point de fusion 198°C.
¹H NMR (CDCl₃ + 2 gouttes de DMSO D₆) δ 1,00 (t, 3H, *J* = 7,3 Hz), 1 64 à 1,79 (m, 2H), 2,66 (t, 2H, *J* = 7,4 Hz), 6,36 (d, 1H, *J* = 16,2 Hz), 7,11 (d, 1H, *J* = 16 2 Hz), 7,27 à 7,38 (m, 7H), 7,47 (d, 2H, *J* = 8,4 Hz), 7,64 à 7,68 (m, 1H), 7,98 (d, 2H, *J* = 8,4 Hz).

### EXEMPLE 27

### Acide 4-[4-(4'-vinyl-biphényl-2-yl)-but-3-èn-(E)-1-ynyl]-benzoïque.

### (a) 4'-vinyl-biphényl-2-carboxaldéhyde.

De manière analogue à l'exemple 21(a), à partir de 5,00 g (33,8 mmoles) d'acide 4-vinylbenzène boronique et 4,17 g (22,5 mmoles) de 2-bromobenzaldéhyde, on obtient 4,33 g (92%) du composé attendu, sous la forme d'une huile jaune.
¹H NMR (CDCl₃) δ 5,24 (d, 1H, *J* = 10,9 Hz), 5,75 (dd, 1H, *J* = 18,2 / 0,6 Hz), 6,69 (dd, 1H, *J* = 17,6 10,9 Hz), 7,26 (d, 2H, *J =* 8,2 Hz), 7,31 à 7,58 (m, 2H), 7,42 (d, 2H, *J.=* 8,2 Hz), 7,53 (dd, 1H, *J* = 7,5 / 1,4 Hz), 7,94 (dd, 1H, *J* = 7,7 */* 1,3 Hz), 9,91 (s, 1H).

### (b) 2-((E)-2-iodovinyl)-4'-vinyl-biphényle.

De manière analogue à l'exemple 1(d), à partir de 2,50 g (12,0 mmoles) de 4'-vinyl-biphényl-2-carboxaldéhyde obtenu à l'exemple 27(a), on obtient 2,08 g (52%) du composé attendu, sous la forme d'une huile jaune.
¹H NMR (CDCl₃) δ 5,30 (d, 1H, *J* = 10,9 Hz), 5,82 (d, 1H, *J* = 17,6 Hz), 6,69 à 6,83 (m, 1H), 6,75 (d, 1H, *J=* 14,7 Hz), 7,24 à 7,50 (m, 9H).

### (c) 4-[4-(4'-vinyl-biphényl-2-yl)-but-3-èn-(E)-1-ynyl]-benzoate de méthyle.

De manière analogue à l'exemple 1(e), par réaction de 2,08 g (6,3 mmoles) de 2-((E)-2-iodovinyl)-4'-vinyl-biphényle obtenu à l'exemple 27(b) avec 912 mg (5,9 mmoles) de 4-éthynyl benzoate de méthyle, on obtient 960 mg (45%) du composé attendu, sous la forme d'une poudre jaune clair de point de fusion 136°C.
¹H NMR (CDCl₃) δ 3,91 (s, 3H), 5,31 (d, 1H, *J* = 11,0 Hz), 5,83 (d, 1H, *J =* 17,2 Hz), 6,35 (d, 1H, *J* = 16,2 Hz), 6,79 (dd, 1H, *J* = 17,6 / 10,9 Hz), 7,09 (d, 1H, *J* = 16,2 Hz), 7,31 à 7,39 (m, 5H), 7,46 à 7,52 (m, 4H), 7,64 à 7,68 (m, 1H), 7,97 (d, 2H, *J* = 8,4 Hz).

### (d) acide 4-[4-(4'-vinyl-biphényl-2-yl)-but-3-èn-(E)-1-ynyl]-benzoïque.

De manière analogue à l'exemple 1(f), à partir de 960 mg (26,3 mmoles) de l'ester méthylique obtenu à l'exemple 27(c), on obtient 910 mg (98%) d'acide 4-[4-(4'-vinyl-biphényl-2-yl)-but-3-èn-(E)-1-ynyl]-benzoïque, sous la forme d'un solide jaune de point de fusion 254-257°C.
¹H NMR (CDCl₃ + 2 gouttes de DMSO D₆) δ 4,67 (d, 1H, *J* = 10,9 Hz), 5,20 (d, 1H, *J* = 17,6 Hz), 5,77 (d, 1H, *J* = 16,2 Hz), 6,14 (dd, 1H, *J* = 17,6 / 10,9 Hz), 6,39 (d, 1H, *J* = 16,2 Hz), 6,66 à 6,76 (m, 4H), 6,81 à 6,84 (m, 3H), 6,88 (d, 2H, *J* = 8,1 Hz), 7,04 à 7,08 (m, 1H), 7,29 (d, 2H, *J* = 8,2 Hz).

### EXEMPLE 28

### Acide 4-[4-(4'-méthoxyméthoxy-biphényl-2-yl)-but-3-èn-(E)-1-ynyl]-benzoïque.

### (a) 4-méthoxyméthoxybromobenzène.

Dans un tricol, on introduit 48,84 g (282,3 mmoles) de 4-bromophénol, 150 ml de THF et 150 ml de DMF. On refroidit la solution obtenue à 0°C, ajoute par petites fractions 11,74 g (366,9 mmoles) d'hydrure de sodium à 75% et agite à 0°C pendant une heure. On ajoute goutte à goutte 25,0 ml (310,5 mmoles) d'éther méthylique de chlorométhyle et agite le milieu réactionnel pendant deux heures à la température ambiante. On verse le milieu réactionnel dans un mélange HCI 1N / acétate d'éthyle, extrait par de l'acétate d'éthyle, décante la phase organique, sèche sur sulfate de magnésium. Après évaporation des solvants, on recueille 63,85 g (100 %) du composé attendu, sous la forme d'une huile beige.
¹H NMR (CDCl₃) δ 3,46 (s, 3H), 5,14 (s, 2H), 6,92 (d, 2H, *J* = 9,0 Hz), 7,57 (d, 2H, *J* = 9,0 Hz).

### (b) acide 4-méthoxyméthoxybenzène boronique.

De manière analogue à l'exemple 2(a), à partir de 63,81 g (293,0 mmoles) de 4-méthoxyméthoxybromobenzène obtenu à l'exemple 28(a), on obtient 35,42 g (80%) du composé attendu; sous la forme d'une poudre blanche de point de fusion 122°C.
¹H NMR (CDCl₃) δ 3,52 (s, 3H), 5,27 (s, 2H), 7,14 (d, 2H, *J* = 8,6 Hz), 8,16 (d, 2H, *J* = 8,6 Hz).

### (c) 4'-méthoxyméthoxy-biphényl-2-carboxaldéhyde.

De manière analogue à l'exemple 21(a), à partir de 14,00 g (9,3 mmoles) d'acide 4-méthoxyméthoxybenzène boronique obtenu à l'exemple 28(b) et 11,56 g (6,2 mmoles) de 2-bromobenzaldéhyde, on obtient 13,10 g (87%) du composé attendu, sous la forme d'une huile jaune.
¹H NMR (CDCl₃) δ 3,51 (s, 3H), 5,24 (s, 2H), 7,14 (d, 2H, *J* = 8,7 Hz), 7,30 (d, 2H, *J* = 8,7 Hz), 7,40 à 7,49 (m, 2H), 7,60 (dd, 1H, *J* = 7,5/1,5 Hz), 8,00 (dd, 1H, *J* = 7,6 / 1,1 Hz), 10,00 (d, 1H, *J* = 0,6 Hz).

### (d) 2-((E)-2-iodovinyl)-4'-méthoxyméthoxy-biphényle.

De manière analogue à l'exemple 1(d), à partir de 13,10 g (54,0 mmoles) de 4'-méthoxyméthoxy-biphényl-2-carboxaldéhyde obtenu à l'exemple 28(c), on obtient 5,51 g (28%) du composé attendu, sous la forme d'une huile jaune.
¹H NMR (CDCl₃) δ 3,54 (s, 3H), 5,24 (s, 2H), 6,73 (d, 1H, *J* = 14,8 Hz), 7,04 à 7,12 (m, 3H), 7,22 à 7,44 (m, 6H).

### (e) 4-[4-(4'-méthoxyméthoxy-biphényl-2-yl)-but-3-èn-(E)-1-ynyl]-benzoate de méthyle.

De manière analogue à l'exemple 1(e), par réaction de 5,51 g (15,1 mmoles) de 2-(2-iodovinyl)-4'-méthoxyméthoxy-biphényle obtenu à l'exemple 28(d) avec 2,19 g (13,7 mmoles) de 4-éthynyl benzoate de méthyle, on obtient 3,42 g (63%) du composé attendu, sous la forme d'une poudre jaune de point de fusion 76-80°C.
¹H NMR (CDCl₃) δ 3,54 (s, 3H), 3,91 (s, 3H), 5,24 (s, 2H), 6,34 (d, 1H, *J* = 16,2 Hz), 7,08 à 7,14 (m, 2H), 7,26 à 7,37 (m, 5H), 7,49 (d, 2H, *J* = 8,4 Hz), 7,97 (d, 2H, *J* = 8,3 Hz).

### (f) acide 4-[4-(4'-méthoxyméthoxy-biphényl-2-yl)-but-3-èn-(E)-1-ynyl]-benzoïque.

De manière analogue à l'exemple 1(f), à partir de 850 mg (2,13 mmoles) de l'ester méthylique obtenu à l'exemple 28(e), on obtient 490 mg (60%) d'acide 4-[4-(4'-méthoxyméthoxy-biphényl-2-yl)-but-3-èn-(E)-1-ynyl]-benzoïque, sous la forme d'une poudre jaune de point de fusion 209-213°C.
¹H NMR (CDCl₃ + 2 gouttes de DMSO D₆) δ 3,30 (s, 3H), 5,01 (s, 2H), 6,13 (d, 1H, *J* = 16,2 Hz), 6,85 (d, 1H, *J* = 16,0 Hz), 6,89 (d, 2H, *J* = 8,4 Hz), 7,04 (d, 2H, *J* = 8,6 Hz), 7,08 à 7,13 (m, 3H), 7,25 (d, 2H, *J* = 8,2 Hz), 7,26 à 7,28 (m, 1H), 7,41 à 7,43 (m, 1H), 7,74 (d, 2H, *J* = 8,2 Hz).

### EXEMPLE 29

### Acide 4-[4-(3'-méthoxyméthoxy-biphényl-2-yl)-but-3-èn-(E)-1-ynyl]-benzoïque.

### (a) 3-méthoxyméthoxybromobenzène.

De manière analogue à l'exemple 28(a), à partir de 48,84 g (282,3 mmoles) de 3-bromophénol et de 25,00 g (310,5 mmoles) d'éther méthylique de chlorométhyle, on obtient 65,00 g (100%) du composé attendu, sous la forme d'une huile jaune.
¹H NMR (CDCl₃) δ 3,48 (s, 3H), 5,15 (s, 2H), 6,92 à 7,00 (m, 2H), 7,10 à 7,18 (m, 4H), 7,18 à 7,22 (m, 2H).

### (b) acide 3-méthoxyméthoxybenzène boronique.

De manière analogue à l'exemple 2(a), à partir de 65,00 g (299,4 mmoles) de 3-méthoxyméthoxybromobenzène obtenu à l'exemple 29(a), on obtient 30,20 g (67%) du composé attendu, sous la forme d'une poudre beige de point de fusion 45°C.
¹H NMR (CDCl₃) δ 3,54 (s, 3H), 5,28 (s, 2H), 7,26 à 7,54 (m, 3H), 7,86 à 7,90 (m, 1H).

### (c) 3'-méthoxyméthoxy-biphényl-2-carboxaldéhyde.

De manière analogue à l'exemple 21(a), à partir de 15,00 g (100,0 mmoles) d'acide 3-méthoxyméthoxybenzène boronique obtenu à l'exemple 29(b) et 12,33 g (66,6 mmoles) de 2-bromobenzaldéhyde, on obtient 7,60 g (31%) du composé attendu, sous la forme d'une huile incolore.
¹H NMR (CDCl₃) δ 3,51 (s, 3H), 5,22 (s, 2H), 7,01 (d, 1H, *J* = 7,5 Hz), 7,07 à 7,14 (m, 2H), 7,36 (d, 1H, *J* = 7,8 Hz), 7,41 à 7,53 (m, 2H), 7,62 (dd, 1H, *J* = 7,5 / 1,5 Hz), 8,02 (dd, 1H, *J* = 7,7 / 1,2 Hz), 10,01 (s, 1H).

### (d) 2-((E)-2-iodovinyl)-3'-méthoxyméthoxy-biphényle.

De manière analogue à l'exemple 1(d), à partir de 7,60 g (31,4 mmoles) de 3'-méthoxyméthoxy-biphényl-2-carboxaldéhyde obtenu à l'exemple 29(c), on obtient 4,25 g (37%) du composé attendu, sous la forme d'une huile brune.
¹H NMR (CDCl₃) δ 3,51 (s, 3H), 5,22 (s, 2H), 6,73 (d, 1H, *J* = 14,8 Hz). 6,95 à 7,07 (m, 3H), 7,30 à 7,43 (m, 5H).

### (e) 4-[4-(3'-méthoxyméthoxy-biphényl-2-yl)-but-3-èn-(E)-1-ynyl]-benzoate de méthyle.

De manière analogue à l'exemple 11(f), par réaction de 6,96 g (17,5 mmoles) de 2-((E)-2-iodovinyl)-3'-méthoxyméthoxy-biphényle obtenu à l'exemple 29(d) avec 5,59 g (34,9 mmoles) de 4-éthynyl benzoate de méthyle, on obtient 1,84 g (25%) du composé attendu, sous la forme d'une poudre jaune clair de point de fusion 102°C.
¹H NMR (CDCl₃) δ 3,50 (s, 3H), 3,91 (s, 3H), 5,22 (s, 2H), 6,98 à 7,09 (m, 4H), 7,35 à 7,40 (m, 4H), 7,47 (d, 2H, *J* = 8,4 Hz), 7,64 à 7,67 (m, 1H), 7,97 (d, 2H, *J* = 8,4 Hz).

### (f) acide 4-[4-(3'-méthoxyméthoxy-biphényl-2-yl)-but-3-én-(E)-1-ynyl]-benzoïque.

De manière analogue à l'exemple 1(f), à partir de 450 mg (1,2 mmole) de l'ester méthylique obtenu à l'exemple 29(e), on obtient 420 mg (93%) d'acide 4-[4-(3'-méthoxyméthoxy-biphényl-2-yl)-but-3-èn-(E)-1-ynyl]-benzoïque, sous la forme d'une poudre beige de point de fusion 213°C.
¹H NMR (CDCl₃ + 2 gouttes de DMSO D₆) δ 3,50 (s, 3H), 5,23 (s, 2H), 6,36 (d, 1H, *J* = 16,2 Hz), 6,98 à 7,08 (m, 3H), 7,09 (d, 1H, *J* = 16,3 Hz), 7,34 à 7,42 (m, 4H), 7,46 (d, 2H, *J* = 8,3 Hz), 7,65 à 7,68 (m, 1H), 7,98 (d, 2H, *J* = 8,3 Hz).

### EXEMPLE 30

### Acide 4-[4-(2-thiophène-3-yl-phényl)-but-3-èn-(E)-1-ynyl]-benzoïque.

### (a) 2-thiophène-3-yl-benzaldéhyde.

De manière analogue à l'exemple 21(a), à partir de 5,15 g (40,2 mmoles) d'acide 3-thiophèneboronique et 4,96 g (26,8 mmoles) de 2-bromobenzaldéhyde, on obtient 4,08 g (81%) du composé attendu, sous la forme d'une huile jaune.
¹H NMR (CDCl₃) à 7,18 à 7,20 (m, 1H), 7,28 à 7,30 (m, 1H), 7,44 à 7,50 (m, 3H), 7,60 (dd, 1H, *J* = 7,0 / 1,5 Hz), 8,00 (dd, 1H, *J* = 6,9/1,6 Hz), 10,11 (s, 1H).

### (b) 2-((E)-2-iodovinyl)-2-thiophène-3-yl-benzène.

De manière analogue à l'exemple 1(d), à partir de 2,50 g (13,3 mmoles) de 2-thiophène-3-yl-benzaldéhyde obtenu à l'exemple 30(a), on obtient 2,74 g (66%) du composé attendu, sous la forme d'une huile orangée.
¹H NMR (CDCl₃) δ 6,74 (d, 1H, *J* = 14,8 Hz), 7,14 à 7,44 (m, 7H), 7,50 (d, 1H, *J* = 14,8 Hz).

### (c) 4-[4-(2-thiophène-3-yl-phényl)-but-3-èn-(E)-1-ynyl]-benzoate de methyle.

De manière analogue à l'exemple 1(e), par réaction de 2,74 g (8,8 mmoles) de 2-(2-iodovinyl)-2-thiophène-3-yl-benzène obtenu à l'exemple 30(b) avec 1,41 g (8,8 mmoles) de 4-éthynyl benzoate de méthyle, on obtient 950 mg (31%) du composé attendu, sous la forme d'une poudre beige de point de fusion 89-91°C.
¹H NMR (CDCl₃) δ 3,91 (s, 3H), 6,33 (d, 1H, *J* = 16,2 Hz), 7,17 à 7,36 (m, 7H), 7,49 (d, 2H, *J* = 8,3 Hz), 7,61 à 7,63 (m, 1H), 7,97 (d, 2H, *J* = 8,3 Hz).

### (d) acide 4-[4-(2-thiophène-3-yl-phényl)-but-3-èn-(E)-1-ynyl]-benzoïque.

De manière analogue à l'exemple 1(f), à partir de 950 mg (2,8 mmoles) de l'ester méthylique obtenu à l'exemple 30(c), on obtient 880 mg (96%) d'acide 4-[4-(2-thiophène-3-yl-phényl)-but-3-èn-(E)-1-ynyl]-benzoïque, sous la forme d'une poudre jaune de point de fusion 218°C.
¹H NMR (CDCl₃ + 2 gouttes de DMSO D₆) δ 6,38 (d, 1H, *J* = 16,2 Hz), 6,99 (d, 1H, *J* = 16,2 Hz), 7,22 à 7,51 (m, 6H), 7,48 (d, 2H, *J* = 8,3 Hz), 7,66 à 7,70 m, 1H), 7,97 (d, 2H, *J* = 8,3 Hz).

### EXEMPLE 31

### Acide 4-[4-(2-thiophène-2-yl-phényl)-but-3-èn-(E)-1-ynyl]-benzoïque.

### (a) 2-thiophène-2-yl-benzaldéhyde.

De manière analogue à l'exemple 21(a), à partir de 4,19 g (32,7 mmoles) d'acide 2-thiophèneboronique et 4,04 g (21,8 mmoles) de 2-bromobenzaldéhyde, on obtient 3,65 g (89%) du composé attendu, sous la forme d'une huile jaune.
¹H NMR (CDCl₃) à 7,07 à 7,09 (m, 1H), 7,14 à 7,17 (m, 1H), 7,46 à 7,57 (m, 3H), 7,61 (dd, 1H, *J* = 7,0/1,3 Hz), 8,01 (dd, 1H, *J =* 7,7/1,4 Hz), 10,19 (d, 1H, *J* = 0,7 Hz).

### (b) 2-((E)-2-iodovinyl)-2-thiophène-2-yl-benzène.

De manière analogue à l'exemple 1(d), à partir de 2,50 g (13,3 mmoles) de 2-thiophène-2-yl-benzaldéhyde obtenu à l'exemple 31(a), on obtient 3,17 g (76%) du composé attendu, sous la forme d'une huile marron qui sera utilisée directement pour l'étape suivante.

### (c) 4-[4-(2-thiophène-2-yl-phényl)-but-3-èn-(E)-1-ynyl]-benzoate de methyle.

De manière analogue à l'exemple 1(e), par réaction de 3,17 g (10,2 mmoles) de 2-(2-iodovinyl)-2-thiophène-2-yl-benzène obtenu à l'exemple 31(b) avec 1,63 g (10,2 mmoles) de 4-éthynyl benzoate de méthyle, on obtient 555 mg (16%) du composé attendu, sous la forme d'un solide jaune de point de fusion 100°C.
¹H NMR (CDCl₃) δ 3,91 (s, 3H), 6,35 (d, 1H, *J =* 15,2 Hz), 7,07 (d, 1H, *J =* 1,2 Hz), 7,08 à 7,14 (m, 1H), 7,30 à 7,46 (m, 5H), 7,50 (d, 2H, *J* = 8,3 Hz), 7,61 à 7,63 (m, 1H), 7,98 (d, 2H, *J* = 8,3 Hz).

### (d) acide 4-[4-(2-thiophène-2-yl-phényl)-but-3-èn-(E)-1-ynyl]-benzoïque.

De manière analogue à l'exemple 1(f), à partir de 550 mg (1,6 mmole) de l'ester méthylique obtenu à l'exemple 31(c), on obtient 530 mg (100%) d'acide 4-[4-(2-thiophène-2-yl-phényl)-but-3-èn-(E)-1-ynyl]-benzoïque, sous la forme d'une poudre jaune de point de fusion 221°C.
¹H NMR (CDCl₃ + 2 gouttes de DMSO D₆) δ 6,37 (d, 1H, *J* = 16,2 H2), 7,11 (s, 1H, *J* = 16,2 Hz), 7,08 à 7,17 (m, 1H), 7,34 à 7,47 (m, 5H), 7,50 (d, 2H, *J* = 8,4 Hz), 7,62 à 7,66 (m, 1H), 8,00 (d, 2H, *J* = 8,4 Hz).

### EXEMPLE 32

### Acide 4-[4-(4'-hydroxy-biphényl-2-yl)-but-3-èn-(E)-1-ynyl]-benzoïque.

### (a) 4-[4-(4'-hydroxy-biphényl-2-yl)-but-3-èn-(E)-1-ynyl]-benzoate de méthyle.

Dans un tricol de 100 ml, et sous courant d'azote, on introduit 1,95 g (4,9 mmoles) du composé obtenu à l'exemple 28(e), 20 ml de méthanol et 20 ml de THF. On ajoute goutte à goutte 2,62 ml (48,9 mmoles) d'acide sulfurique concentré en solution dans 20 ml de méthanol. Le milieu réactionnel est agité pendant seize heures à la température ambiante, puis on ajoute de l'eau, extrait par de l'éther éthylique, lave la phase organique à l'eau jusqu'à pH neutre, sèche sur sulfate de magnésium, filtre et évapore les solvants. Le résidu obtenu est purifié par chromatographie sur colonne de silice, élué par de l'heptane, puis par un mélange composé de 20% d'acétate d'éthyle et 80% d'heptane. Après évaporation des solvants, on recueille 1,50 g (86%) du composé attendu, sous la forme d'une poudre beige de point de fusion 130°C.
¹H NMR (CDCl₃) δ 3,92 (s, 3H), 4,96 (s, 1H), 6,34 (d, 1H, *J* = 16,2 Hz), 6,93 (d, 2H, *J* = 8,6 Hz), 7,09 (d, 1H, *J* = 16,3 Hz), 7,24 (d, 2H, *J* = 8,6 Hz), 7,26 à 7,37 (m, 3H), 7,48 (d, 2H, *J* = 8,4 Hz), 7,62 à 7,66 (m, 1H), 7,98 (d, 2H, *J* = 8,3 Hz).

### (b) acide 4-[4-(4'-hydroxy-biphényl-2-yl)-but-3-èn-(E)-1-ynyl]-benzoïque.

De manière analogue à l'exemple 1(f), à partir de 660 mg (1,9 mmole) de l'ester méthylique obtenu à l'exemple 32(a), on obtient 610 mg (96%) d'acide 4-[4-(4'-hydroxy-biphényl-2-yl)-but-3-èn-(E)-1-ynyl]-benzoïque, sous la forme d'une poudre beige de point de fusion 235°C.
¹H NMR (CDCl₃ + 2 gouttes de DMSO D₆) δ 6,27 (d, 1H, *J* = 16,2 Hz), 6,83 (d, 2H, *J* = 8,5 Hz), 7,00 (d, 1H, *J* = 16,3 Hz), 7,07 (d, 2H, *J* = 8,5 Hz), 7,18 à 7,26 (m, 3H), 7,39 (d, 2H, *J* = 8,3 Hz), 7,54 à 7,58 (m, 1H), 7,88 (d, 2H, *J* = 8,3 Hz), 9,10 (s, 1H).

### EXEMPLE 33

### Acide 4-[4-(4'-méthoxy-biphényl-2-yl)-but-3-èn-(E)-1-ynyl]-benzoïque.

### (a) 4-[4-(4'-méthoxy-biphényl-2-yl)-but-3-èn-(E)-1-ynyl]-benzoate de méthyle.

Dans un tricol de 100 ml, et sous courant d'azote, on introduit 420 mg (1,2 mmole) du composé obtenu à l'exemple 32(a), 20 ml de DMF et 20 ml de THF. On refroidit et ajoute par petites fractions 49 mg (1,5 mmole) d'hydrure de sodium à 75% dans l'huile. Le milieu réactionnel est agité pendant quarante minutes à la température ambiante, puis on ajoute goutte à goutte une solution composée de 81 µl (1,3 mmole) d'iodure de méthyle en solution dans 5 ml de DMF. Le milieu réactionnel est agité pendant trois heures à la température ambiante, puis on ajoute de l'eau, extrait par de l'éther éthylique, lave la phase organique à l'eau, sèche sur sulfate de magnésium, filtre et évapore les solvants. On recueille 470 mg (100%) du composé attendu, sous la forme d'une poudre jaune de point de fusion 190°C.
¹H NMR (CDCl₃) δ 3,88 (s, 3H), 3,91 (s, 3H), 6,35 (d, 1H, *J* = 16,2 Hz), 7,00 (d, 2H, *J* = 8,7 Hz), 7,08 (d, 1H, *J* = 16,3 Hz), 7,28 (d, 2H, *J* = 8,6 Hz), 7,32 à 7,38 (m, 2H), 7,43 à 7,50 (m, 1H), 7,47 (d, 2H, *J* = 8,4 Hz), 7,63 à 7,70 (m, 1H), 7,97 (d, 2H, *J* = 8,7 Hz).

### (b) acide 4-[4-(4'-méthoxy-biphényl-2-yl)-but-3-èn-(E)-1 -ynyl]-benzoïque.

De manière analogue à l'exemple 1(f), à partir de 430 mg (1,2 mmole) de l'ester méthylique obtenu à l'exemple 33(a), on obtient 390 mg (94%) d'acide 4-[4-(4'-méthoxy-biphényl-2-yl)-but-3-èn-(E)-1-ynyl]-benzoïque, sous la forme d'une poudre jaune de point de fusion 253°C.
¹H NMR (CDCl₃ + 2 gouttes de DMSO D₆) δ 3,88 (s, 3H), 6,35 (d, 1H, *J* = 16,2 Hz), 7,00 (d, 2H, *J* = 8,7 Hz), 7,08 (d, 1H, *J* = 16,2 Hz), 7,28 (d, 2H, *J* = 8,8 Hz), 7,32 à 7,40 (m, 3H), 7,47 (d, 2H, *J* = 8,3 Hz), 7,63 à 7,67 (m, 1H), 7,98 (d, 2H, *J* = 8,3 Hz).

### EXEMPLE 34

### Acide 4-[4-(4'-propoxy-biphényl-2-yl)-but-3-èn-(E)-1-ynyl]-benzoïque.

### (a) 4-[4-(4'-propoxy-biphényl-2-yl)-but-3-èn-(E)-1-ynyl]-benzoate de méthyle.

De manière analogue à l'exemple 33(a), à partir de 420 mg (1,2 mmole) du composé obtenu à l'exemple 32(a) et de 127 µl (1,3 mmole) d'iodure de n.propyle, on obtient 460 mg (98%) du composé attendu, sous la forme d'une poudre beige de point de fusion 110-113°C.
¹H NMR (CDCl₃) δ 1,07 (t, 3H, *J* = 7,5 Hz), 1,85 (q, 2H, *J* = 7,2 Hz), 3,91 (s, 3H), 3,99 (t, 2H, J = 6,5 Hz), 6,34 (d, 1H, *J* = 16,2 Hz), 6,92 à 7,00 (m, 3H), 7,11 (d, 1H, *J* = 16,2 Hz), 7,29 à 7,35 (m, 4H), 7,48 (d, 2H, *J* = 8,3 Hz), 7,62 à 7,69 (m, 1H), 7,97 (d, 2H, *J* = 8,4 Hz).

### (b) acide 4-[4-(4'-propoxy-biphényl-2-yl)-but-3-èn-(E)-1-ynyl]-benzoïque.

De manière analogue à l'exemple 1(f), à partir de 460 mg (1,2 mmole) de l'ester méthylique obtenu à l'exemple 34(a), on obtient 400 mg (90%) d'acide 4-[4-(4'-propoxy-biphényl-2-yl)-but-3-èn-(E)-1-ynyl]-benzoïque, sous la forme d'une poudre jaune de point de fusion 235°C.
¹H NMR (CDCl₃ + 2 gouttes de DMSO D₆) δ 1,07 (t, 3H, *J* = 7,4 Hz), 1,85 (m, 2H, *J* = 7,0 Hz), 3,99 (t, 2H, *J* = 6,5 Hz), 6,35 (d, 1H, *J* = 16,2 Hz), 6,99 (d, 2H, *J* = 8,7 Hz), 7,09 (d, 1H, *J* = 16,2 Hz), 7,27 (d, 2H, *J* = 8,6 Hz), 7,32 à 7,38 (m, 3H), 7,47 (d, 2H, *J* = 8,3 Hz), 7,63 à 7,66 (m, 1H), 7,98 (d, 2H, *J* = 8,3 Hz).

### EXEMPLE 35

### Acide 4-[4-(3'-hydroxy-biphényl-2-yl)-but-3-èn-(E)-1-ynyl]-benzoïque.

### (a) 4-[4-(3'-hydroxy-biphényl-2-yl)-but-3-èn-(E)-1-ynyl]-benzoate de méthyle.

De manière analogue à l'exemple 32(a) à partir de 1,39 g (3,5 mmoles) de l'ester méthylique obtenu à l'exemple 32(a), on obtient 1,17 g (95%) du composé attendu, sous la forme d'une poudre beige de point de fusion 120-125°C.
¹H NMR (CDCl₃) δ 3,91 (s, 3H), 5,02 (s, 1H), 6,35 (d, 1H, *J* = 16,2 Hz), 6,82 à 6,93 (m, 3H), 7,10 (d, 1H, *J* = 16,2 Hz), 7,30 à 7,39 (m, 4H), 7,48 (d, 2H, *J* = 8,5 Hz), 7,64 à 7,67 (m, 1H), 7,97 (d, 2H, *J* = 8,5 Hz).

### (b) acide 4-[4-(3'-hydroxy-biphényl-2-yl)-but-3-èn-(E)-1-ynyl]-benzoïque.

De manière analogue à l'exemple 1(f) à partir de 450 mg (1,3 mmole) de l'ester méthylique obtenu à l'exemple 35(a), on obtient 420 mg (97%) d'acide 4-[4-(3'-hydroxy-biphényl-2-yl)-but-3-èn-(E)-1-ynyl]-benzoïque, sous la forme d'une poudre blanche de point de fusion 219°C.
¹H NMR (CDCl₃ + 2 gouttes de DMSO D₆) δ 6,34 (d, 1H, *J* = 16,2 Hz), 6,80 à 6,91 (m, 3H), 7,13 (d, 1H, *J* = 16,3 Hz), 7,24 à 7,37 (m, 4H), 7,47 (d, 2H, *J* = 8,3 Hz), 7,63 à 7,65 (m, 1H), 7,99 (d, 2H, *J* = 8,3 Hz).

### EXEMPLE 36

### Acide 4-[4-(3'-méthoxy-biphényl-2-yl)-but-3-èn-(E)-1-ynyl]-benzoïque.

### (a) 4-[4-(3'-méthoxy-biphényl-2-yl)-but-3-èn-(E)-1-ynyl]-benzoate de méthyle.

De manière analogue à l'exemple 33(a), à partir de 360 mg (1,0 mmole) du composé obtenu à l'exemple 35(a) et 70 µl (1,1 mmole) d'iodure de méthyle, on obtient 370 mg (100%) du composé attendu, sous la forme d'une poudre blanche de point de fusion 105°C.
¹H NMR (CDCl₃) δ 3,85 (s, 3H), 3,91 (s, 3H), 6,35 (d, 1H, *J* = 16,2 Hz), 6,89 à 6,96 (m, 3H), 7,10 (d, 1H, *J* = 16,2 Hz), 7,34 à 7,39 (m, 4H), 7,48 (d, 2H, *J* = 3,4 Hz), 7,62 à 7,64 (m, 1H), 7,97 (d, 2H, *J* = 8,4 Hz).

### (b) acide 4-[4-(3'-méthoxy-biphényl-2-yl)-but-3-èn-(E)-1-ynyl]-benzoïque.

De manière analogue à l'exemple 1(f), à partir de 370 mg (1,0 mmole) de l'ester méthylique obtenu à l'exemple 36(a), on obtient 330 mg (92%) d'acide 4-[4-(3'-méthoxy-biphényl-2-yl)-but-3-èn-(E)-1-ynyl]-benzoïque, sous la forme d'une poudre beige de point de fusion 214°C.
¹H NMR (CDCl₃ + 2 gouttes de DMSO D₆) δ 3,85 (s, 3H), 6,35 (d, 1H *J* = 16,2 Hz), 6,88 à 6,97 (m, 3H), 7,08 (d, 1H, *J* = 16,2 Hz), 7,30 à 7,40 (m, 4H), 7,47 (d, 2H, *J* = 8,3 Hz), 7,65 à 7,68 (m, 1H), 7,98 (d, 2H, *J* = 8,2 Hz).

### EXEMPLE 37

### Acide 4-[4-(3'-propoxy-biphényl-2-yl)-but-3-èn-(E)-1-ynyl]-benzoïque.

### (a) 4-[4-(3'-propoxy-biphényl-2-yl)-but-3-èn-(E)-1-ynyl]-benzoate de méthyle.

De manière analogue à l'exemple 33(a), à partir de 360 mg (1,0 mmole) du composé obtenu à l'exemple 35(a) et de 109 µl (1,1 mmole) d'iodure de n.propyle, on obtient 400 mg (99%) du composé attendu, sous la forme d'une huile jaune.
¹H NMR (CDCl₃) δ 1,82 (q, 2H, *J* = 7,1 Hz), 3,91 (s, 3H), 3,97 (t, 3H, *J* = 6,6 Hz), 6,34 (d, 1H, *J* = 16,2 Hz), 6,87 à 6,95 (m, 3H), 7,11 (d, 1H, *J* = 16,2 Hz), 7,31 à 7, 39 (m, 4H), 7,48 (d, 2H, *J* = 8,4 Hz), 7,64 à 7,67 (m, 1H), 7,97 (d, 2H, *J* = 8,4 Hz).

### (b) acide 4-[4-(3'-propoxy-biphényl-2-yl)-but-3-èn-(E)-1-ynyl]-benzoïque.

De manière analogue à l'exemple 1(f), à partir de 400 mg (1,0 mmole) de l'ester méthylique obtenu à l'exemple 37(a), on obtient 330 mg (86%) d'acide 4-[4-(3'-propoxy-biphényl-2-yl)-but-3-èn-(E)-1-ynyl]-benzoïque, sous la forme d'une poudre jaune clair de point de fusion 177°C.
¹H NMR (CDCl₃ + 2 gouttes de DMSO D₆) δ 1,03 (t, 3H, *J* = 7,4 Hz), 1,83 (m, 2H, *J* = 7,1 Hz), 3,97 (t, 2H, *J* = 6,6 Hz), 6,35 (d, 1H, *J =* 16,2 Hz), 6,89 à 6,95 (m, 3H), 7,10 (d, 1H, *J* = 16,2 Hz), 7,30 à 7,38 (m, 4H), 7,47 (d, 2H, *J* = 8,2 Hz), 7,64 à 7,67 (m, 1H), 7,99 (d, 2H, *J* = 8,3 Hz).

### EXEMPLE 38

### Acide 4-[4-(4'-méthyl-4-hydroxy-biphényl-2-yl)-but-3-èn-(E)-1-ynyl]-benzoïque.

### (a) 4-[4-(4'-méthyl-4-hydroxy-biphényl-2-yl)-but-3-èn-(E)-1-ynyl]-benzoate de méthyle.

De manière analogue à l'exemple 32(a), à partir de 700 mg (1,7 mmole) de l'ester méthylique obtenu à l'exemple 15(e), on obtient 630 mg (100%) du composé attendu, sous la forme d'une poudre jaune de point de fusion 205°C.
¹H NMR (CDCl₃) δ 2,41 (s, 3H), 3,91 (s, 3H), 6,30 (d, 1H, *J* = 16,1 Hz), 6,89 (dd, 1H, *J* = 8,4 / 2,4 Hz), 7,14 à 7,30 (m, 6H), 7,47 (d, 2H, *J =* 8,4 Hz), 7,97 (d, 2H, *J* = 8,4 Hz), 8,59 (s, 1H).

### (b) acide 4-[4-(4'-méthyl-4-hydroxy-biphényl-2-yl)-but-3-èn-(E)-1-ynyl]-benzoïque.

De manière analogue à l'exemple 1(f), à partir de 270 mg (0,7 mmole) de l'ester méthylique obtenu à l'exemple 38(a), on obtient 210 mg (85%) d'acide 4-[4-(4'-méthyl-4-hydroxy-biphényl-2-yl)-but-3-èn-(E)-1-ynyl]-benzoïque, sous la forme d'une poudre jaune de point de fusion 285°C.
¹H NMR (CDCl₃ + 2 gouttes de DMSO D₆) δ 2,32 (s, 3H), 6,24 (d, 1H *J* = 16,2 Hz), 6,77 (dd, 1H, *J* = 8,3 / 2,2 Hz), 6,91 (d, 1H, *J* = 16,2 Hz), 7,01 à 7,05 (m, 2H), 7,09 (d, 2H, *J* = 8,1 Hz), 7,16 (d, 2H, *J* = 8,0 Hz), 7,39 (d, 2H, *J* = 8,3 Hz), 7,86 (d, 2H, *J* = 8,3 Hz), 9,29 (br s, 1H).

### EXEMPLE 39

### Acide 4-[4-(4'-méthyl-4-méthoxy-biphényl-2-yl)-but-3-èn-(E)-1-ynyl]-benzoïque.

### (a) 4-[4-(4'-méthyl-4-méthoxy-biphényl-2-yl)-but-3-èn-(E)-1-ynyl]-benzoate de méthyle.

De manière analogue à l'exemple 33(a), à partir de 180 mg (0,49 mmole) du composé obtenu à l'exemple 38(a) et 79 mg (0,56 mmole) d'iodure de méthyle, on obtient 140 mg (75%) du composé attendu, sous la forme d'une poudre jaune de point de fusion 111°C.
¹H NMR (CDCl₃) δ 2,42 (s, 3H), 3,88 (s, 3H), 3,91 (s, 3H), 6,34 (d, 1H, *J* = 16,2 Hz), 6,94 (dd, 1H, *J* = 8,5 / 2,6 Hz), 7,08 (d, 1H, *J* = 16,2 Hz), 7,15 (d, 1H, *J* = 2,6 Hz), 7,19 à 7,26 (m, 5H), 7,47 (d, 2H, *J* = 8,3 Hz), 7,97 (d, 2H, *J* = 8,3 Hz).

### (b) acide 4-[4-(4'-méthyl-4-méthoxy-biphényl-2-yl)-but-3-èn-(E)-1-yny]-benzoïque.

De manière analogue à l'exemple 1(f), à partir de 140 mg (0,37 mmole) de l'ester méthylique obtenu à l'exemple 39(a), on obtient 110 mg (81%) d'acide 4-[4-(4'-méthyl-4-méthoxy-biphényl-2-yl)-but-3-èn-(E)-1-ynyl]-benzoïque, sous la forme d'une poudre jaune de point de fusion 225°C.
¹H NMR (CDCl₃ + 2 gouttes de DMSO D₆) δ 2,37 (s, 3H), 3,85 (s, 3H), 6,72 (d, 1H, *J* = 16,2 Hz), 6,95 (d, 1H, *J* = 16,3 Hz), 7,01 (dd, 1H, *J* = 8,5 / 2,3 Hz), 7,16 à 7,30 (m, 5H), 7,38 (d, 1H, *J* = 2,3 Hz), 7,55 (d, 2H, J = 8,2 Hz), 7,92 (d, 2H, J = 8,2 Hz), 13,14 (br s, 1H).

### EXEMPLE 40

### Acide 4-[4-(4'-méthyl-4-propoxy-biphényl-2-yl)-but-3-èn-(E)-1-ynyl]-benzoïque.

### (a) 4-[4-(4'-méthyl-4-propoxy-biphényl-2-yl)-but-3-èn-(E)-1-ynyl]-benzoate de méthyle.

De manière analogue à l'exemple 33(a), à partir de 180 mg (0,49 mmole) du composé obtenu à l'exemple 38(a) et de 95 mg (0,56 mmole) d'iodure de n.propyle, on obtient 131 mg (65%) du composé attendu, sous la forme d'une poudre jaune de point de fusion 87°C.
¹H NMR (CDCl₃) δ 1,07 (t, 3H, *J* = 7,5 Hz), 1,85 (m, 2H, *J* = 7,2 Hz), 2,42 (s, 3H), 3,91 (s, 3H), 3,99 (t, 2H, *J* = 6,6 Hz), 6,33 (d, 1H, *J* = 16,2 Hz), 6,93 (dd, 1H *J* = 8,5 / 2,6 Hz), 7,08 (d, 1H, *J* = 16,2 Hz), 7,15 (d, 1H, *J* = 2,5 Hz), 7,19 à 7,25 (m, 5H), 7,47 (d, 2H, *J* = 8,3 Hz), 7,97 (d, 2H, *J* = 8,3 Hz).

### (b) acide 4-[4-(4'-méthyl-4-propoxy-biphényl-2-yl)-but-3-èn-(E)-1-ynyl]-benzoïque.

De manière analogue à l'exemple 1(f), à partir de 131 mg (0,32 mmole) de l'ester méthylique obtenu à l'exemple 40(a), on obtient 126 mg (100%) d'acide 4-[4-(4'-méthyl-4-propoxy-biphényl-2-yl)-but-3-èn-(E)-1-ynyl]-benzoïque, sous la forme d'une poudre jaune de point de fusion 181°C.
¹H NMR (CDCl₃) δ 1,07 (t, 3H, *J* = 7,4 Hz), 1,85 (m, 2H, *J* = 7,0 Hz), 2,42 (s, 3H), 3,99 (t, 2H, *J* = 6,5 Hz), 6,34 (d, 1H, *J* = 16,1 Hz), 6,93 (dd, 1H, *J* = 8,4 / 2,5 Hz), 7,10 (d, 1H, *J* = 16,2 Hz), 7,16 (d, 1H, *J* = 2,4 Hz), 7,20 à 7,27 (m, 5H), 7,50 (d, 2H, *J* = 8,3 Hz), 8,04 (d, 2H, *J* = 8,3 Hz).

### EXEMPLE 41

### Acide 4-[4-(4'-méthyl-5-hydroxy-biphényl-2-yl)-but-3-èn-(E)-1-ynyl]-benzoïque.

### (a) 4-[4-(4'-méthyl-5-hydroxy-biphényl-2-yl)-but-3-èn-(E)-1-ynyl]-benzoate de méthyle.

De manière analogue à l'exemple 32(a), à partir de 2,00 g (4,8 mmoles) de l'ester méthylique obtenu à l'exemple 16(e), on obtient 1,57 g (88%) du composé attendu, sous la forme d'une poudre jaune de point de fusion 166°C.
¹H NMR (CDCl₃ + 2 gouttes de DMSO D₆) δ 2,42 (s, 3H), 3,91 (s, 3H), 6,18 (d, 1H, *J* = 16,2 Hz), 6,80 (d, 1H, *J* = 2,5 Hz), 6,86 (d, 1H, *J* = 8,6 Hz), 7,02 (d, 1H, *J* = 16,2 Hz). 7,24 (s, 4H), 7,45 (d, 2H, *J* = 8,4 Hz), 7,53 (d, 1H, *J* = 8,6 Hz), 7,95 (d, 2H, *J* = 8,4 Hz), 8,99 (s, 1H).

### (b) acide 4-[4-(4'-méthyl-5-hydroxy-biphényl-2-yl)-but-3-èn-(E)-1-ynyl]-benzoïque.

De manière analogue à l'exemple 1(f), à partir de 700 mg (1,9 mmole) de l'ester méthylique obtenu à l'exemple 41(a), on obtient 500 mg (75%) d'acide 4-[4-(4'-méthyl-5-hydroxy-biphényl-2-yl)-but-3-èn-(E)-1-ynyl]-benzoïque, sous la forme d'une poudre jaune de point de fusion 230-232°C.
¹H NMR (CDCl₃ + 2 gouttes de DMSO D₆) δ 2,38 (s, 3H), 6,42 (d, 1H, *J* = 16,2 Hz), 6,67 (d, 1H, *J* = 2,4 Hz), 6,83 (dd, 1H, *J* = 8,6 / 2,4 Hz), 6,87 (d, 1H, *J* = 2,4 Hz), 7,19 (d, 2H, *J* = 8,0 Hz), 7,30 (d, 2H, *J* = 7,9 Hz), 7,52 (d, 2H, *J* = 8,3 Hz), 7,71 (d, 1H, *J* = 8,7 Hz), 7,90 (d, 2H, *J* = 8,3 Hz), 9,95 (br s, 1H), 13,10 (br s, 1H).

### EXEMPLE 42

### Acide 4-[4-(4'-méthyl-5-méthoxy-biphényl-2-yl)-but-3-èn-(E)-1-ynyl]-benzoïque.

### (a) 4-[4-(4'-méthyl-5-méthoxy-biphényl-2-yl)-but-3-èn-(E)-1-ynyl]-benzoate de méthyle.

De manière analogue à l'exemple 33(a), à partir de 400 mg (1,1 mmole) du composé obtenu à l'exemple 41(a) et 185 mg (1,3 mmole) d'iodure de méthyle, on obtient 420 mg (100%) du composé attendu, sous la forme d'une poudre jaune de point de fusion 130°C.
¹H NMR (CDCl₃) δ 2,43 (s, 3H), 3,84 (s, 3H), 3,91 (s, 3H), 6,22 (d, 1H, *J* = 16,2 Hz), 6,82 (d, 1H, *J* = 2,7 Hz), 6,91 (dd, 1H, *J* = 8,7 / 2,7 Hz), 7,03 (d, 1H, *J* = 16,2 Hz), 7,26 (s, 4H), 7,46 (d, 2H, *J* = 8,4 Hz), 7,60 (d, 1H, *J* = 8,7 Hz), 7,96 (d, 2H, *J* = 8,4 Hz).

### (b) acide 4-[4-(4'-méthyl-5-méthoxy-biphényl-2-yl)-but-3-èn-(E)-1-ynyl]-benzoïque.

De manière analogue à l'exemple 1(f), à partir de 420 mg (1,1 mmole) de l'ester méthylique obtenu à l'exemple 42(a), on obtient 300 mg (74%) d'acide 4-[4-(4'-méthyl-5-méthoxy-biphényl-2-yl)-but-3-èn-(E)-1-ynyl]-benzoïque, sous la forme d'une poudre jaune de point de fusion 258-260°C.
¹H NMR (DMSO D₆) δ 2,38 (s, 3H), 3,82 (s, 3H), 6,50 (d, 1H, *J* = 16,2 Hz), 6,82 (d, 1H, *J* = 2,5 Hz), 6,90 (d, 1H, *J* = 16,3 Hz), 6,99 (dd, 1H, *J* = 8,8 / 2,4 Hz), 7,23 (d, 2H, *J* = 8,0 Hz), 7,31 (d, 2H, *J* = 7,9 Hz), 7,53 (d, 2H, *J* = 8,2 Hz), 7,81 (d, 1H, *J* = 8,8 Hz), 7,91 (d, 2H, *J* = 8,2 Hz), 13,12 (br s, 1H).

### EXEMPLE 43

### Acide 4-[4-(4'-méthyl-5-propoxy-biphényl-2-yl)-but-3-èn-(E)-1-ynyl]-benzoïque.

### (a) 4-[4-(4'-méthyl-5-propoxy-biphényl-2-yl)-but-3-èn-(E)-1-ynyl]-benzoate de méthyle.

De manière analogue à l'exemple 33(a), à partir de 350 mg (0,95 mmole) du composé obtenu à l'exemple 41(a) et de 194 mg (1,1 mmole) d'iodure de n.propyle, on obtient 410 mg (100%) du composé attendu, sous la forme de cristaux jaunes de point de fusion 107°C.
¹H NMR (CDCl₃) δ 1,04 (t, 3H, *J* = 7,5 Hz), 1,82 (m, 2H, *J* = 7,2 Hz), 2,43 (s, 3H), 3,91 (s, 3H), 3,96 (t, 2H, *J* = 6,6 Hz), 6,21 (d, 1H, *J* = 16,2 Hz), 6,82 (d, 1H, *J* = 2,6 Hz), 6,90 (dd, 1H, *J* = 8,6 / 2,6 Hz), 7,03 (d, 1H, *J* = 16,2 Hz), 7,25 (s, 3H), 7,45 (d, 2H, *J* = 8,4 Hz), 7,59 (d, 1H, *J* = 8,7 Hz), 7,96 (d, 2H, *J* = 9,4 Hz), 8,02 8,05 (m, 1H).

### (b) acide 4-[4-(4'-méthyl-5-propoxy-biphényl-2-yl)-but-3-èn-(E)-1-ynyl]-benzoïque.

De manière analogue à l'exemple 1(f), à partir de 400 mg (0,97 mmole) de l'ester méthylique obtenu à l'exemple 43(a), on obtient 270 mg (71%) d'acide 4-[4-(4'-méthyl-5-propoxy-biphényl-2-yl)-but-3-èn-(E)-1-ynyl]-benzoïque, sous la forme d'une poudre jaune de point de fusion 255-257°C.
¹H NMR (DMSO D₆) δ 0,98 (t, 3H, *J* = 7,4 Hz), 1,73 (m, 2H, *J* = 7,1 Hz), 2,38 (s, 3H), 3,99 (t, 2H, *J* = 6,5 Hz), 6,49 (d, 1H, *J* = 16,2 Hz), 6,80 (d, 1H, *J* = 2,5 Hz), 6,90 (d, 1H, *J* = 16,3 Hz), 6,98 (dd, 1H, *J* = 8,7 / 2,3 Hz), 7,23 (d, 2H, *J* = 8,0 Hz), 7,31 (d, 2H, *J* = 8,0 Hz), 7,52 (d, 2H, *J* = 8,2 Hz), 7,79 (d, 1H, *J* = 8,8 Hz), 7,91 (d, 2H, *J* = 8,3 Hz).

### EXEMPLE 44

### Acide 4-[4-(4'-méthyl-6-hydroxy-biphényl-2-yl)-but-3-èn-(E)-1-ynyl]-benzoïque.

### (a) 4-[4-(4'-méthyl-6-hydroxy-biphényl-2-yl)-but-3-èn-(E)-1-ynyl]-benzoate de méthyle.

De manière analogue à l'exemple 32(a), à partir de 700 mg (1,7 mmole) de l'ester méthylique obtenu à l'exemple 17(f), on obtient 620 mg (99%) du composé attendu, sous la forme d'une poudre beige de point de fusion 110°C.
¹H NMR (CDCl₃) δ 2,46 (s, 3H), 3,91 (s, 3H), 4,92 (s, 1H), 6,29 (d, 1H, *J* = 16,2 Hz), 6,77 (d, 1H, *J* = 16,2 Hz), 6,95 à 6,99 (m, 1H), 7,20 (d, 2H, *J* = 8,0 Hz), 7,25 à 7,27 (m, 2H), 7,36 (d, 2H, *J* = 7,8 Hz), 7,45 (d, 2H, *J* = 8,4 Hz), 7,96 (d, 2H, *J =* 8,4 Hz).

### (b) acide 4-[4-(4'-méthyl-6-hydroxy-biphényl-2-yl)-but-3-èn-(E)-1-ynyl]-benzoïque.

De manière analogue à l'exemple 1(f), à partir de 350 mg (0,95 mmole) de l'ester méthylique obtenu à l'exemple 44(a), on obtient 300 mg (90%) d'acide 4-[4-(4'-méthyl-6-hydroxy-biphényl-2-yl)-but-3-èn-(E)-1-ynyl]-benzoïque, sous la forme d'une poudre beige de point de fusion 234-236°C.
¹H NMR (DMSO D₆) δ 2,37 (s, 3H), 6,51 (d, 1H, *J* = 16,3 Hz), 6,71 (d, 1H, *J* = 16,3 Hz), 6,92 (d, 2H, *J* = 7,8 Hz), 7,08 (d, 1H, *J* = 7,8 Hz), 7,16 à 7,31 (m, 4H), 7,52 (d, 2H, *J* = 8,2 Hz), 7,90 (d, 2H, J = 8,2 Hz), 9,41 (br s, 1H).

### EXEMPLE 45

### Acide 4-[4-(4'-méthyl-6-méthoxy-biphényl-2-yl)-but-3-èn-(E)-1-ynyl]-benzoïque.

### (a) 4-[4-(4'-méthyl-6-méthoxy-biphényl-2-yl)-but-3-èn-(E)-1-ynyl]-benzoate de méthyle.

De manière analogue à l'exemple 33(a), à partir de 270 mg (0,73 mmole) du composé obtenu à l'exemple 44(a) et 50 µl (0,80 mmole) d'iodure de méthyle, on obtient 270 mg (98%) du composé attendu, sous la forme d'une poudre beige de point de fusion 116-118°C.
¹H NMR (CDCl₃) δ 2,43 (s, 3H), 3,74 (s, 3H), 3,91 (s, 3H), 6,29 (d, 1H, *J* = 16,2 Hz), 6,84 (d, 1H, *J* = 16,2 Hz), 6,93 (dd, 1H, *J* = 7,1 / 1,9 Hz), 7,14 (d, 2H, *J* = 8,0 Hz), 7,25 à 7,36 (m, 4H), 7,45 (d, 2H, *J* = 8,4 Hz), 7,96 (d, 2H, *J* = 8,4 Hz).

### (b) acide 4-[4-(4'-méthyl-6-méthoxy-biphényl-2-yl)-but-3-èn-(E)-1-ynyl]-benzoïque.

De manière analogue à l'exemple 1(f), à partir de 270 mg (0,72 mmole) de l'ester méthylique obtenu à l'exemple 45(a), on obtient 240 mg (92%) d'acide 4-[4-(4'-méthyl-6-méthoxy-biphényl-2-yl)-but-3-èn-(E)-1-ynyl]-benzoïque, sous la forme de paillettes beiges de point de fusion 235-236°C.
¹H NMR (DMSO D₆) δ 2,37 (s, 3H), 3,66 (s, 3H), 6,57 (d, 1H, *J* = 16.3 Hz), 6,67 (d, 1H, *J* = 16,3 Hz), 7,04 à 7,09 (m, 3H), 7,25 (d, 2H, *J* = 7,7 Hz), 7,33 à 7,47 (m, 2H), 7,52 (d, 2H, *J* = 8,3 Hz), 7,89 (d, 2H, *J* = 8,3 Hz), 13,12 (br s, 1H).

### EXEMPLE 46

### Acide 4-[4-(4'-trifuorométhyl-biphényl-2-yl)-but-3-èn(E)-1-ynyl]-benzoïque.

### (a) 4'-trifluorométhyl-biphényl-2-carboxaldéhyde.

De manière analogue à l'exemple 21(a), à partir de 1,43 g (7,5 mmoles) d'acide 4-trifluorométhylbenzène boronique et 928 mg (5,0 mmoles) de 2-bromobenzaldéhyde, on obtient 1,13 g (60 %) du composé attendu, sous la forme d'une huile légèrement jaune.
¹H NMR (CDCl₃) δ 7,43 (d, 1H, *J* = 7,5 Hz), 7,51 (d, 2H, *J* = 7,8 Hz), 7,57 (d, 1H, *J* = 7,6 Hz), 7,67 (d, 1H, *J* = 7,5 Hz), 7,74 (d, 2H, J = 8,3 Hz), 8,06 (d, 1H, *J* = 7,7 Hz), 9,96 (s, 1H).

### (b) 2-((E)-2-iodovinyl)-4'-trifluorométhyl-biphényle.

De manière analogue à l'exemple 1(d), à partir de 1,13 g (4,5 mmoles) de 4'-trifluorométhyl-biphényl-2-carboxaldéhyde obtenu à l'exemple 46(a), on obtient 520 mg (31%) du composé attendu, sous la forme d'une huile jaune.
¹H NMR (CDCl₃) δ 6,80 (d, 1H, *J* = 14,8 Hz), 7,05 à 7,08 (m, 2H) 7,28 à 7,52 (m, 5H), 7,64 à 7,72 (m, 2H).

### (c) 4-[4-(4'-trifluorométhyl-biphényl-2-yl)-but-3-èn-(E)-1-ynyl]-benzoate de méthyle.

De manière analogue à l'exemple 11(f), par réaction de 520 mg (1,4 mmole) de 2-((E)-2-iodovinyl)-4'-trifluorométhyl-biphényle obtenu à l'exemple 46(b) avec 245 mg (1,5 mmole) de 4-éthynyl benzoate de méthyle, on obtient 200 mg (35%) du composé attendu, sous la forme d'une poudre jaune de point de fusion 119°C.
¹H NMR (CDCl₃) δ 3,91 (s, 3H), 6,38 (d, 1H, *J* = 16,2 Hz), 6,98 (d, 1H, *J* = 16,2 Hz), 7,28 à 7,50 (m, 7H), 7,66 à 7,70 (m, 1H), 7,72 (d, 2H, *J* = 8,4 Hz), 7,97 (d, 2H, *J* = 8,2 Hz).

### (d) acide 4-[4-(4'-trifluorométhyl-biphényl-2-yl)-but-3-èn-(E)-1-ynyl]-benzoïque.

De manière analogue à l'exemple 1(f), à partir de 200 mg (0,49 mmole) de l'ester méthylique obtenu à l'exemple 46(c), on obtient 150 mg (77%) d'acide 4-[4-(4'-trifluorométhyl-biphényl-2-yl)-but-3-èn-(E)-1-ynyl]-benzoïque, sous a forme d'une poudre jaune de point de fusion 270°C.
¹H NMR (CDCl₃ + 2 gouttes de DMSO D₆) δ 6,31 (d, 1H, *J* = 16,2 Hz), 6,88 (d, 1H, *J* = 16,2 Hz), 7,20 à 7,24 (m, 1H), 7,31 à 7,46 (m, 6H), 7,59 à 7,62 (m, 1H), 7,64 (d, 2H, *J* = 8,3 Hz), 7,88 (d, 2H, *J* = 8,2 Hz).

### EXEMPLE 47

### Acide 4-[4-(4'-hydroxyméthyl-biphényl-2-yl)-but-3-èn-(E)-1-ynyl]-benzoïque.

### (a) 4-éthoxyméthoxyméthyl bromobenzène.

Dans un tricol, on introduit 8,00 g (42,8 mmoles) d'alcool 4-bromobenzylique, 1,02 g d'hydrogénosulfate de tétrabutylammonium et 200 ml de toluène. On refroidit la solution obtenue à 0°C et ajoute goutte à goutte 11,9 ml (128,3 mmoles) d'éther éthylique de chlorométhyle, puis 200 ml d'une solution aqueuse de soude 10 N. Le milieu réactionnel est agité pendant deux heures à 0°C, puis versé dans un mélange HCI 1N / éther éthylique et extrait par de l'éther éthylique. On décante la phase organique et sèche sur sulfate de magnésium. Après évaporation des solvants, on recueille 10,48 g (100%) du composé attendu, sous la forme d'une huile beige.
¹H NMR (CDCl₃) δ 1,23 (t, 3H, *J* = 7,0 Hz), 3,64 (q, 2H, *J =* 7,1 Hz), 4,55 (s, 2H), 4,75 (s, 2H), 7,23 (d, 2H, *J* = 8,3 Hz), 7,47 (d, 2H, *J* = 8,3 Hz).

### (b) acide 4-éthoxyméthoxyméthyl phénylboronique.

De manière analogue à l'exemple 2(a), à partir de 10,58 g (43,2 mmoles) de 4-éthoxyméthoxyméthyl bromobenzène obtenu à l'exemple 47(a), on obtient 9,00 g (99%) du composé attendu, sous la forme d'une huile jaune.
¹H NMR (CDCl₃) δ 1,26 (t, 3H, *J* = 7,0 Hz), 3,69 (q, 2H, *J* = 7,1 Hz), 4,71 (s, 2H), 4,81 (s, 2H), 7,49 (d, 2H, *J* = 7,7 Hz), 8,22 (d, 2H, *J* = 7,7 Hz).

### (c) 4'-éthoxyméthoxyméthyl-biphényl-2-carboxaldéhyde.

De manière analogue à l'exemple 21(a), à partir de 9,00 g (42,8 mmoles) d'acide 4-éthoxyméthoxyméthyl phénylboronique obtenu à l'exemple 47(b) et 5,33 g (28.8 mmoles) de 2-bromobenzaldéhyde, on obtient 6,04 g (77%) du composé attendu, sous la forme d'une huile jaune.
¹H NMR (CDCl₃) δ 1,26 (t, 3H, *J* = 7,1 Hz), 3,69 (q, 2H, *J* = 7,1 Hz), 4,69 (s, 2H), 4,82 (s, 2H), 7,38 (d, 2H, *J* = 8,1 Hz), 7,42 à 7,56 (m, 2H), 7,48 (d, 2H, *J* = 8,4 Hz), 7,63 (dd, 1H, *J* = 7,4 1,4 Hz), 8,03 (dd, 1H, *J* = 7,7 / 1,2 Hz), 9,99 (s, 1H).

### (d) 2-((E)-2-iodovinyl)-4'-éthoxyméthoxyméthyl-biphényle.

De manière analogue à l'exemple 1(d), à partir de 3,50 g (12,9 mmoles) de 4'-éthoxyméthoxyméthyl-biphényl-2-carboxaldéhyde obtenu à l'exemple 47(c), on obtient 1,97 g (39%) du composé attendu, sous la forme d'une huile orangée.
¹H NMR (CDCl₃) δ 1,29 (t, 3H, *J* = 7,0 Hz), 3,69 (q, 2H, *J* = 7,0 Hz), 4,67 (s, 2H), 4,83 (s, 2H), 6,75 (d, 1H, *J*= 14,8 Hz), 7,28 à 7,49 (m, 9H).

### (e) 4-[4-(4'-éthoxyméthoxyméthyl-biphényl-2-yl)-but-3-èn-(E)-1-ynyl]-benzoate de méthyle.

De manière analogue à l'exemple 11(f), par réaction de 1,97 g (5,0 mmoles) de 2-((E)-2-iodovinyl)-4'-éthoxyméthoxyméthyl-biphényle obtenu à l'exemple 47(d) avec 1,20 g (7,5 mmoles) de 4-éthynyl benzoate de méthyle, on obtient 537 mg (25%) du composé attendu, sous la forme d'une poudre jaune clair de point de fusion 60°C.
¹H NMR (CDCl₃) δ 1,27 (t, 3H, *J* = 7,1 Hz), 3,70 (q, 2H, *J* = 7,1 Hz), 3,91 (s, 3H), 4,68 (s, 2H), 4,84 (s, 2H), 6,35 (d, 1H, *J* = 16,2 Hz), 7,08 (d, 1H, *J* = 16,2 Hz), 7,29 à 7,50 (m, 7H), 7,48 (d, 2H, *J* = 8,3 Hz), 7,64 à 7,68 (m, 1H), 7,97 (d, 2H, *J* = 8,4 Hz).

### (f) 4-[4-(4'-hydroxyméthyl-biphényl-2-yl)-but-3-èn-(E)-1-ynyl]-benzoate de méthyle.

De manière analogue à l'exemple 32(a), à partir de 387 mg (0,91 mmole) de l'ester méthylique obtenu à l'exemple 47(e), on obtient 310 mg (93%) du composé attendu, sous la forme d'une poudre beige de point de fusion 142°C.
¹H NMR (CDCl₃) δ 1,79 (br s, 1H), 3,91 (s, 3H), 4,79 (s, 2H), 6,36 d, 1H, *J* = 16,2 Hz), 7,07 (d, 1H, *J* = 16,2 Hz), 7,32 (m, 5H), 7,45 à 7,49 (m, 2H), 7,47 (d, 2H, *J* = 8,4 Hz), 7,62 à 7,68 (m, 1H), 7,97 (d, 2H, *J* = 8,4 Hz).

### (g) acide 4-[4-(4'-hydroxyméthyl-biphényl-2-yl)-but-3-èn-(E)-1-ynyl]-benzoïque.

De manière analogue à l'exemple 1(f), à partir de 310 mg (0,34 mmole) de l'ester méthylique obtenu à l'exemple 47(f), on obtient 250 mg (84%) d'acide 4-[4-(4'-hydroxyméthyl-biphényl-2-yl)-but-3-èn-(E)-1-ynyl]-benzoïque, sous la forme d'une poudre beige de point de fusion 227°C.
¹H NMR (CDCl₃ + 2 gouttes de DMSO D₆) δ 4,69 (s, 2H), 6,33 (d, 1H, *J* = 16,2 Hz), 7,03 (d, 1H, *J* = 16,3 Hz), 7,27 à 7,36 (m, 5H), 7,42 à 7,46 (m, 2H), 7,44 (d, 2H, *J* = 8,3 Hz), 7,62 à 7,66 (m, 1H), 7,94 (d, 2H, *J* = 8,3 Hz).

### EXEMPLE 48

### Acide 4-{4-[4'-(2-hydroxy-éthyl)-biphényl-2-yl]-but-3-èn-(E)-1-ynyl}-benzoïque.

### (a) 2-(4-bromo-phényl)-éthanol.

Dans un tricol de deux litres et sous courant d'azote, on introduit 15,00 g (81,9 mmoles) de 4-bromostyrène et 300 ml de THF. On refroidit à 0°C, ajoute goutte à goutte 492 ml (245,8 mmoles) de 9-BBN (0,5 M dans le THF), en maintenant la température inférieure à 5°C. Le milieu réactionnel est agité pendant quatre heures à la température ambiante, puis on refroidit à 0°C, coule goutte à goutte 25 ml (254,0 mmoles) d'une solution aqueuse de soude 10 N, suivie de 209 ml (2,05 moles) d'eau oxygénée à 30% (9,8 M), en maintenant la température inférieure à 10°C. Le milieu réactionnel est agité pendant quatre heures à la température ambiante, puis on ajoute de l'eau, une solution d'HCI 1N, extrait par de l'éther éthylique, lave la phase organique à l'eau jusqu'à pH neutre, sèche sur sulfate de magnésium, filtre et évapore les solvants. Le résidu obtenu est purifié par chromatographie sur colonne de silice, élué par un mélange composé de 30% d'acétate d'éthyle et 70% d'heptane. Après évaporation des solvants, on recueille 12,92 g (78%) du composé attendu, sous la forme d'une huile jaune.
¹H NMR (CDCl₃) δ 1,54 (s, 1H), 2,81 (t, 2H, *J =* 6,5 Hz), 3,83 (t, 2H, *J* = 6,4 Hz), 7,10 (d, 2H, *J* = 8,32 Hz), 7,43 (d, 2H, *J* = 8,3 Hz).

### (b) 4-éthoxyméthoxyéthyl bromobenzène.

De manière analogue à l'exemple 47(a), à partir de 12,92 g (64,3 mmoles) de 2-(4-bromo-phényl)-éthanol obtenu à l'exemple 48(a), on obtient 16,65 g (100%) du composé attendu, sous la forme d'une huile jaune.
¹H NMR (CDCl₃) δ 1,17 (t, 3H, *J* = 7,0 Hz), 2,84 (t, 2H, *J* = 6,7 Hz), 3,50 (q, 2H, *J* = 7,1 Hz), 3,74 (t, 2H, *J* = 6,7 Hz), 4,64 (s, 2H), 7,10 (d, 2H, *J* = 8,2 Hz), 7,40 (d, 2H, *J* = 8,2 Hz).

### (c) acide 4-éthoxyméthoxyéthyl phénylboronique.

De manière analogue à l'exemple 2(a), à partir de 17,31 g (66,8 mmoles) de 4-éthoxyméthoxyéthyl bromobenzène obtenu à l'exemple 48(b), on obtient 14,07 g (94%) du composé attendu, sous la forme d'une huile jaune.
¹H NMR (CDCl₃) δ 1,19 (t, 3H, *J* = 7,0 Hz), 2,99 (t, 2H, *J* = 6,7 Hz), 3,54 (q, 2H, *J* = 7,1 Hz), 3,84 (t, 2H, *J* = 6,9 Hz), 4,70 (s, 2H), 7,38 (d, 2H, *J* = 7,9 Hz), 8,16 (d, 2H, *J* = 7,8 Hz).

### (d) 4'-éthoxyméthoxyéthyl-biphényl-2-carboxaldéhyde.

De manière analogue à l'exemple 21(a), à partir de 14,00 g (67,0 mmoles) d'acide 4-éthoxyméthoxyéthyl phénylboronique obtenu à l'exemple 48(c) et 8,30 g (44,8 mmoles) de 2-bromobenzaldéhyde, on obtient 11,68 g (93%) du composé attendu, sous la forme d'une huile orangée.
¹H NMR (CDCl₃) δ 1,18 (t, 3H, *J* = 7,1 Hz), 2,98 (t, 2H, *J* = 6,8 Hz), 3,54 (q, 2H, *J* = 7,1 Hz), 3,84 (t, 2H, *J* = 6,9 Hz), 4,70 (s, 2H), 7,29 à 7,37 (m, 4H), 7,44 (d, 1H, *J =* 7,5 Hz), 7,49 (d, 1H, *J* = 7,6 Hz), 7,61 (dd, 1H, *J* = 7,4 / 1,4 Hz), 8,02 (dd, 1H, *J* = 7,7 / 1,3 Hz), 9,98 (s, 1H).

### (e) 2-((E)-2-iodovinyl)-4'-éthoxyméthoxyéthyl-biphényle.

De manière analogue à l'exemple 1(d), à partir de 3,50 g (12,6 mmoles) de 4'-éthoxyméthoxyéthyl-biphényl-2-carboxaldéhyde obtenu à l'exemple 48(d), on obtient 2,07 g (40%) du composé attendu, sous la forme d'une huile jaune clair.
¹H NMR (CDCl₃) δ 1,19 (t, 3H, *J* = 7,1 Hz), 2,97 (t, 2H, *J* = 6,8 Hz), 3,54 (q, 2H, *J* = 7,1 Hz), 3,85 (t, 2H, *J* = 6,8 Hz), 4,71 (s, 2H), 6,73 (d, 1H, *J* = 14,8 Hz), 7,23 à 7,49 (m, 9H).

### (f) 4-[4-(4'-éthoxyméthoxyéthyl-biphényl-2-yl)-but-3-èn-(E)-1-ynyl]-benzoate de méthyle.

De manière analogue à l'exemple 11(f), par réaction de 2,07 g (5,1 mmoles) de 2-((E)-2-iodovinyl)-4'-éthoxyméthoxyéthyl-biphényle obtenu à l'exemple 48(e) avec 1,22 g (7,6 mmoles) de 4-éthynyl benzoate de méthyle, on obtient 1,02 g (46%) du composé attendu, sous la forme d'une poudre jaune clair de point de fusion 68°C.
¹H NMR (CDCl₃) δ 1,19 (t, 3H, *J* = 7,1 Hz), 2,98 (t, 2H, *J* = 6,9 Hz), 3,54 (q, 2H, *J* = 7,1 Hz), 3,86 (t, 2H, *J* = 6,9 Hz), 3,91 (s, 3H), 4,71 (s, 2H), 6,35 (d, 1H, J = 16,2 Hz), 7,09 (d, 1H, *J* = 16,2 Hz), 7,30 à 7,38 (m, 7H), 7,48 (d, 2H, *J* = 8,3 Hz), 7,64 à 7,67 (m, 1H), 7,97 (d, 2H, *J* = 8,3 Hz).

### (g) 4-{4-[4'-(2-hydroxy-éthyl)-biphényl-2-yl]-but-3-èn-(E)-1-ynyl}-benzoate de méthyle.

De manière analogue à l'exemple 32(a), à partir de 670 mg (1,5 mmole) de l'ester méthylique obtenu à l'exemple 48(f), on obtient 540 mg (93%) du composé attendu, sous la forme d'une huile jaune.
¹H NMR (CDCl₃) δ 1,47 (t, 1H, *J* = 5,9 Hz), 2,96 (t, 2H, *J* = 6,5 Hz), 3,91 (s, 3H), 3,95 (q, 2H, *J* = 6,4 Hz), 6,36 (d, 1H, *J* = 16,2 Hz), 7,10 (d, 1H, *J* = 16,2 Hz), 7,32 à 7,39 (m, 7H), 7,48 (d, 2H, *J* = 8,4 Hz), 7,64 à 7,68 (m, 1H), 7,97 (d, 2H, *J* = 8,4 Hz).

### (h) acide 4-{4-[4'-(2-hydroxy-éthyl)-biphényl-2-yl]-but-3-èn-(E)-1-ynyl-benzoïque.

De manière analogue à l'exemple 1(f), à partir de 540 mg (1,4 mmole) de l'ester méthylique obtenu à l'exemple 48(g), on obtient 470 mg (90%) d'acide 4-{4-[4'-(2-hydroxy-éthyl)-biphényl-2-yl]-but-3-èn-(E)-1-ynyl}-benzoïque, sous la forme d'une poudre beige de point de fusion 205°C.
¹H NMR (CDCl₃ + 2 gouttes de DMSO D₆) δ 2,84 (t, 2H, *J* = 6,8 Hz), 3,78 (t, 2H, *J* = 6,8 Hz), 6,29 (d, 1H, *J* = 16,2 Hz), 7,00 (d, 1H, *J* = 16,3 Hz), 7,19 (d, 2H, *J* = 8,1 Hz), 7,26 (d, 2H, *J* = 8,2 Hz), 7,27 à 7,30 (m, 3H), 7,38 (d, 2H, *J* = 8,2 Hz), 7,57 à 7,60 (m, 1H), 7,88 (d, 2H, *J* = 8,2 Hz).

### EXEMPLE 49

### Acide 4-[4-(3'-méthyl-biphényl-3-yl)-but-3-èn-(E)-1-ynyl]-benzoïque.

### (a) acide 3-méthyl-phényl boronique.

De manière analogue à l'exemple 2(a), à partir de 9,07 g (53,0 mmoles) de 3-méthylbromobenzène, on obtient 7,12 g (99%) du composé attendu, sous la forme d'un solide blanc de point de fusion inférieur à 30°C.
¹H NMR (CDCl₃) δ 2,46 (s, 3H), 7,28 à 7,55 (m, 2H), 7,90 à 8,06 (m, 2H).

### (b) 3'-méthyl-biphényl-3-carboxaldéhyde.

De manière analogue à l'exemple 21(a), à partir de 7,12 g (52,4 mmoles) d'acide 3-méthyl-phényl boronique et 6,46 g (34,9 mmoles) de 3-bromobenzaldéhyde, on obtient 6,56 g (96%) du composé attendu, sous la forme d'une huile jaune.
¹H NMR (CDCl₃) δ 2,44 (s, 3H), 7,23 (t, 1H, *J* = 7,2 Hz), 7,33 à 7,44 (m, 3H), 7,60 (t, 1H, *J* = 7,6 Hz), 7,83 à 7,87 (m, 2H), 8,09 à 8,10 (m, 1H), 10,09 (s, 1H)

### (c) 3-((E)2-iodo-vinyl)-3'-méthyl-biphényle.

De manière analogue à l'exemple 1(d), à partir de 3,34 g (17,0 mmoles) de 3'-méthyl-biphényl-3-carboxaldéhyde obtenu à l'exemple 49(b), on obtient 5,47 g (100%) du composé attendu, sous la forme d'une huile jaune.
¹H NMR (CDCl₃) δ 2,42 (s, 3H), 6,90 (d, 1H, *J* = 14,9 Hz), 7,16 à 7,52 (m, 8H), 7,51 (d, 1H, *J* = 14,9 Hz).

### (d) 4-[4-(3'-méthyl-biphényl-3-yl)-but-3-èn-(E)-1-ynyl]-benzoate de méthyle.

De manière analogue à l'exemple 1(e), par réaction de 5,47 g (17,1 mmoles) de 3-((E)2-iodo-vinyl)-3'-méthyl-biphényle obtenu à l'exemple 49(c) avec 2,46 g (15,4 mmoles) de 4-éthynyl benzoate de méthyle, on obtient 3,09 g (57%) du composé attendu, sous la forme d'une poudre jaune de point de fusion 98-100°C.
¹H NMR (CDCl₃) δ 2,43 (s, 3H), 3,92 (s, 3H), 6,46 (d, 1H, *J* = 16,2 Hz), 7,15 (d, 1H, *J* = 16,2 Hz), 7,18 à 7,21 (m, 1H), 7,31 à 7,45 (m, 5H), 7,51 à 7,55 (m 1H), 7,53 (d, 2H, *J* = 8,5 Hz), 7,62 (s, 1H), 8,00 (d, 2H, *J* = 8,4 Hz).

### (e) acide 4-[4-(3'-méthyl-biphényl-3-yl)-but-3-èn-(E)-1-ynyl]-benzoïque.

De manière analogue à l'exemple 1(f), à partir de 1,25 g (mmoles) de l'ester méthylique obtenu à l'exemple 49(d), on obtient 1,15 g (96%) d acide 4-[4-(3'-méthyl-biphényl-3-yl)-but-3-èn-(E)-1-ynyl]-benzoïque, sous la forme d'une poudre jaune clair de point de fusion 224-228°C.
¹H NMR (DMSO D₆) à 2,26 (s, 3H), 6,69 (d, 1H, *J* = 16,3 Hz), 7,05 à 7,38 (m, 3H), 7,41 à 7,49 (m, 5H), 7,47 (d, 2H, *J* = 8,4 Hz), 7,74 (s, 1H), 7,83 (d, 2H, *J* = 8,3 Hz), 13,00 (br s, 1H).

### EXEMPLE 50

### Acide 4-[4-(2-pyridin-4-yl-phényl)-but-3-èn-(E)-1-ynyl]-benzoïque.

### (a) 2-pyridin-4-yl-benzaldéhyde.

De manière analogue à l'exemple 21(a) à partir de 2,50 g (16,7 mmoles) d'acide 2-formylbenzène boronique et 2,16 g (11,1 mmoles) de chlorhydrate de 4-bromopyridine, on obtient 1,98 g (97%) du composé attendu, sous la forme d'une poudre blanche de point de fusion 59°C.
¹H NMR (CDCl₃) δ 7,33 (d, 2H, *J* = 6,0 Hz), 7,43 (dd, 1H, *J* = 8,4 */* 0,9 Hz), 7,59 (t, 1H, *J* = 7,4 Hz), 7,70 (dt, 1H, *J =* 7,5 1,4 Hz), 8,07 (dd, 1H, *J* = 7,6 / 1,1 Hz), 8,73 (d, 2H, *J =* 6,0 Hz), 9,99 (s, 1H).

### (b) 4-[2-((E)-2-iodo-vinyl)-phényl]-pyridine.

De manière analogue à l'exemple 1(d), à partir de 1,98 g (10,8 mmoles) de 2-pyridin-4-yl-benzaldéhyde obtenu à l'exemple 50(a), on obtient 490 mg (15%) du composé attendu, sous la forme d'une huile verte.
¹H NMR (CDCl₃) δ 6,84 (d, 1H, *J* = 14,8 Hz), 7,28 à 7,51 (m, 7H), 8,69 (d, 2H, *J* = 5,5 Hz).

### (c) 4-[4-(2-pyridin-4-yl-phényl)-but-3-èn-(E)-1-ynyl]-benzoate de méthyle.

De manière analogue à l'exemple 11(f), par réaction de 490 mg (1,6 mmole) de 4-[2-((E)-2-iodo-vinyl)-phényl]-pyridine obtenu à l'exemple 50(b) avec 380 mg (2,4 mmoles) de 4-éthynyl benzoate de méthyle, on obtient 200 mg (37%) du composé attendu, sous la forme d'une poudre orange de point de fusion 120°C.
¹H NMR (CDCl₃) δ 3,91 (s, 3H), 6,38 (d, 1H, *J* = 16,1 Hz), 6,99 (d, 1H, *J* = 16,1 Hz), 7,29 à 7,32 (m, 3H), 7,39 à 7,44 (m, 2H), 7,49 (d, 2H, *J* = 8,3 Hz), 7,67 à 7,70 (m, 1H), 7,98 (d, 2H, *J* = 8,3 Hz), 8,70 (d, 2H, *J* = 5,2 Hz).

### (d) acide 4-[4-(2-pyridin-4-yl-phényl)-but-3-èn-(E)-1-ynyl]-benzoïque.

De manière analogue à l'exemple 1(f), à partir de 200 mg (C,6 mmole) de l'ester méthylique obtenu à l'exemple 50(c), on obtient 190 mg (99% d'acide 4-{4-[4'-(2-hydroxy-éthyl)-biphényl-2-yl]-but-3-èn-(E)-1-ynyl}-benzoïque, sous la forme d'une poudre blanche de point de fusion 169-177°C.
¹H NMR (Pyridine D₅) δ 6,70 (d, 1H, *J* = 16,2 Hz), 7,28 à 7,45 (m, 4H), 7,64 (d, 2H, *J* = 8,0 Hz), 7,80 à 7,93 (m, 2H), 7,97 à 8,10 (m, 1H), 8,38 (d, 2H, *J =* 7,7 Hz), 8,77 (m, 2H).

### EXEMPLE 51

### Acide 4-[4-(2-pyridin-3-yl-phényl)-but-3-èn-(E)-1-ynyl]-benzoïque.

### (a) 2-pyridin-3-yl-benzaldéhyde.

De manière analogue à l'exemple 21(a), à partir de 2,50 g (16,7 mmoles) d'acide 2-formylbenzène boronique et 1,76 g (11,1 mmoles) de 3-bromopyridine, on obtient 1,48 g (73%) du composé attendu, sous la forme d'une poudre jaune de point de fusion 54°C.
¹H NMR (CDCl₃) δ 7,40 à 7,45 (m, 2H), 7,58 (t, 1H, *J* = 7,5 Hz), 7,69 (dd, 1H, *J* = 7,4 / 1,5 Hz), 7,72 (dd, 1H, *J* = 7,9 / 2,0 Hz), 8,07 (dd, 1H, *J* = 7,8 / 1,3 Hz), 8,67 à 8,72 (m, 2H), 9,99 (s, 1H).

### (b) 3-[2-((E)-2-iodo-vinyl)-phényl]-pyridine.

De manière analogue à l'exemple 1(d), à partir de 1,48 g (8,1 mmoles) de 2-pyridin-3-yl-benzaldéhyde obtenu à l'exemple 51(a), on obtient 600 mg (24%) du composé attendu, sous la forme d'une huile jaune.
¹H NMR (CDCl₃) δ 6,82 (d, 1H, *J* = 14,8 Hz), 7,26 à 7,30 (m, 2H), 7,34 à 7,42 (m, 3H), 7,50 à 7,53 (m, 1H), 7,63 à 7,67 (m, 1H), 8,61 à 8,65 (m, 2H).

### (c) 4-[4-(2-pyridin-3-yl-phényl)-but-3-èn-(E)-1-ynyl]-benzoate de méthyle.

De manière analogue à l'exemple 11(f), par réaction de 600 mg (2,0 mmoles) de 3-[2-((E)-2-iodo-vinyl)-phényl]-pyridine obtenu à l'exemple 51(b) avec 469 mg (2,9 mmoles) de 4-éthynyl benzoate de méthyle, on obtient 550 mg (83%) du composé attendu, sous la forme d'une poudre jaune de point de fusion 85°C.
¹H NMR (CDCl₃) δ 3,91 (s, 3H), 6,38 (d, 1H, *J* = 16,1 Hz), 7,00 (d, 1H, *J* = 16,2 Hz), 7,30 à 7,50 (m, 6H), 7,68 à 7,71 (m, 2H), 7,98 (d, 2H, *J* = 8,3 Hz), 8,65 (d, 2H, *J* = 8,3 Hz).

### (d) acide 4-[4-(2-pyridin-3-yl-phényl)-but-3-èn-(E)-1-ynyl]-benzoïque.

Dans un ballon, on introduit 550 mg (1,6 mmole) de l'ester obtenu à l'exemple 51(c), 10 ml de méthanol et 10 ml dé THF. On ajoute 1,6 ml (16,0 mmoles) d'une solution de soude méthanolique (10N) et chauffe à reflux pendant une heure. On évapore à sec, ajoute de l'acétate d'éthyle, acidifie à pH 5 avec de l'acide citrique, filtre le précipité, lave à l'eau, sèche à l'étuve sous vide. On obtient 290 mg d'acide 4-[4-(2-pyridin-3-yl-phényl)-but-3-èn-(E)-1-ynyl]-benzoïque, sous la forme d'un solide beige de point de fusion 264°C.
¹H NMR (Pyridine D₅) δ 6,68 (d, 1H, *J* = 16,1 Hz), 7,23 à 7,34 (m, 4H), 7,38 à 7,44 (m, 2H), 7,58 à 7,70 (m, 1H), 7,61 (d, 2H, *J* = 8,3 Hz), 7,82 à 7,85 (m, 1H), 8,36 (d, 2H, *J* = 8,2 Hz), 8,72 à 8,74 (m, 1H), 8,84 (d, 1H, *J* = 2,0 Hz).

### EXEMPLE 52

### Acide 4-[4-(3-méthoxyméthoxy-4'-méthyl-biphényl-2-yl)-but-3-èn-(E)-1-ynyl]-benzoïque.

### (a) trifluorométhanesulfonate de 2-formyl-3-méthoxyphényle.

De manière analogue à l'exemple 12(b), à partir de 11,00 g (72,3 mmoles) de 2-hydroxy-6-méthoxybenzaldéhyde, on obtient 15,00 g (73%) du composé attendu, sous la forme d'une poudre beige de point de fusion 65-67°C.
¹H NMR (CDCl₃) δ 3,98 (s, 3H), 6,90 (d, 1H, *J* = 8,3 Hz), 7,07 (d, 1H, *J* = 8,6 Hz), 7,60 (t, 1H, *J* = 8,5 Hz), 10,46 (s, 1H).

### (b) 3-méthoxy-4'-méthyl-biphényl-2-carboxaldéhyde.

De manière analogue à l'exemple 12(c), à partir de 8,30 g (61,3 mmoles) d'acide 4-méthylphényl boronique et 14,50 g (51,0 mmoles) du triflate obtenu à l'exemple 52(a), on obtient 10,80 g (94%) du composé attendu, sous la forme de cristaux jaune clair de point de fusion 89-91°C.
¹H NMR (CDCl₃) δ 2,40 (s, 3H), 3,95 (s, 3H), 6,98 (t, 2H, *J* = 7,3 Hz), 7,22 (s, 4H), 7,51 (t, 1H, *J* = 7,7 Hz), 10,07 (s, 1H).

### (c) 3-hydroxy-4'-méthyl-biphényl-2-carboxaldéhyde.

De manière analogue à l'exemple 17(c), à partir de 10,00 g (44,2 mmoles) de 3-méthoxy-4'-méthyl-biphényl-2-carboxaldéhyde obtenu à l'exemple 52(b), on obtient 7,00 g (75%) du composé attendu, sous la forme d'une poudre orange de point de fusion 52-53°C.
¹H NMR (CDCl₃) δ 2,42 (s, 3H), 6,87 (dd, 1H, *J* = 7,7 / 0,8 Hz), 6,97 (d, 1H, *J* = 8,5 Hz), 7,26 (s, 4H), 7,51 (t, 1H, *J* = 7,6 Hz), 9,85 (s, 1H).

### (d) 3-méthoxyméthoxy-4'-méthyl-biphényl-2-carboxaldéhyde.

De manière analogue à l'exemple 28(a), à partir de 7,00 g (33,0 mmoles) du phénol obtenu à l'exemple 52(c) et de 3,18 g (40,0 mmoles) d'éther méthylique de chlorométhyle, on obtient 7,95 g (94%) du composé attendu, sous la forme d'une huile jaune.
¹H NMR (CDCl₃) δ 2,40 (s, 3H), 3,54 (s, 3H), 5,31 (s, 2H), 7,02 (d, 1H, *J* = 7,6 Hz), 7,18 à 7,26 (m, 5H), 7,49 (t, 1H, *J* = 7,8 Hz), 10,11 (s, 1H).

### (e) 2-((E)-2-iodovinyl)-3-méthoxyméthoxy-4'-méthyl-biphényle.

De manière analogue à l'exemple 1(d), à partir de 7,95 g (31,0 mmoles) de 3-méthoxyméthoxy-4'-méthyl-biphényl-2-carboxaldéhyde obtenu à l'exemple 52(d), on obtient 1,30 g (11%) du composé attendu, sous la forme d'une huile orange.
¹H NMR (CDCl₃) δ 2,41 (s, 3H), 3,51 (s, 3H), 5,25 (s, 2H), 6,87 (d, 1H, *J* = 14,8 Hz), 6,93 (dd, 1H, *J* = 7,5 / 1,2 Hz), 7,10 à 7,35 (m, 5H), 9,84 (s, 1H).

### (f) 4-[4-(3-méthoxyméthoxy-4'-méthyl-biphényl-2-yl)-but-3-èn-(E)-1-ynyl]-benzoate de méthyle.

De manière analogue à l'exemple 1(e), par réaction de 1,30 g (3,4 mmoles) de 2-((E)-2-iodovinyl)-3-méthoxyméthoxy-4'-méthyl-biphényle obtenu à l'exemple 52(e), avec 650 mg (4,1 mmoles) de 4-éthynyl benzoate de méthyle, on obtient 750 mg (53%) du composé attendu, sous la forme d'une poudre jaune correspondant au mélange d'isomères Z/E.
¹H NMR (CDCl₃) δ 2,42 (s, 3H), 3,54 (s, 3H), 3,91 (s, 3H), 5,30 (s, 2H), 6,57 (d, 1H, *J* = 16,5 Hz), 7,48 (d, 1H, *J* = 16,2 Hz), 7,18 à 7,26 (m, 7H), 7,46 (d, 2H, *J* = 8,3 Hz), 7,96 (d, 2H, *J* = 8,4 Hz).

### (g) acide 4-[4-(3-méthoxyméthoxy-4'-méthyl-biphényl-2-yl)-but-3-èn-(E)-1-ynyl]-benzoïque.

De manière analogue à l'exemple 1(f), à partir de 200 mg (0,48 mmole) de l'ester méthylique obtenu à l'exemple 52(f), on obtient 152 mg (80%) d'acide 4-[4-(3-méthoxyméthoxy-4'-méthyl-biphényl-2-yl)-but-3-èn-(E)-1-ynyl]-benzoïque, sous la forme d'une poudre jaune de point de fusion 160-162°C.
¹H NMR (DMSO D₆) δ 2,37 (s, 3H), 3,44 (s, 3H), 5,36 (s, 2H), 6,57 (d, 1H, *J* = 16,5 Hz), 6,89 (d, 1H, *J* = 16,3 Hz), 6,91 (d, 1H, *J* = 7,8 Hz), 7,18 à 7,36 (m, 6H), 7,53 (d, 2H, *J* = 8,2 Hz), 7,90 (d, 2H, *J* = 8,3 Hz), 13,08 (br s, 1H).

### EXEMPLE 53

### Acide 4-[4-(3-hydroxy-4'-méthyl-biphényl-2-yl)-but-3-èn-(E)-1-ynyl]-benzoïque.

### (a) 4-[4-(3-hydroxy-4'-méthyl-biphényl-2-yl)-but-3-èn-(E)-1-ynyl]-benzoate de méthyle.

De manière analogue à l'exemple 32(a), à partir de 500 mg (1,2 mmole) de l'ester méthylique obtenu à l'exemple 52(f), on obtient 350 mg (80%) du composé attendu, sous la forme d'une poudre beige composée du mélange des isomères (Z) et (E).
¹H NMR (CDCl₃) (Isomère (E)) δ 2,42 (s, 3H), 3,91 (s, 3H), 5,38 (s, 1H), 6,53 (d, 1H, *J* = 16,6 Hz), 6,88 à 6,95 (m, 3H), 7,20 à 7,26 (m, 5H), 7,47 (d, 2H, *J* = 8,3 Hz), 7,97 (d, 2H, *J* = 8,4 Hz),

### (b) acide 4-[4-(3-hydroxy-4'-méthyl-biphényl-2-yl)-but-3-èn-(E)-1-ynyl]-benzoïque.

De manière analogue à l'exemple 1(f), à partir de 320 mg (0,87 mmole) du mélange des esters méthyliques obtenus à l'exemple 53(a), on obtient 250 mg (80%) d'acide 4-[4-(3-hydroxy-4'-méthyl-biphényl-2-yl)-but-3-èn-(E)-1-ynyl]-benzoïque, sous la forme d'une poudre jaune de point de fusion 221-223°C.
¹H NMR (DMSO D₆) δ 2,37 (s, 3H), 6,70 (d, 1H, *J* = 8,3 Hz), 6,75 (d, 1H, *J* = 16,6 Hz), 6,87 (d, 1H, *J =* 16,4 Hz), 6,93 (d, 1H, *J =* 7,8 Hz), 7,16 (d, 1H, *J* = 7,6 Hz), 7,18 (d, 2H, *J* = 8,0 Hz), 7,28 (d, 2H, *J* = 8,0 Hz), 7,51 (d, 2H, *J* = 8,3 Hz), 7,89 (d, 2H, *J* = 8,3 Hz), 10,39 (s, 1H).

### EXEMPLE 54

### Acide 4-[4-(3-méthoxy-4'-méthyl-biphényl-2-yl)-but-3-èn-(E)-1-ynyl]-benzoïque.

### (a) 2-((E)-2-iodovinyl)-3-méthoxy-4'-méthyl-biphényle.

De manière analogue à l'exemple 1(d), à partir de 4,43 g (19,6 mmoles) du composé obtenu à l'exemple 52(b), on obtient 3,61 g (53%) du composé attendu, sous la forme d'une huile orange.
¹H NMR (CDCl₃) δ 2,41 (s, 3H), 3,89 (s, 3H), 6,86 à 6,97 (m, 3H), 7,14 à 7,41 (m, 6H).

### (b) 4-[4-(3-méthoxy-4'-méthyl-biphényl-2-yl)-but-3-èn-(E)-1-ynyl]-benzoate de méthyle.

De manière analogue à l'exemple 1(e), par réaction de 3,61 g (10,3 mmoles) du composé obtenu à l'exemple 54(a), avec 1,80 g (11,3 mmoles) de 4-éthynyl benzoate de méthyle, on obtient 900 mg (23%) du composé attendu, sous la forme d'une poudre jaune de point de fusion 124-126°C.
¹H NMR (CDCl₃) δ 2,42 (s, 3H), 3,91 (s, 3H), 3,93 (s, 3H), 6,90 (d, 1H, *J* = 7,4 Hz), 6,93 (d, 1H, *J* = 7,1 Hz), 7,00 (d, 1H, *J* = 16,5 Hz), 7,24 à 7,30 (m, 5H), 7,45 (d, 2H, *J* = 8,4 Hz), 7,95 (d, 2H, *J* = 8,4 Hz).

### (c) acide 4-[4-(3-méthoxy-4'-méthyl-biphényl-2-yl)-but-3-èn-(E)-1-ynyl]-benzoïque.

De manière analogue à l'exemple 1(f), à partir de 900 mg (2,3 mmoles) de l' ester méthylique obtenu à l'exemple 54(b), on obtient 700 mg (82%) d acide 4-[4-(3-méthoxy-4'-méthyl-biphényl-2-yl)-but-3-èn-(E)-1-ynyl]-benzoïque, sous la forme d'une poudre jaune clair de point de fusion 200-202°C.
¹H NMR (CDCl₃ + 2 gouttes de DMSO D₆) δ 2,51 (s, 3H), 4,03 (s, 3H), 6,71 (d, 1H, *J* = 16,5 Hz), 7,07 (d, 1H, *J* = 16,4 Hz), 6,98 (d, 1H, *J* = 7,6 Hz), 7,03 (d, 1H, *J* = 7,9 Hz), 7,32 à 7,40 (m, 5H), 7,53 (d, 2H, *J* = 8,3 Hz), 8,05 (d, 2H, *J* = 8,3 Hz).

### EXEMPLE 55

### Acide 4-[4-(4'-éthoxyméthoxyméthyl-biphényl-2-yl)-but-3-èn-(E)-1-ynyl]-benzoïque

De manière analogue à l'exemple 1(f), à partir de 150 mg (0,35 mmole) de l'ester méthylique obtenu à l'exemple 47(e), on obtient 137 mg (94%) d'acide 4-[4-(4'-éthoxyméthoxyméthyl-biphényl-2-yl)-but-3-èn-(E)-1-ynyl]-benzoïque, sous la forme d'une poudre beige de point de fusion 183°C.
¹H NMR (CDCl₃ + 2 gouttes de DMSO D₆) δ 1,27 (t, 3H, *J* = 7,1 Hz), 3,70 (q, 2H, *J* = 7,1 Hz), 4,68 (s, 2H), 4,83 (s, 2H), 6,36 (d, 1H, *J* = 16,2 Hz), 7,07 (d, 1H, *J* = 16,2 Hz), 7,29 à 7,47 (m, 7H), 7,47 (d, 2H, *J* = 8,3 Hz), 7,65 à 7,68 (m, 1H), 7,99 d, 2H, *J* = 8,3 Hz).

### EXEMPLE 56

### Acide 4-[4-(4'-éthoxyméthoxyéthyl-biphényl-2-yl)-but-3-èn-(E-1-ynyl]-benzoate de méthyle.

De manière analogue à l'exemple 1(f), à partir de 350 mg (0,79 mmole) de l'ester méthylique obtenu à l'exemple 48(f), on obtient 320 mg (94%) d'acide 4-[4-(4'-éthoxyméthoxyéthyl-biphényl-2-yl)-but-3-èn-(E)-1-ynyl]-benzoïque, sous la forme d'une poudre beige de point de fusion 135°C.
¹H NMR (CDCl₃ + 2 gouttes de DMSO D₆) δ 1,19 (t, 3H, *J* = 7,1 Hz), 2,99 (t, 2H, *J* = 6,8 Hz), 3,55 (q, 2H, *J* = 7,1 Hz), 3,87 (t, 2H, *J* = 6,8 Hz), 4,72 (s, 2H) 6,36 (d, 1H, *J* = 16,2 Hz), 7,11 (d, 1H, *J* = 16,2 Hz), 7,25 à 7,38 (m, 7H), 7,50 (d, 2H, *J* = 8,2 Hz), 7,64 à 7,68 (m, 1H), 8,04 (d, 2H, *J* = 8,2 Hz).

### EXEMPLE 57

### Acide 2-méthyl-4-[4-(4'-méthyl-biphényl-2-yl)-but-3-èn-(E)-1-ynyl]-benzoïque.

### (a) 4-triméthylsilanyléthynyl-2-méthyl-benzoate de méthyle.

De manière analogue à l'exemple 1(a), à partir de 4,00 g (18,4 mmoles) de 4-bromo-2-méthyl-benzoate de méthyle on obtient 3,54 g (100%) du composé attendu, sous la forme d'une huile jaune.
¹H NMR (CDCl₃) à 0,26 (s, 9H), 2,56 (s, 3H), 3,88 (s, 3H), 7,31 à 7,35 (m, 2H), 7,85 (d, 1 H, *J* = 8,0 Hz).

### (b) 4-éthynyl-2-méthyl-benzoate de méthyle.

On mélange dans un tricol de 500 ml, 3,29 g (17,6 mmoles) du composé obtenu à l'exemple 57(a) avec 50 ml de THF, et on ajoute goutte à goutte 19,3 ml (19,3 mmoles) d'une solution de fluorure de tétrabutylammonium (1,0 M dans le THF). On agite à la température ambiante pendant une heure, verse le milieu réactionnel dans l'eau, extrait avec de l'éther éthylique, décante la phase organique, sèche sur sulfate de magnésium, évapore. On recueille 1,20 g (59%) du composé attendu, sous la forme d'une huile jaune.
¹H NMR (CDCl₃) à 2,58 (s, 3H), 3,18 (s, 1H), 3,89 (s, 3H), 7,34 a 7,42 (m, 2H), 7,87 (d, 1H, *J* = 7,9 Hz).

### (c) 2-méthyl-4-[4-(4'-méthyl-biphényl-2-yl)-but-3-èn-(E)-1-ynyl]-benzoate de méthyle.

De manière analogue à l'exemple 1(e), par réaction de 1,20 g (10,4 mmoles) du composé obtenu à l'exemple 57(b) avec 3,67 g (11,4 mmoles) du composé obtenu à l'exemple 2(d), on obtient 900 mg (23%) du composé attendu, sous la forme d'une poudre blanche de point de fusion 105°C.
¹H NMR (CDCl₃) δ 2,43 (s, 3H), 2,57 (s, 3H), 3,88 (s, 3H), 6,34 (d, 1H, *J* = 16,2 Hz), 7,09 (d, 1H, *J* = 16,2 Hz), 7,26 à 7,38 (m, 9H), 7,63 à 7,67 (m, 1H), 7,86 (d, 1H, *J* = 8,0 Hz).

### (d) acide 2-méthyl-4-[4-(4'-méthyl-biphényl-2-yl)-but-3-èn-(E)-1-ynyl]-benzoïque.

De manière analogue à l'exemple 1(f), à partir de 900 mg (2,4 mmoles) de l'ester méthylique obtenu à l'exemple 57(c), on obtient 800 mg (92%) d'acide 2-méthyl-4-[4-(4'-méthyl-biphényl-2-yl)-but-3-èn-(E)-1-ynyl]-benzoïque, sous la forme d'une poudre blanche de point de fusion 187°C.
¹H NMR (CDCl₃ + 2 gouttes de DMSO D₆) δ 2,43 (s, 3H), 2,59 (s, 3H), 6,34 (d, 1H, *J* = 16,2 Hz), 7,08 (d, 1H, *J* = 16,2 Hz), 7,26 à 7,37 (m, 6H), 7,34 (d, 2H, *J* = 7,9 Hz), 7,63 à 7,67 (m, 1H), 7,91 (d, 2H, *J* = 7,8 Hz).

### EXEMPLE 58

### Acide 2-hydroxy-4-[4-(4'-méthyl-biphényl-2-yl)-but-3-èn-(E)-1-ynyl]-benzoïque.

### (a) 4-triméthylsilanyléthynyl-2-hydroxy-benzoate de méthyle.

De manière analogue à l'exemple 1(a), à partir de 4,00 g (14,4 mmoles) de 4-iodo-2-hydroxy-benzoate de méthyle, on obtient 3,07 g (86%) du composé attendu, sous la forme d'une huile orangée.
¹H NMR (CDCl₃) δ 0,06 (s, 9H), 3,75 (s, 3H), 6,76 (dd, 1H, *J* = 8,2 / 1,5 Hz), 6,87 (d, 1H, *J* = 1,4 Hz), 7,56 (d, 1H, *J* = 8,2 Hz), 10,53 (s, 1H).

### (b) 4-éthynyl-2-hydroxy-benzoate de méthyle.

De manière analogue à l'exemple 57(b), à partir de 3,07 g (12,4 mmoles) du composé obtenu à l'exemple 58(a), on obtient 2,48 g (100%) du composé attendu, sous la forme d'une poudre beige de point de fusion 62°C.
¹H NMR (CDCl₃) δ 3,21 (s, 1H), 3,96 (s, 3H), 6,98 (dd, 1H, *J* = 8,2 / 1,5 Hz), 7,10 (d, 1H, *J* = 1,3 Hz), 7,78 (d, 1H, *J =* 8,2 Hz), 10,76 (s, 1H).

### (c) 2-hydroxy-4-[4-(4'-méthyl-biphényl-2-yl)-but-3-èn-(E)-1-ynyl]-benzoate de méthyle.

De manière analogue à l'exemple 1(e), par réaction de 2,29 g (13,0 mmoles) du composé obtenu à l'exemple 58(b) avec 4,59 g (14,3 mmoles) du composé obtenu à l'exemple 2(d), on obtient 1,24 g (26%) du composé attendu, sous la forme d'une poudre jaune de point de fusion 96°C.
¹H NMR (CDCl₃) δ 2,43 (s, 3H), 3,94 (s, 3H), 6,33 (d, 1H, *J* = 16,2 Hz), 6,92 (dd, 1H, *J* = 8,2 / 1,5 Hz), 7,03 (d, 1H, *J* = 1,3 Hz), 7,11 (d, 1H, *J* = 16,2 Hz), 7,22 à 7,29 (m, 4H), 7,31 à 7,38 (m, 3H), 7,63 à 7,66 (m, 1H), 7,75 (d, 1H, *J =* 8,2 Hz), 10,74 (s, 1H).

### (d) acide 2-hydroxy-4-[4-(4'-méthyl-biphényl-2-yl)-but-3-èn-(E)-1-ynyl]-benzoïque.

De manière analogue à l'exemple 1(f), à partir de 1,24 g (3,4 mmoles) de l'ester méthylique obtenu à l'exemple 58(c), on obtient 910 mg (76%) d'acide 2-hydroxy-4-[4-(4'-méthyl-biphényl-2-yl)-but-3-èn-(E)-1-ynyl]-benzoïque, sous la forme d'une poudre jaune de point de fusion 211°C.
¹H NMR (CDCl₃ + 2 gouttes de DMSO D₆) δ 2,43 (s, 3H), 6,34 (d, 1H, *J* = 16,2 Hz), 6,90 (dd, 1H, *J* = 8,2 /1,5 Hz), 6,98 (d, 1H, *J* = 1,3 Hz), 7,09 (d, 1H, *J* = 16,3 Hz), 7,22 à 7,38 (m, 7H), 7,63 à 7,67 (m, 1H), 7,79 (d, 1H, *J* = 8,1 Hz), 11,29 (br s, 1H).

### EXEMPLE 59

### Acide 6-[4-(4'-méthyl-biphényl-2-yl)-but-3-èn-(E)-1-ynyl]-pyridine-3-carboxylique.

### (a) 6-triméthylsilanyléthynyl-pyridine-3-carboxylate d' éthyle.

De manière analogue à l'exemple 1(a), à partir de 4,00 g (14,4 mmoles) de 6-iodo-pyridine-3-carboxylate d' éthyle, on obtient 3,29 g (92%) du composé attendu, sous la forme d'une poudre beige de point de fusion 55°C.
¹H NMR (CDCl₃) δ 0,10 (s, 9H), 1,22 (t, 2H, *J* = 7,1 Hz), 4,23 (q, 3H *J =* 7,1 Hz), 7,33 (d, 1H, *J* = 8,2 Hz), 8,06 (dd, 1H, *J* = 8,1 / 2,1 Hz), 8,97 (d, 1H, *J* = 2,1 Hz).

### (b) 6-éthynyl-pyridine-3-carboxylate de méthyle.

De manière analogue à l'exemple 57(b), à partir de 3,29 g (13,3 mmoles) du composé obtenu à l'exemple 59(a), on obtient 1,00 g (43%) du composé attendu, sous la forme de paillettes beiges de point de fusion 35°C.
¹H NMR (CDCl₃) δ 1,42 (t, 3H, *J =* 7,1 Hz), 3,33 (s, 1H), 4,42 (q, 2H, *J* = 7,2 Hz), 7,56 (d, 1H, *J* = 8,1 Hz), 8,28 (dd, 1H, *J* = 8,1 / 2,1 Hz), 9,18 (d, 1H, *J* = 2,0 Hz).

### (c) 6-[4-(4'-méthyl-biphényl-2-yl)-but-3-èn-(E)-1-ynyl]-pyridine-3-carboxylate de méthyle.

De manière analogue à l'exemple 1(e), par réaction de 920 mg (5,3 mmoles) du composé obtenu à l'exemple 59(b) avec 1,86 g (5,8 mmoles) du composé obtenu à l'exemple 2(d), on obtient 680 mg (35%) du composé attendu, sous la forme d'une poudre beige de point de fusion 75-80°C.
¹H NMR (CDCl₃) δ 1,41 (t, 3H, *J* = 7,1 Hz), 2,43 (s, 3H), 4,41 (q, 2H, *J* = 7,1 Hz), 6,35 (d, 1H, *J* = 16,2 Hz), 7,19 à 7,26 (m, 5H), 7,34 à 7,39 (m, 3H), 7,47 (d, 1H, *J* = 8,2 Hz), 7,64 à 7,68 (m, 1H), 8,23 (dd, 1H, *J* = 8,2 / 2,1 Hz), 9,15 (d, 1H, *J* = 1,6 Hz).

### (d) acide 6-[4-(4'-méthyl-biphényl-2-yl)-but-3-èn-(E)-1-ynyl]-pyridine-3-carboxylique.

De manière analogue à l'exemple 1(f), à partir de 680 mg (1,9 mmole) de l'ester méthylique obtenu à l'exemple 60(c), on obtient 530 mg (84%) d'acide 6-[4-(4'-méthyl-biphényl-2-yl)-but-3-èn-(E)-1-ynyl]-pyridine-3-carboxylique, sous la forme d'une poudre jaune de point de fusion 207°C.
¹H NMR (CDCl₃ + 2 gouttes de DMSO D₆) δ 2,25 (s, 3H), 6,18 (d, 1H, *J* = 16,2 Hz), 6,99 à 7,21 (m, 7H), 7,48 à 7,51 (m, 1H), 8,06 (dd, 1H, *J* = 8,1 / 2,0 Hz), 8,96 (d, 1H, *J* = 1,3 Hz).

### EXEMPLE 60

### Acide 5-[4-(4'-méthyl-biphényl-2-yl)-but-3-èn-(E)-1-ynyl]-pyridine-2-carboxylique.

### (a) 6-triméthylsilanyléthynyl-pyridine-2-carboxylate de méthyle.

De manière analogue à l'exemple 1(a), à partir de 7,00 g (26,6 mmoles) de 6-iodo-pyridine-2-carboxylate de méthyle, on obtient 4,25 g (68%) du composé attendu, sous la forme d'une poudre orangée de point de fusion 45°C.
¹H NMR (CDCl₃) δ 0,28 (s, 9H), 4,01 (s, 3H), 7,87 (dd, 1H, *J* = 8,1 / 2,0 Hz), 8,08 (d, 1H, *J* = 8,1 Hz), 8,77 (d, 1H, *J* = 1,3 Hz).

### (b) 6-éthynyl-pyridine-2-carboxylate de méthyle.

De manière analogue à l'exemple 57(b), à partir de 2,25 g (9,6 mmoles) du composé obtenu à l'exemple 60(a), on obtient 380 mg (24%) du composé attendu, sous la forme d'une poudre jaune de point de fusion 40-45°C.
¹H NMR (CDCl₃) δ 3,40 (s, 1H), 4,02 (s, 3H), 7,93 (dd, 1H, *J =* 8,1 / 2,0 Hz), 8,12 (d, 1H, *J* = 8,1 Hz), 8,83 (d, 1H, *J* = 1,9 Hz).

### (c) 5-[4-(4'-méthyl-biphényl-2-yl)-but-3-èn-(E)-1-ynyl]-pyridine-2-carboxylate de méthyle.

De manière analogue à l'exemple 1(e), par réaction de 260 mg (1,6 mmole) du composé obtenu à l'exemple 60(b) avec 568 mg (1,8 mmole) du composé obtenu à l'exemple 2(d), on obtient 130 mg (23%) du composé attendu, sous la forme d'une poudre beige de point de fusion 140°C.
¹H NMR (CDCl₃) δ 2,44 (s, 3H), 4,01 (s, 3H), 6,35 (d, 1H, *J* = 16,3 Hz), 7,17 (d, 1H, *J* = 16,2 Hz), 7,26 à 7,38 (m, 6H), 7,62 à 7,75 (m, 2H), 7,84 (dd, 1H, *J* = 8,2 / 1,2 Hz), 8,08 (d, 1H, *J* = 8,1 Hz), 8,75 (d, 1H, *J* = 1,2 Hz).

### (d) acide 5-[4-(4'-méthyl-biphényl-2-yl)-but-3-èn-(E)-1-ynyl]-pyridine-2-carboxylique.

De manière analogue à l'exemple 1(f), à partir de 130 mg (0,37 mmole) de l'ester méthylique obtenu à l'exemple 60(c), on obtient 90 mg (72%) d'acide 5-[4-(4'-méthyl-biphényl-2-yl)-but-3-èn-(E)-1-ynyl]-pyridine-2-carboxylique, sous la forme d'une poudre beige de point de fusion 190°C.
¹H NMR (CDCl₃ + 2 gouttes de DMSO D₆) δ 2,44 (s, 3H), 6,36 (d, 1H, *J =* 16,2 Hz), 7,19 (d, 1H, *J* = 16,3 Hz), 7,22 à 7,40 (m, 7H), 7,65 à 7,68 (m, 1H), 7,92 (dd, 1H, *J* = 8,1 / 1,8 Hz), 8,16 (d, 1H, *J =* 8,1 Hz), 8,63 (s, 1H).

### EXEMPLE 61 Cet exemple est exclu de l'objet revendiqué

### Acide 5-[4-(4'-méthyl-biphényl-2-yl)-but-3-èn-(E)-1-ynyl]-thiophène-3-carboxylique.

### (a) thiophène-3-carboxylate de méthyle.

Dans un tricol de deux litres, et sous courant d'azote, on introduit 19,85 g (154,9 mmoles) d'acide thiophène-3-carboxylique et 700 ml de méthanol. On ajoute goutte à goutte 7 ml d'acide sulfurique concentré et chauffe à reflux pendant seize heures. On refroidit, évapore le méthanol, ajoute de l'eau, extrait par de l'éther éthylique, lave la phase organique à l'eau jusqu'à pH neutre, sèche sur sulfate de magnésium, filtre et évapore les solvants. On recueille 20,26 g (92%) du composé attendu, sous la forme d'une huile jaune claire.
¹H NMR (CDCl₃) δ 3,87 (s, 3H), 7,30 (dd, 1H, *J* = 3,1 / 5,0 Hz), 7,53 (dd, 1H, *J* = 1,1 / 5,1 Hz), 8,10 (dd, 1H, *J* = 1,1 / 3,0 Hz).

### (b) 5-bromo-thiophène-3-carboxylate de méthyle.

Dans un tricot de deux litres, et sous courant d'azote, on introduit 24,74 g (174,0 mmoles) du composé obtenu à l'exemple 61(a) et 410 ml de dichlorométhane. On refroidit à 10°C et ajoute par petites fractions 51,04 g (382,8 mmoles) de chlorure d'aluminium. Le milieu est alors chauffé à 35°C et on ajoute goutte à goutte 30,60 g de brome en solution dans 30 ml de dichlorométhane. Le reflux obtenu est poursuivi pendant une heure, puis le milieu réactionnel est refroidi et versé dans un mélange eau/éther éthylique. Le produit est extrait par de l'éther éthylique la phase organique lavée à l'eau jusqu'à pH neutre, séchée sur sulfate de magnésium, filtrée et les solvants évaporés. Le résidu obtenu est purifié par chromatographie sur colonne de silice, élué par un mélange composé de 20% de dichlorométhane et 80% d'heptane. Après évaporation des solvants, on recueille 27,42 g (71%) du composé attendu, sous la forme de cristaux jaune clair de point de fusion inférieur à 40°C.
¹H NMR (CDCl₃) δ 3,86 (s, 3H), 7,47 (d, 1H, *J* = 1,5 Hz), 7,99 (d, 1H *J* = 1,4 Hz).

### (c) 5-triméthylsilanyléthynyl-thiophène-3-carboxylate de méthyle.

De manière analogue à l'exemple 1(a), à partir de 27,42 g (124,0 mmoles) du composé obtenu à l'exemple 61(b), on obtient 16,70 g (66%) du composé attendu, sous la forme d'une huile orangée.
¹H NMR (CDCl₃) δ 0,25 (s, 9H), 3,85 (s, 3H), 7,60 (d, 1H, *J* = 1,2 Hz, 7,96 (d, 1H, *J* = 1,2 Hz).

### (d) 5-éthynyl-thiophène-3-carboxylate de méthyle.

De manière analogue à l'exemple 57(b), à partir de 16,70 g (82,5 mmoles) du composé obtenu à l'exemple 61(c), on obtient 3,67 g (27%) du composé attendu, sous la forme d'une poudre jaune de point de fusion 42-45°C.
¹H NMR (CDCl₃) δ 3,36 (s, 1H), 3,87 (s, 3H), 7,65 (d, 1H, *J* = 1,2 Hz), 8,00 (d, 1H, *J* = 1,2 Hz).

### (e) 5-[4-(4'-méthyl-biphényl-2-yl)-but-3-èn-(E)-1-ynyl]-thiophène-3-carboxylate de méthyle.

De manière analogue à l'exemple 1(e), par réaction de 1,00 g (6,0 mmoles) du composé obtenu à l'exemple 61(d) avec 2,12 g (6,0 mmoles) du composé obtenu à l'exemple 2(d), on obtient 1,50 g (69%) du composé attendu, sous la forme d'une huilé jaune.
¹H NMR (CDCl₃) δ 2,43 (s, 3H), 3,85 (s, 3H), 6,32 (d, 1H, *J* = 16,2 Hz), 7,07 (d, 1H, *J* = 16,2 Hz), 7,24 à 7,29 (m, 4H), 7,32 à 7,41 (m, 3H), 7,55 (d, 1H, *J* = 1,2 Hz), 7,62 à 7,65 (m, 1H), 7,97 (d, 1H, *J* = 1,2 Hz).

### (f) acide 5-[4-(4'-méthyl-biphényl-2-yl)-but-3-èn-(E)-1-ynyl]-thiophène-3-carboxylique.

De manière analogue à l'exemple 1(f), à partir de 1,50 g (4,2 mmoles) de l'ester méthylique obtenu à l'exemple 61(e), on obtient 170 mg (12%) d'acide 5-[4-(4'-méthyl-biphényl-2-yl)-but-3-én-(E)-1-ynyl]-thiophène-3-carboxylique, sous la forme d'une poudre beige de point de fusion 156°C.
¹H NMR (CDCl₃ + 2 gouttes de DMSO D₆) δ 2,43 (s, 3H), 6,32 (d, 1H, *J* = 16,2 Hz), 7,08 (d, 1H, *J* = 16,3 Hz), 7,25 à 7,36 (m, 6H), 7,57 (s, 1H), 7,61 à 7,66 (m, 1H), 8,09 (s, 1H).

### EXEMPLE 62

### Acide 3-méthoxyméthoxy-4-[4-(4'-méthyl-biphényl-2-yl)-but-3-èn-(E)-1-ynyl]-benzoïque.

### (a) acide 3-hydroxy-4-iodo-benzoïque.

Dans un tricot de un litre, et sous courant d'azote, on introduit 25,00 g (180,0 mmoles) d'acide 3-hydroxybenzoïque, 7,20 g (180,0 mmoles) ce soude en pastilles, 27,13 g (180,0 mmoles) d'iodure de sodium et 500 ml de méthanol. On refroidit à 0°C et ajoute goutte à goutte, en une heure et cinquante minutes, 374,30 g (180,0 mmoles) d'une solution aqueuse d'hypochlorite de sodium. Le milieu réactionnel est agité pendant deux heures à 0°C, puis on ajoute une solution de thiosulfate de sodium, acidifie à pH 5, extrait avec de l'éther éthylique, lave la phase organique à l'eau jusqu'à pH neutre, sèche sur sulfate de magnésium, filtre et évapore les solvants. On recueille 43,80 g (92%) du composé attendu, sous la forme d'une poudre beige de point de fusion 198°C.
¹H NMR (DMSO D₆) δ 7,13 (dd, 1H, *J* = 8,1/1,9 Hz), 7,43 (d, 1H, *J* = 1,8 Hz), 7,80 (d, 1H, *J* = 8,1 Hz), 10,69 (br s, 1H), 12,98 (br s, 1H).

### (b) 3-hydroxy-4-iodo-benzoate de méthyle.

De manière analogue à l'exemple 61(a), à partir de 43,80 g (166,0 mmoles) de l'acide obtenu à l'exemple 62(a), on obtient 43,54 g (94%) de 3-hydroxy-4-iodo-benzoate de méthyle, sous la forme d'une poudre beige de point de fusion 153°C.
¹H NMR (CDCl₃) δ 3,89 (s, 3H), 7,25 (dd, 1H, *J* = 8,2 / 1,9 Hz), 7,58 (d, 1H, *J* = 1,9 Hz), 7,77 (d, 1H, *J* = 8,2 Hz), 8,79 (br s, 1H).

### (c) 3-méthoxyméthoxy-4-iodo-benzoate de méthyle.

De manière analogue à l'exemple 28(a), à partir de 20,00 g (71,9 mmoles) de 3-hydroxy-4-iodo-benzoate de méthyle et de 6,0 ml (79,1 mmoles) d'éther méthylique de chlorométhyle, on obtient 12,38 g (53%) du composé attendu, sous la forme d'une huile jaune.
¹H NMR (CDCl₃) δ 3,52 (s, 3H), 3,91 (s, 3H), 5,30 (s, 2H), 7,41 (dd, H, *J* = 8,2 / 1,8 Hz), 7,66 (d, 1H, *J* = 1,8 Hz), 7,86 (d, 1H, *J* = 8,2 Hz).

### (d) 4-triméthylsilanyléthynyl-3-méthoxyméthoxy-benzoate de méthyle.

De manière analogue à l'exemple 1(a), à partir de 12,38 g (38,4 mmoles) du composé obtenu à l'exemple 62(c), on obtient 11,73 g (100%) du composé attendu, sous la forme d'une huile orangée.
¹H NMR (CDCl₃) δ 0,08 (s, 9H), 3,35 (s, 3H), 3,71 (s, 3H), 5,10 (s, 2H), 7,30 (d, 1H, *J* = 8,0 Hz), 7,44 (dd, 1H, *J* = 8,0 / 1,5 Hz), 7,53 (d, 1H, *J* = 1,4 Hz).

### (e) 4-éthynyl-3-méthoxyméthoxy-benzoate de méthyle.

De manière analogue à l'exemple 57(b), à partir de 11,73 g (40,1 mmoles) du composé obtenu à l'exemple 62(d), on obtient 4,14 g (47%) du composé attendu, sous la forme d'une huile jaune.
¹H NMR (CDCl₃) δ 3,43 (s, 1H), 3,53 (s, 3H), 3,92 (s, 3H), 5,32 (s, 2H), 7,52 (d, 1H, *J* = 8,0 Hz), 7,66 (dd, 1H, *J* = 8,0 / 1,3 Hz), 7,78 (d, 1H, *J* = 1,3 Hz).

### (f) 3-méthoxyméthoxy-4-[4-(4'-méthyl-biphényl-2-yl)-but-3-èn-(E)-1-ynyl]-benzoate de méthyle.

De manière analogue à l'exemple 1(e), par réaction de 3,89 g (17,7 mmoles) du composé obtenu à l'exemple 62(e) avec 6,22 g (19,4 mmoles) du composé obtenu à l'exemple 2(d), on obtient 3,46 g (47%) du composé attendu, sous la forme d'une poudre de point de fusion 64°C.
¹H NMR (CDCl₃) δ 2,42 (s, 3H), 3,50 (s, 3H), 3,90 (s, 3H), 5,28 (s, 2H), 6,40 (d, 1H, *J* = 16,2 Hz), 7,11 (d, 1H, *J* = 16,2 Hz), 7,20 à 7,30 (m, 3H), 7,32 à 7,37 (m, 4H), 7,45 (d, 1H, *J* = 8,0 Hz), 7,62 à 7,65 (m, 2H), 7,74 (s, 1H).

### (g) acide 3-méthoxyméthoxy-4-[4-(4'-méthyl-biphényl-2-yl)-but-3-èn-(E)-1-ynyl]-benzoïque.

De manière analogue à l'exemple 1(f), à partir de 1,00 g (2,4 mmoles) de l'ester méthylique obtenu à l'exemple 62(f), on obtient 540 mg (56%) d'acide 3-méthoxyméthoxy-4-[4-(4'-méthyl-biphényl-2-yl)-but-3-èn-(E)-1-ynyl]-benzoïque, sous la forme d'une poudre beige de point de fusion 185°C.
¹H NMR (CDCl₃ + 2 gouttes de DMSO D₆) δ 2,43 (s, 3H), 3,49 (s 3H), 5,27 (s, 2H), 6,40 (d, 1H, *J* = 16,2 Hz), 7,09 (d, 1H, *J* = 16,2 Hz), 7,25 à 7,37 (m, 8H), 7,43 (d, 1H, *J* = 8,0 Hz), 7,63 à 7,66 (m, 2H), 7,75 (s, 1H).

### EXEMPLE 63

### Acide 3-hydroxy-4-[4-(4'-méthyl-biphényl-2-yl)-but-3-èn-(E)-1-ynyl]-benzoïque.

### (a) 3-hydroxy-4-[4-(4'-méthyl-biphényl-2-yl)-but-3-èn-(E)-1-ynyl]-benzoate de méthyle.

De manière analogue à l'exemple 32(a), à partir de 2,46 g (6,0 mmoles) de l'ester obtenu à l'exemple 62(f), on obtient 1,81 g (82%) du composé attendu, sous la forme d'une poudre beige de point de fusion 111°C.
¹H NMR (CDCl₃) δ 2,43 (s, 3H), 3,90 (s, 3H), 6,37 (d, 1H, *J* = 16,2 Hz), 7,13 (d, 1H, *J* = 16,2 Hz), 7,25 à 7,29 (m, 4H), 7,34 à 7,39 (m, 4H), 7,55 (dd, 1H, *J* = 8,0 / 1,5 Hz), 7,59 (d, 1H, *J* = 1,5 Hz), 7,63 à 7,67 (m, 1H).

### (b) acide 3-hydroxy-4-[4-(4'-méthyl-biphényl-2-yl)-but-3-èn-(E)-1-ynyl]-benzoïque.

De manière analogue à l'exemple 1(f), à partir de 900 mg (2,4 mmoles) de l'ester méthylique obtenu à l'exemple 63(a), on obtient 130 mg (15%) d'acide 3-hydroxy-4-[4-(4'-méthyl-biphényl-2-yl)-but-3-èn-(E)-1-ynyl]-benzoïque, sous la forme d'une poudré jaune de point de fusion 101°C.
¹H NMR (CDCl₃ + 2 gouttes de DMSO D₆) δ 2,47 (s, 3H), 6,69 (s, 1H), 6,97 (d, 1H, *J* = 16,1 Hz), 7,26 à 7,49 (m, 7H), 7,56 (d, 1H, *J* = 8,2 Hz), 7,60 à 7,74 (m, 2H), 7,97 (d, 1H, *J* = 9,4 Hz), 8,15 (s, 1H).

### EXEMPLE 64

### Acide 3-méthoxy-4-[4-(4'-méthyl-biphényl-2-yl)-but-3-èn-(E)-1-ynyl]-benzoïque.

### (a) 3-méthoxy-4-[4-(4'-méthyl-biphényl-2-yl)-but-3-èn-(E)-1-ynyl]-benzoate de méthyle.

De manière analogue à l'exemple 33(a), à partir de 910 mg (2,5 mmoles) du composé obtenu à l'exemple 63(a) et 170 µl (2,7 mmoles) d'iodure de méthyle, on obtient 870 mg (92%) du composé attendu, sous la forme d'une huile marron.
¹H NMR (CDCl₃) δ 2,43 (s, 3H), 3,92 (s, 3H), 6,40 (d, 1H, *J* = 16,2 Hz), 7,09 (d, 1H, *J* = 16,2 Hz), 7,32 à 7,37 (m, 5H), 7,44 (d, 2H, *J* = 8,0 Hz), 7,53 (s, 1H), 7,58 (dd, 1H, *J* = 8,2 1,2 Hz), 7,62 à 7,72 (m, 1H).

### (b) acide 3-méthoxy-4-[4-(4'-méthyl-biphényl-2-yl)-but-3-èn-(E)-1-ynyl]-benzoïque.

De manière analogue à l'exemple 1(f), à partir de 870 mg (2,3 mmoles) de l'ester méthylique obtenu à l'exemple 64(a), on obtient 500 mg (60%) d'acide 3-méthoxy-4-[4-(4'-méthyl-biphényl-2-yl)-but-3-èn-(E)-1-ynyl]-benzoïque, sous la forme d'une poudre beige de point de fusion 217°C.
¹H NMR (CDCl₃ + 2 gouttes de DMSO D₆) δ 2,43 (s, 3H), 3,92 (s, 3H), 6,40 (d, 1H, *J* = 16,2 Hz), 7,10 (d, 1H, *J* = 16,2 Hz), 7,25 à 7,37 (m, 9H), 7,43 (d, 1H, *J* = 7,9 Hz), 7,56 à 7,65 (m, 3H).

### EXEMPLE 65

### {4-[4-(4'-Méthyl-biphényl-2-yl)-but-3-èn-(E)-1-ynyl]-phényl}-méthanol.

De manière analogue à l'exemple 12(d), à partir de 1,00 g (2,8 mmoles) d'ester obtenu à l'exemple 2(e), on obtient 900 mg (98%) de {4-[4-(4'-méthyl-biphényl-2-yl)-but-3-èn-(E)-1-ynyl]-phényl}-méthanol, sous la forme de cristaux blancs de point de fusion 118-120°C.
¹H NMR (CDCl₃) δ 1,67 (t, 1H, *J* = 5,9 Hz), 2,43 (3H), 4,68 (d, 2H, *J* = 5,7 Hz), 6,34 (d. 1H, *J =* 16,2 Hz), 7,06 (d, 1H, *J =* 16,2 Hz), 7,26 à 7,36 (m, 9H), 7,42 (d, 2H, *J* = 8,2 Hz). 7,62 à 7,66 (m, 1H).

### EXEMPLE 66

### 4-[4-(4'-Méthyl-biphényl-2-yl)-but-3-èn-(E)-1-ynyl]-benzaldéhyde.

De manière analogue à l'exemple 11(d), à partir de 500 mg (1,5 mmole) de l'alcool obtenu à l'exemple 65, on obtient 470 mg (95%) de 4-[4-(4'-méthyl-biphényl-2-yl)-but-3-èn-(E)-1-ynyl]-benzaldéhyde, sous la forme d'une poudre jaune de point de fusion 85°C.
¹H NMR (CDCl₃) δ 2,44 (s, 3H), 6,36 (d, 1H, *J =* 16,2 Hz), 7,13 (d, H, *J* = 16,2 Hz), 7,26 à 7,30 (m, 4H), 7,34 (d, 1H, *J* = 4,1 Hz), 7,35 (d, 1H, *J* = 1,7 Hz), 7,38 (d, 1H, *J* = 4,1 Hz), 7,56 (d, 2H, *J* = 8,2 Hz), 7,64 à 7,68 (m, 1H), 7,82 (d, 2H, *J* = 8,2 Hz), 9,99 (s, 1H).

### EXEMPLE 67

### 4-[4-(4'-Méthyl-biphényl-2-yl)-but-3-èn-(E)-1-ynyl]-phénol.

### (a) 4-triméthylsilanyléthynyl-phénol.

De manière analogue à l'exemple 1(a), à partir de 8,00 g (38,4 mmoles) de 4-iodophénol, on obtient 7,50 g (100%) du composé attendu, sous la forme d'une huile brune.
¹H NMR (CDCl₃) δ 0,23 (s, 9H), 6,75 (d, 2H, *J* = 8,8 Hz), 7,36 (d, 2H, *J* = 8,7 Hz).

### (b) 4-éthynyl-phénol.

Dans un tricol et sous courant d'azote, on introduit 500 mg (2,6 mmoles) du composé obtenu à l'exemple 67(a), 500 mg (8,6 mmoles) de fluorure de potassium, 500 mg (3,3 mmoles) de fluorure de césium, 300 ml de méthanol et 30 ml de tétrahydrofuranne. Le milieu réactionnel est agité pendant seize heures à la température ambiante, puis on évapore les solvants et purifie le résidu obtenu par passage sur un cake de silice, élué par un mélange composé de 80% d'heptane et de 20% de dichlorométhane. On obtient, après évaporation des solvants, 280 mg (90%) du composé attendu, sous la forme d'une huile brune.
¹H NMR (CDCl₃) δ 3,16 (s, 1H), 6,55 (d, 2H, *J* = 8,6 Hz), 7,09 (d, 2H, *J* = 8,6 Hz), 9,67 (s, 1H).

### (c) 4-[4-(4'-méthyl-biphényl-2-yl)-but-3-èn-(E)-1-ynyl]-phénol.

De manière analogue à l'exemple 1(e), par réaction de 200 mg (1,7 mmole) du composé obtenu à l'exemple 67(b) avec 420 mg (1,3 mmole) du composé obtenu à l'exemple 2(d), on obtient 60 mg (15%) de 4-[4-(4'-méthyl-biphényl-2-yl)-but-3-èn-(E)-1-ynyl]-phénol, sous la forme d'une poudre marron de point de fusion 80-82°C.
¹H NMR (CDCl₃) δ 2,42 (s, 3H), 5,03 (s, 1H), 6,32 (d, 1H, *J* = 16,2 Hz), 6,76 (d, 2H, *J* = 8,7 Hz), 7,01 (d, 1H, *J* = 16,2 Hz), 7,25 à 7,36 (m, 9H), 7,61 à 7,63 (m, 1H).

### EXEMPLE 68

### 4-[4-(4'-Méthyl-biphényl-2-yl)-but-3-èn-(E)-1-ynyl]-benzamide.

### (a) chlorure de 4-[4-(4'-méthyl-biphényl-2-yl)-but-3-èn-(E)-1-ynyl]-benzoyle.

Dans un tricol et sous courant d'azote, on introduit 450 mg (1,3 mmole) de l'acide obtenu à l'exemple 2(f) et 20 ml de dichlorométhane. On coule goutte à goutte 279 µl (1,4 mmole) de dicyclohexylamine et agite la solution obtenue pendant dix minutes à la température ambiante. On coule goutte à goutte 101 µl (1,4 mmole) de chlorure de thionyle et agite la solution obtenue pendant quinze minutes à la température ambiante. Le milieu réactionnel est évaporé à sec, repris par de l'éther éthylique, filtré et le filtrat évaporé à sec. Le résidu obtenu est utilisé directement pour l'étape suivante.

### (b) 4-[4-(4'-méthyl-biphényl-2-yl)-but-3-èn-(E)-1-ynyl]-benzamide.

Le chlorure d'acide obtenu à l'étape précédente est solubilisé dans 10 ml de THF et la solution ainsi obtenue est coulée goutte à goutte sur une solution composée de 87 µl (1,5 mmole) d'une solution aqueuse d'ammoniaque à 32%, 222 µl (1,6 mmole) de triéthylamine, et 20 ml de THF. Le milieu réactionnel est agité pendant une heure à la température ambiante, versé dans l'eau et extrait à l'éther éthylique. La phase organique est lavée à l'eau jusqu'à pH neutre, séchée sur sulfate de magnésium, filtrée, et les solvants évaporés. Le résidu obtenu est trituré dans un mélange composé de 90% d'heptane et de 10% d'éther éthylique, filtré et séché. On recueille 380 mg (84%) de 4-[4-(4'-méthyl-biphényl-2-yl)-but-3-èn-(E)-1-ynyl]-benzamide, sous la forme d'une poudre jaune clair de point de fusion 195-197°C.
¹H NMR (CDCl₃ + 2 gouttes de DMSO D₆) δ 2,43 (s, 3H), 5,98 (br s, 1H), 6,35 (d, 1H, *J* = 16,2 Hz), 7,10 (d, 1H, *J =* 16,2 Hz), 7,26 à 7,29 (m, 4H), 7,32 (d, 1H, *J* = 4,2 Hz), 7,34 (d, 1H, *J* = 1,1 Hz), 7,37 (d, 1H, *J* = 4,8 Hz), 7,48 (d, 2H, *J* = 8,4 Hz) 7,63 à 7,67 (m, 1H), 7,78 (d, 2H, *J* = 8,4 Hz).

### EXEMPLE 69

### N-Ethyl-4-[4-(4'-méthyl-biphényl-2-yl)-but-3-èn-(E)-1-ynyl]-beinzamide.

De manière analogue à l'exemple 68(b), à partir de 498 mg (1,4 mmole) du chlorure d'acide obtenu à l'exemple 68(a) et 3 ml (4,1 mmoles) d'une solution aqueuse d'éthylamine à 70%, on obtient 400 mg (80%) de N-éthyl-4-[4-(4'-méthyl-biphényl-2-yl)-but-3-èn-(E)-1-ynyl]-benzamide, sous la forme d'une poudre blanche de point de fusion 133-135°C.
¹H NMR (CDCl₃ + 2 gouttes de DMSO D₆) δ 1,12 (t, 3H, *J* = 7,1 Hz), 2,38 (s, 3H), 3,28 (q, 2H, *J* = 6,9 Hz), 6,64 (d, 1H, *J =* 16,2 Hz), 6,96 (d, 1H, *J* = 16,3 Hz), 7,22 (d, 2H, *J* = 8,0 Hz), 7,31 (d, 2H, *J* = 7,8 Hz), 7,29 à 7,33 (m, 1H), 7,40 à 7,43 ( n, 2H), 7,53 (d, 2H, *J* = 8,3 Hz), 7,82 à 7,85 (m, s, 1H), 7,84 (d, 2H, *J =* 8,3 Hz), 8,56 (t, 1H, *J =* 5,4 Hz).

### EXEMPLE 70

### {4-[4-(4'-Méthyl-biphényl-2-yl)-but-3-èn-(E)-1-ynyl]-phényl}-morpholin-4-yl-méthanone.

De manière analogue à l'exemple 68(b), à partir de 498 mg (1,4 mmole) dû chlorure d'acide obtenu à l'exemple 68(a) et 3 ml (34,4 mmoles) de morpholine, on obtient 510 mg (92%) de {4-[4-(4'-méthyl-biphényl-2-yl)-but-3-èn-(E)-1-ynyl]-phényl}-morpholin-4-yl-méthanone, sous la forme d'une meringue orangée de point de fusion 50°C.
¹H NMR (CDCl₃) δ 2,43 (s, 3H), 3,30 à 3,90 (br m, 8H), 6,34 (d, 1H, *J =* 16,2 Hz), 7,09 (d, 1H, *J* = 16,2 Hz), 7,26 à 7,29 (m, 4H), 7,33 à 7,36 (m, 5H), 7,46 (d, 2H, *J* = 8,3 Hz), 7,63 à 7,67 (m, 1H).

### EXEMPLE 71

### N-(4-Hydroxy-phényl)-4-[4-(4'-méthyl-biphényl-2-yl)-but-3-èn-(E)-1-ynyl]-benzamide.

De manière analogue à l'exemple 68(b), à partir de 380 mg (1,1 mmole) du chlorure d'acide obtenu à l'exemple 68(a) et 138 mg (1,2 mmole) de 4-aminophénol, on obtient 340 mg (70%) de N-(4-hydroxy-phényl)-4-[4-(4'-méthyl-biphényl-2-yl)-but-3-èn-(E)-1-ynyl]-benzamide, sous la forme d'une poudre blanc cassé de point de fusion 253-255°C.
¹H NMR (CDCl₃ + 2 gouttes de DMSO D₆) δ 2,35 (s, 3H), 6,28 (d, 1H, *J* = 16,2 Hz), 6,73 (d, 2H, *J* = 8,8 Hz), 7,00 (d, 1H, *J* = 16,2 Hz), 7,14 à 7,30 (m, 6H), 7,41 (d, 2H, *J* = 8,5 Hz), 7,44 (d, 2H, *J* = 9,1 Hz), 7,56 à 7,60 (m, 1H), 7,83 (d, 2H, *J* = 8,3 Hz), 8,67 (s, 1H), 9,31 (s, 1H).

### B. EXEMPLES DE FORMULATION

### 1) VOIE ORALE

(a) On prépare la composition suivante sous la forme d'un comprimé de 0,8 g

| | |
|---|---|
| Composé de l'exemple 1 | 0,005 g |
| Amidon prégélatinisé | 0,265 g |
| Cellulose microcristalline | 0,300 g |
| Lactose | 0,200 g |
| Stéarate de magnésium | 0,030 g |

Pour le traitement de l'acné, on administrera à un individu adulte 1 à 3 comprimés par jour pendant 3 à 6 mois selon la gravité du cas traité.
(b) On prépare une suspension buvable, destinée à être conditionnée en ampoules de 5 ml

| | |
|---|---|
| Composé de l'exemple 2 | 0,050 g |
| Glycérine | 0,500 g |
| Sorbitol à 70 % | 0,500 g |
| Saccharinate de sodium | 0,010 g |
| Parahydroxybenzoate de méthyle | 0,040 g |
| Arôme q.s. | |
| Eau purifiée q.s.p | 5 ml |

Pour le traitement de l'acné, on administrera à un individu adulte 1 ampoule par jour pendant 3 mois selon la gravité du cas traité.
(c) On prépare la formulation suivante destinée à être conditionnée en gélules :

| | |
|---|---|
| Composé de l'exemple 1 | 0,025 g |
| Amidon de maïs | 0,060 g |
| Lactose q.s.p | 0,300 g |

Les gélules utilisées sont constituées de gélatine, d'oxyde de titane et d'un conservateur.
Dans le traitement du psoriasis, on administrera à un individu adulte, 1 gélule par jour pendant 30 jours.

### 2) VOIE TOPIQUE

(a) On prépare la crème Eau-dans l'Huile non ionique suivante :

| | |
|---|---|
| Composé de l'exemple 1 | 0,100 g |
| Mélange d'alcools de lanoline émulsifs, de cires et d'huiles raffinés, vendu par la Société BDF sous la dénomination "Eucérine anhydre" | 39,900 g |
| Parahydroxybenzoate de méthyle | 0,075 g |
| Parahydroxybenzoate de propyle | 0,075 g |
| Eau déminéralisée stérile q.s.p | 100,000 g |

Cette crème sera appliquée sur une peau psoriatique 1 à 2 fois par jour pendant 30 jours.
(b) On prépare un gel en réalisant la formulation suivante

| | |
|---|---|
| Composé de l'exemple 2 | 0,050 g |
| Erythromycine base | 4,000 g |
| Butylhydroxytoluène | 0,050 g |
| Hydroxypropylcellulose vendue par la société Hercules sous le nom de "KLUCEL HF" | 2,000 g |
| Ethanol (à 95°) q.s.p. | 100,000 g |

Ce gel sera appliqué sur une peau atteinte de dermatose ou une peau acnéique 1 à 3 fois par jour pendant 6 à 12 semaines selon la gravité du cas traité.
(c) On prépare une lotion antiséborrhéique en procédant au mélange des ingrédients suivants :

| | |
|---|---|
| Composé de l'exemple 5 | 0,030 g |
| Propylène glycol | 5,000 g |
| Butylhydroxytoluène | 0,100 g |
| Ethanol (à 95°) q.s.p. | 100,000 g |

Cette lotion sera appliquée deux fois par jour sur un cuir chevelu séborrhéique et on constate une amélioration significative dans un délai compris entre 2 et 6 semaines.
(d) On prépare une composition cosmétique contre les effets néfastes du soleil en procédant au mélange des ingrédients suivants :

| | |
|---|---|
| Composé de l'exemple 8 | 1,000 g |
| Benzylidène camphre | 4,000 g |
| Triglycérides d'acides gras | 31,000 g |
| Monostéarate de glycérol | 6,000 g |
| Acide stéarique | 2,000 g |
| Alcool cétylique | 1,200 g |
| Lanoline | 4,000 g |
| Conservateurs | 0,300 g |
| Propylène glycol | 2,000 g |
| Triéthanolamine | 0,500 g |
| Parfum | 0,400 g |
| Eau déminéralisée q.s.p | 100,000 g |

Cette composition sera appliquée quotidiennement, elle permet de lutter contre le vieillissement photoinduit.
(e) On prépare la crème Huile dans l'Eau non ionique suivante :

| | |
|---|---|
| Composé de l'exemple 10 | 0,500 g |
| Vitamine D3 | 0,020 g |
| Alcool cétylique | 4,000 g |
| Monostéarate de glycérol | 2,500 g |
| Stéarate de PEG 50 | 2,500 g |
| Beurre de Karité | 9,200 g |
| Propylène glycol | 2,000 g |
| Parahydroxybenzoate de méthyle | 0,075 g |
| Parahydroxybenzoate de propyle | 0,075 g |
| Eau déminéralisée stérile q.s.p. | 100,000 g |

Cette crème sera appliquée sur une peau psoriatique 1 à 2 fois par jour pendant 30 Jours.
(f) On prépare un gel topique en procédant au mélange des ingrédients suivants :

| | |
|---|---|
| Composé de l'exemple 9 | 0,050 g |
| Ethanol | 43,000 g |
| a -tocophérol | 0,050 g |
| Polymère carboxyvinylique vendu sous la dénomination "Carbopol 941" par la société "Goodrich" | 0,500 g |
| Triéthanolamine en solution aqueuse à 20 % en poids | 3,800 g |
| Eau | 9,300 g |
| Propylène glycol qsp | 100,000 g |

Ce gel sera appliqué dans le traitement de l'acné 1 à 3 fois par jour pendant 6 à 12 semaines selon la gravité du cas traité.
(g) On prépare une lotion capillaire anti-chute et pour la repousse des cheveux en procédant au mélange des ingrédients suivants :

| | |
|---|---|
| Composé de l'exemple 12 | 0,05 g |
| Composé vendu sous la dénomination "Minoxidil" | 1,00 g |
| Propylène glycol | 20,00g |
| Ethanol | 34,92 g |
| Polyéthylèneglycol (masse moléculaire = 400) | 40,00 g |
| Butylhydroxyanisole | 0,01 g |
| Butylhydroxytoluène | 0,02 g |
| Eau qsp | 100,00 g |

On appliquera cette lotion 2 fois par jour pendant 3 mois sur un cuir chevelu ayant subi une chute de cheveu importante.
(h) On prépare une crème anti-acnéique en procédant au mélange des ingrédients suivants :

| | |
|---|---|
| Composé de l'exemple 18 | 0,050 g |
| Acide rétinoïque | 0,010 g |
| Mélange de stéarates de glycérol et de polyéthylène glycol (75 moles) vendu sous le nom de "Gelot 64" ..par la société "GATTEFOSSE" | 15,000 g |
| Huile de noyau polyoxyéthylénée à 6 moles d'oxyde d'éthylène vendue sous le nom de "Labrafil M2130 CS" par la société "GATTEFOSSE" | 8,000 g |
| Perhydrosqualène | 10,000 g |
| Conservateurs | qs |
| Polyéthylèneglycol (masse moléculaire = 400) | 8,000 g |
| Sel disodique de l'acide éthylène-diamine tétracétique | 0,050 g |
| Eau purifiée qsp | 100,000g |

Cette crème sera appliquée sur une peau atteinte de dermatose ou une peau acnéique 1 à 3 fois par jour pendant 6 à 12 semaines.
(i) On prépare une crème huile dans l'eau en réalisant la formulation suivante :

| | |
|---|---|
| Composé de l'exemple 33 | 0,020 g |
| 17-valérate de bétamethasone | 0,050 g |
| S-carboxyméthyl cystéine | 3, 000 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de "Tween 20" par la société "ATLAS" | 1,800 g |
| Mélange de mono et distéarate de glycérol vendu sous la dénomination de "Géléol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" par la société "DYNAMIT NOBEL" | 4,000 g |
| Triéthanolamine (99 % en poids) | 2,500 g |
| Eau q.s.p | 100,000g |

Cette crème sera appliquée 2 fois par jour sur une peau atteinte de dermatose pendant 30 jours.
(j) On prépare la crème de type huile dans l'eau suivante :

| | |
|---|---|
| Acide lactique | 5,000 g |
| Composé de l'exemple 56 | 0,020 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de Tween 20" par la société "ATLAS" | 1,800g |
| Mélange de mono et distéarate de glycérol vendu sous la dénomination de "Geleol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" .. par la société "DYNAMIT NOBEL" | 4,000 g |
| Eau q.s.p. | 100,000 g |

Cette crème sera appliquée 1 fois par jour, elle aide à lutter contre le vieillissement qu'il soit photoinduit ou chronologique.

### C. EXEMPLES DE TESTS

### EXEMPLE 1

Résultats de test pour identifier des molécules antagonistes des RARs tel que décrit dans la demande de brevet français n° 95-07302 déposée le 19 juin 1995 par la Demanderesse.
Le test utilisé est celui de l'oedème de l'oreille de souris induit par application topique de l'acide 2-(5,6,7,8-tétrahydro-5,5,8,8-tétraméthyl-2-naphtyl)-6-benzo[b]thiophencarboxylique (CD270) à 0,01% en poids par volume.
Selon ce modèle, une application topique du composé CD270 sur l'oreille provoque une inflammation qui se caractérise par une augmentation de l'épaisseur de l'oreille de souris. La réponse peut être inhibée par l'administration par voie topique d'une dose (exprimée en % en poids par volume) de composé X.

Les résultats sont regroupés dans le tableau suivant :

| N° exemple | dose (%) | % d'inhibition de l'activité de l'agoniste |
|---|---|---|
| 1 | 0,01 | 78 |
| 2 | 0,01 | 85 |
| 11 | 0,1 | 99 |
| 12 | 0,01 | 45 |
| 14 | 0,1 | 93 |
| 15 | 0,01 | 43 |
| 16 | 0,01 | 34 |
| 17 | 0,1 | 94 |
| 18 | 0,1 | 41 |
| 28 | 0,01 | 31 |
| 42 | 0,01 | 26 |
| 43 | 0,01 | 24 |
| 44 | 0,1 | 54 |
| 45 | 0,1 | 99 |
| 52 | 0,1 | 97 |

Les résultats de ce tableau indiquent que les exemples 1, 2, 11, 12, 14 à 18, 28, 42 à 45 et 52 sont des composés antagonistes RARs.

### EXEMPLE 2

Résultats de test de différenciation des cellules (F9) de tératocarcinome embryonnaire de souris pour identifier des molécules antagonistes des RARs décrits dans Cancer Research 43, p. 5268, 1983.
Dans le test utilisé, un composé est considéré comme artagoniste RAR s'il diminue l'activité de l'agoniste (l'acide 4-[(5,5,8,8-tetramethyl-5,6,7,8-tetrahydro-naphtalen-2-ylamino)-methyl]-benzoique appelé le composé CD2043) sur la morphologie et sur la sécrétion de l'activateur plasminogène par les cellules F9. Dans ce test, les composés sont testés à 10⁻⁶M et le CD2043 est testé à 10⁻⁸M.

Les résultats sont regroupés dans le tableau suivant :

| N° exemple | % d'inhibition de l'activité de l'agoniste |
|---|---|
| 1 | 100 |
| 2 | 94 |
| 11 | 96 |
| 12 | 87 |
| 14 | 95 |
| 15 | 93 |
| 16 | 92 |
| 17 | 98 |
| 18 | 69 |
| 28 | 96 |
| 42 | 91 |
| 43 | 84 |
| 44 | 97 |
| 45 | 95 |
| 52 | 97 |

Les résultats de ce tableau indiquent que les exemples 1, 2, 11, 12, 14 à 18, 28, 42 à 45 et 52 sont des composés antagonistes RARs.

## Revendications

1. Composés triaromatiques, **caractérisés par le fait qu'**ils répondent à la formule générale (I) suivante : 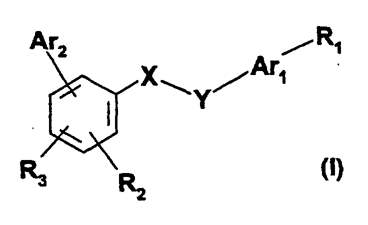 dans laquelle:
- R₁ représente
(i) le radical -CH₃
(ii) le radical -CH₂OH
(iii) le radical -O-R₄
(iv) le radical -CO-R₅
R₄ et R₅ ayant les significations données ci-après.
- Ar₁ est un radical choisi parmi les radicaux de formules (a) ou (b) suivantes: 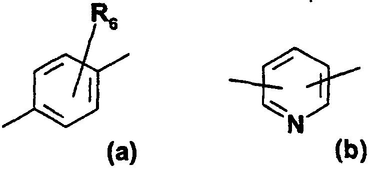 R₆ ayant la signification donnée ci-après.
- X-Y représente une liaison choisie parmi les liaisons de formules (e) à (m) suivantes pouvant être lues de gauche à droite ou inversement : 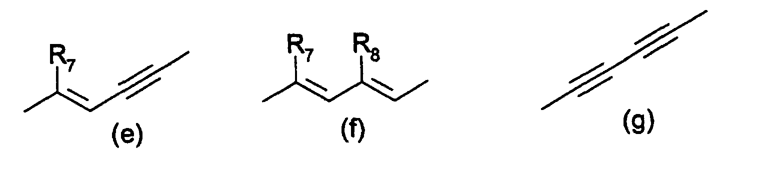 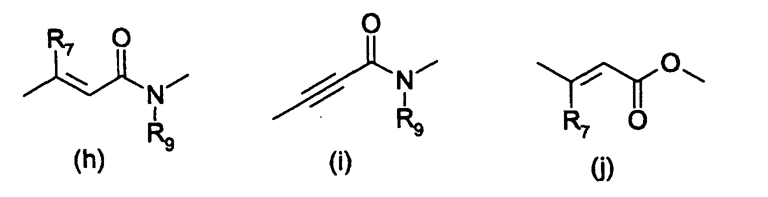 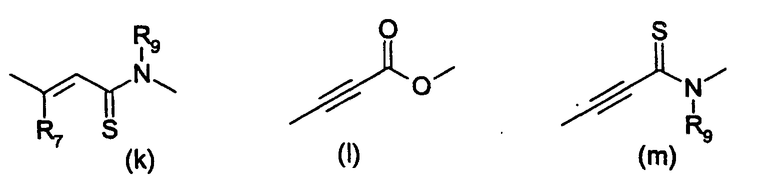 R_{7,} R₈ et R₉ ayant les significations données ci-après
- Ar₂ est un radical choisi parmi les radicaux de formules (j) à (m) suivantes: 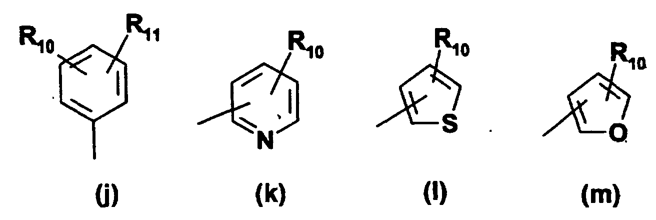 Ar₂ étant en position ortho ou méta sur le phényl par rapport à la liaison X-Y
R₁₀ et R₁₁ ayant les significations données ci-après,
- R₂ et R₃, identiques ou différents, représentent un atome d'hydrogène, un radical alkyle linéaire ou ramifié ayant de 1 à 20 atomes de carbone, un atome d'halogène, un radical hydroxyle éventuellement protégé sous forme d'acétoxy, un radical alkoxy, un radical polyéther, un radical nitro, un radical amino événtuellement protégé sous forme d'acétamide ou substitué par un ou deux groupements alkyles ayant de 1 à 6 atomes de carbone,
- R₄ représente un atome d'hydrogène, un radical alkyle ayant de 1 à 6 atomes de carbone ou un radical -CO-R₁₂,.
R₁₂ ayant la signification donnée ci-après,
- R₅ représente :
(a) un atome d'hydrogène
(b) un radical alkyle ayant de 1 à 6 atomes de carbone
(c) un radical de formule : 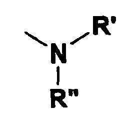 R' et R" ayant les significations données ci-après,
(d) un radical -OR₁₃,
R₁₃ ayant la signification donnée ci-après,
- R₆ représente un atome d'hydrogène, un atome d'halogène, un radical alkyle linéaire ou ramifié ayant de 1 à 20 atomes de carbone, un radical hydroxyle, un radical -OR₁₄ ou -OCOR₁₄, ou un radical polyéther
R₁₄ ayant la signification donnée ci-après,
- R₇ et R₈, identiques ou différents, représentent un atome d'hydrogène, un radical alkyle ayant de 1 à 6 atomes de carbone,
- R₉ représente un atome d'hydrogène ou un radical alkyle ayant de 1 à 6 atomes de carbone,
- R₁₀ et R₁₁, identiques ou différents, représentent un atome d'hydrogène, un radical alkyle linéaire ou ramifié ayant de 1 à 20 atomes de carbone, un atome d'halogène, un radical hydroxyle éventuellement protégé sous forme d'acétoxy, un radical alkoxy, un radical polyéther, un radical nitro, un radical amino événtuellement protégé sous forme d'acétamide ou substitué par un ou deux groupements alkyles ayant de 1 à 6 atomes de carbone, un radical CF₃, un radical alkényle ou un radical
-(CH₂)ₙ-R₁₅,
n et R₁₅ ayant les significations données ci-après,
- R' et R", identiques ou différents, représentent un atome d'hydrogène, un radical alkyle ayant de 1 à 6 atomes de carbone, un radical mono ou polyhydroxyalkyle, un radical aryle éventuellement substitué ou un reste d'aminoacide ou de peptide ou encore pris ensemble, forment un hétérocycle,
- R₁₂ représente un radical alkyle ayant de 1 à 6 atomes de carbone,
- R₁₃ représente un atome d'hydrogène, un radical alkyle linéaire ou ramifié, ayant de 1 à 20 atomes de carbone, un radical alkényle, un radical mono ou polyhydroxyalkyle, un radical aryle ou aralkyle éventuellement substitué(s) ou un reste de sucre,
- R₁₄ représente un radical alkyle ayant de 1 à 6 atomes de carbone,
- R₁₅ représente un radical hydroxyle éventuellement protégé sous forme d'acétoxy, un radical alkoxy, ou un radical polyéther,
- n représente un nombre entier compris entre 1 et 6 inclus,
et les isomères optiques et géométriques desdits composés de formule (I) ainsi que leurs sels.

2. Composés, selon la revendication 1, **caractérisés par le fait qu'**ils se présentent sous forme de sels d'un métal alcalin ou alcalino-terreux, de zinc, d'une amine organique, d'un acide minéral ou organique.

3. Composés selon la revendication 1, **caractérisés par le fait qu'**ils sont pris, seuls ou en mélanges, dans le groupe constitué par :
Acide 4-[4-(biphényl-2-yl)but-3-en-1-ynyl)benzoïque
Acide 4-[4-(4'-méthylbiphényl-2-yl)but-3-en-1-ynyl]benzoïque.
Acide 4-[3-(4'-méthyl-biphényl-2-yl)acryloylamino]-benzoique.
Acide 4-[3-(4'-méthyl-biphényl-2-yl)-(E)-thioacryloylamino]-benzoïque.
Acide 4-[3-(4'-méthyl-biphényl-2-yl)-acryloyloxy]-benzoïque.
Acide 4-[4-(4'-méthyl-biphényl-2-yl)-buta-1(E),3(Z)-diènyl]-benzoïque.
Acide 4-[4-(4'-méthyl-biphényl-2-yl)-buta-1(E),3(E)-diènyl]-benzoïque.
Acide 4-[3-(4'-méthyl-biphényl-2-yl)-propynoyloxy]-benzoïque.
Acides 4-[4-(4'-méthyl-biphényl-2-yl)-(E)/(Z)-but-1-èn-3-ynyl]-benzoïques.
Acide 4-[4-(4'-méthyl-biphényl-2-yl)-buta-1,3-diynyl]-benzoïque.
Acide 4-[4-(3-fluoro-4'-méthyl-biphényl-2-yl)-but-3-èn-(E)-1-ynyl]-benzoïque.
Acide 4-[4-(4,4'-diméthyl-biphényl-2-yl)-but-3-èn-1-ynyl]-benzoïque.
Acide 4-[4-(5,4'-diméthyl-biphényl-2-yl)-but-3-èn-1-ynyl]-benzoïque.
Acide 4-[4-(6,4'-diméthyl-biphényl-2-yl)-but-3-èn-(E)-1-ynyl]-benzoïque.
Acide 4-[4-(4-hydroxy-4'-méthyl-biphényl-2-yl)-but-3-èn-(E)-1-ynyl]-benzoïque.
Acide 4-[4-(5-hydroxy-4'-méthyl-biphényl-2-yl)-but-3-èn-(E)-1-ynyl]-benzoïque.
Acide 4-[4-(6-hydroxy-4'-méthyl-biphényl-2-yl)-but-3-èn-(E)-1-ynyl]-benzoïque.
Acide 4-[4-(4'-méthy)-biphényl-2-yl)-pent-3-èn-(E)-1-ynyl]-benzoïque.
Acide 4-[3-(4'-méthyl-biphényl-2-yl)-propynoylamino]-benzoïque.
Acide 4-[3-(4'-méthyl-biphényl-2-yl)-propynethioylamino]-benzoïque.
Acide 4-[4-(3'-méthyl-biphényl-2-yl)-but-3-èn-1-ynyl]-benzoïque.
Acide 4-[4-(2'-méthyl-biphényl-2-yl)-but-3-èn-(E)-1-ynyl]-benzoïque.
Acide 4-[4-(4'-chloro-biphényl-2-yl)-but-3-èn-1-ynyl]-benzoïque.
Acide 4-[4-(3'-chloro-biphényl-2-yl)-but-3-èn-(E)-1-ynyl]-benzoïque
Acide 4-[4-(4'-fluoro-biphényl-2-yl)-but-3-èn-(E)-1-ynyl]-benzoïque
Acide 4-[4-(4'-propyl-biphényl-2-yl)-but-3-èn-(E)-1-ynyl]-benzoïque.
Acide 4-[4-(4'-vinyl-biphényl-2-yl)-but-3-èn-(E)-1-ynyl]-benzoïque.
Acide 4-[4-(4'-méthoxyméthoxy-biphényl-2-yl)-but-3-èn-(E)-1-ynyl]-benzoïque.
Acide 4-[4-(3'-méthoxyméthoxy-biphényl-2-yl)-but-3-èn-(E)-1-ynyl]-benzoïque.
Acide 4-[4-(2-thiophène-3-yl-phényl)-but-3-èn-(E)-1-ynyl]-benzoïque.
Acide 4-[4-(2-thiophène-2-yl-phényl)-but-3-èn-(E)-1-ynyl]-benzoïque.
Aride 4-[4-(4'-hydroxy-biphényl-2-yl)-but-3-èn-(E)-1-ynyl]-benzoïque.
Acide 4-[4-(4'-méthoxy-biphényl-2-yl)-but-3-èn-(E)-1-ynyl]-benzoïque.
Acide 4-[4-(4'-propoxy-biphényl-2-yl)-but-3-èn-(E)-1-ynyl]-benzoïque.
Acide 4-[4-(3'-hydroxy-biphényl-2-yl)-but-3-èn-(E)-1-ynyl]-benzoïque.
Acide 4-[4-(3'-méthoxy-biphényl-2-yl)-but-3-èn-(E)-1-ynyl]-benzoïque.
Acide 4-[4-(3'-propoxy-biphényl-2-yl)-but-3-èn-(E)-1-ynyl]-benzoïque.
Acide 4-[4-(4'-méthyl-4-hydroxy-biphényl-2-yl)-but-3-èn-(E)-1-ynyl]-benzoïque.
Acide 4-[4-(4'-méthyl-4-méthoxy-biphényl-2-yl)-but-3-èn-(E)-1-ynyl]-benzoïque.
Acide 4-[4-(4'-méthyl-4-propoxy-biphényl-2-yl)-but-3-èn-(E)-1-ynyl]-benzoïque.
Acide 4-[4-(4'-méthyl-5-hydroxy-biphényl-2-yl)-but-3-èn-(E)-1 -ynyl]-benzoïque.
Acide 4-[4-(4'-méthyl-5-méthoxy-biphényl-2-yl)-but-3-èn-(E)-1-ynyl]-benzoïque.
Acide 4-[4-(4'-méthyl-5-propoxy-biphényl-2-yl)-but-3-èn-(E)-1-ynyl]-benzoïque.
Acide 4-[4-(4'-méthyl-6-hydroxy-biphényl-2-yl)-but-3-èn-(E)-1-ynyl]-benzoïque.
Acide 4-[4-(4'-méthyl-6-méthoxy-biphényl-2-yl)-but-3-èn-(E)-1-ynyl]-benzoïque.
Acide 4-[4-(4'-trifuorométhyl-biphényl-2-yl)-but-3-èn(E)-1-ynyl]-benzoïque.
Acide 4-[4-(4'-hydroxyméthyl-biphényl-2-yl)-but-3-èn-(E)-1-ynyl]-benzoïque.
Acide 4-{4-[4'-(2-hydroxy-éthyl)-biphényl-2-yl]-but-3-èn-(E)-1-ynyl}-benzoïque.
Acide 4-[4-(3'-méthyl-biphényl-3-yl)-but-3-èn-(E)-1-ynyl]-benzoïque.
Acide 4-[4-(2-pyridin-4-yl-phényl)-but-3-èn-(E)-1-ynyl]-benzoïque
Acide 4-[4-(2-pyridin-3-yl-phényl)-but-3-èn-(E)-1-ynyl]-benzoïque
Acide 4-[4-(3-méthoxyméthoxy-4'-méthyl-biphényl-2-yl)-but-3-èn-(E)-1-ynyl]-benzoïque.
Acide 4-[4-(3-hydroxy-4'-méthyl-biphényl-2-yl)-but-3-èn-(E)-1-ynyl]-benzoïque.
Acide 4-[4-(3-méthoxy-4'-méthyl-biphényl-2-yl)-but-3-èn-(E)-1-ynyl]-benzoïque.
Acide 4-[4-(4'-éthoxyméthoxyméthyl-biphényl-2-yl)-but-3-èn-(E)-1-ynyl]-benzoïque.
Acide 4-[4-(4'-éthoxyméthoxyéthlyl-biphényl-2-yl)-but-3-èn-(E)-1-ynyl]-benzoate de méthyle.
Acide 2-méthyl-4-[4-(4'-méthyl-biphényl-2-yl)-but-3-èn-(E)-1-ynyl]-benzoïque.
Acide 2-hydroxy-4-[4-(4'-méthyl-biphényl-2-yl)-but-3-èn-(E)-1-ynyl]-benzoïque.
Acide 6-[4-(4'-méthyl-biphényl-2-yl)-but-3-èn-(E)-1-ynyl]-pyridire-3-carboxylique.
Acide 5-[4-(4'-méthyl-biphényl-2-yl)-but-3-èn-(E)-1-ynyl]-pyridine-2-carboxylique.
Acide 3-méthoxyméthoxy-4-[4-(4'-méthyl-biphényl-2-yl)-but-3-èn-(E)-1-ynyl]-benzoïque.
Acide 3-hydroxy-4-[4-(4'-méthyl-biphényl-2-yl)-but-3-èn-(E)-1-ynyl]-benzoïque.
Acide 3-méthoxy-4-[4-(4'-méthyl-biphényl-2-yl)-but-3-èn-(E)-1-ynyl]-benzoïque.
{4-[4-(4'-Méthyl-biphényl-2-yl)-but-3-èn-(E)-1-ynyl]-phényl)-méthanol.
4-[4-(4'-Méthyl-biphényl-2-yl)-but-3-èn-(E)-1-ynyl]-benzaldéhyde.
4-[4-(4'-Méthyl-biphényl-2-yl)-but-3-èn-(E)-1-ynyl]-phénol.
4-[4-(4'-Méthyl-biphényl-2-yl)-but-3-èn-(E)-1-ynyl]-benzamide.
N-Ethyl-4-[4-(4'-méthyl-biphényl-2-yl)-but-3-èn-(E)-1-ynyl]-benzamide.
{4-[4-(4'-Méthyl-biphényl-2-yl)-but-3-èn-(E)-1-ynyl]-phényl}-morpholin-4-yl-méthanone.
N-(4-Hydroxy-phényl)-4-[4-(4'-méthyl-biphényl-2-yl)-but-3-èn-(E)-1-ynyl]-benzamide.

4. Composés selon la revendication 1, **caractérisés par le fait qu'**ils présentent l'une au moins des caractéristiques suivantes :
- R₁ est le radical -CO-R₅,
- Ar₁ représente les radicaux de formules (a) ou (b),
- X-Y représente les liaisons de formule (e), (f), ou (h).
- Ar₂ représente le radical de formule (j),

5. Composés selon l'une quelconque des revendications précédentes, **caractérisé en ce que** R₁ est le radical -CO-OR₁₃, avec R₁₃ représentant un atome d'hydrogène ou un radical alkyle linéaire ou ramifie ayant de 1 à 20 atomes de carbone.

6. Composés selon l'une quelconque des revendications précédentes pour une utilisation comme médicament.

7. Utilisation d'un composé selon la revendication 1 dans la fabrication d'un médicament destiné au traitement des affections dermatologiques liées à un désordre de la kératinisation portant sur la différenciation et sur la prolifération notamment les acnés vulgaires, comédoniennes, polymorphes, rosacées, les acnés nodulokystiques, conglobata, les acnés séniles, les acnés secondaires telles que l'acné solaire, médicamenteuse ou professionnelle; d'autres types de troubles de la kératinisation, notamment les ichtyoses, les états ichtyosiformes, la maladie de Darier, les kératodermies palmoplantaires, les leucoplasies et les états leucoplasiformes, le lichen cutané ou muqueux (buccal); d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale; certaines affections inflammatoires ne présentant pas de trouble de la kératinisation; de toutes les proliférations dermiques ou épidermiques qu'elles soient bénignes ou malignes, qu'elles soient ou non d'origine virale telles que les verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires; d'autres désordres dermatologiques tels que les dermatoses bulleuses et les maladies du collagène; certains troubles ophtalmologiques, notamment les coméopathies; du vieillissement de la peau, qu'il soit photoinduit ou chronologique ou des pigmentations et les kératoses actiniques, ou toutes pathologies associées au vieillissement chronologique ou actinique; des stigmates de l'atrophie épidermique et/ou dermique induite par les corticostéroïdes locaux ou systémiques, ou tout autre forme d'atrophie cutanée, des troubles de la cicatrisation ou des vergetures; de la cicatrisation, des troubles de la fonction sébacée tels que l'hyperséborrhée de l'acné ou la séborrhée simple; des états cancéreux ou précancéreux, plus particulièrement les leucémies promyélocytaires; d'affections inflammatoires telles que l'arthrite, de toute affection d'origine virale au niveau cutané ou général; de l'alopécie; d'affections dermatologiques à composante immunitaire; d'affections du système cardiovasculaire telles que l'artériosclérose, l'hypertension, le diabète non-insulino dépendant, ainsi que l'obésité, de désordres cutanés dus à une exposition aux rayonnements U.V..

8. Composition pharmaceutique, **caractérisée par le fait qu'**elle comprend, dans un support pharmaceutiquement acceptable, au moins l'un des composés tels que définis à l'une quelconque des revendications 1 à 5.

9. Composition selon la revendication 8, **caractérisée en ce que** la concentration en composé(s) selon l'une des revendications 1 à 5 est comprise entre 0,001 % et 5 % en poids par rapport à l'ensemble de la composition.

10. Composition cosmétique, **caractérisée par le fait qu'**elle comprend, dans un support cosmétiquement acceptable, au moins l'un des composés tels que définis à l'une quelconque des revendications 1 à 5.

11. Composition selon la revendication 10, **caractérisée en ce que** la concentration en composé(s) selon l'une des revendications 1 à 5 est comprise entre 0,001 % et 3 % en poids par rapport à l'ensemble de la composition.

12. Utilisation d'une composition cosmétique telle que définie à l'une des revendications 10 ou 11 pour l'hygiène corporelle ou capillaire.

## Patentansprüche

1. Triaromatische Verbindungen, **dadurch gekennzeichnet, daß** sie der folgenden allgemeinen Formel (I) entsprechen: 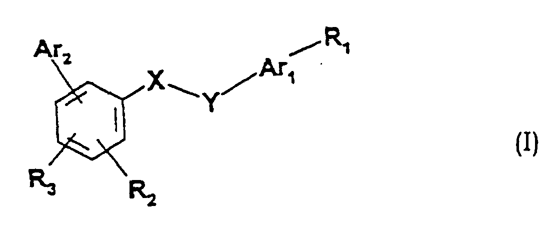 worin bedeuten:
- R₁
(i) die Gruppe -CH₃,
(ii) die Gruppe -CH₂OH,
(iii) eine Gruppe -O-R₄,
(iv) eine Gruppe -CO-R₅,
wobei R₄ und R₅ die nachfolgend angegebenen Bedeutungen aufweisen;
- Ar₁ eine Gruppe, die unter den folgenden Gruppen (a) oder (b) ausgewählt sind: 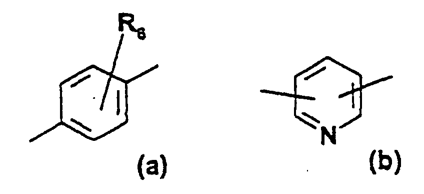 wobei R₆ die nachfolgend angegebenen Bedeutungen aufweist;
- X-Y eine Verbindungsgruppe, die unter den folgenden Verbindungsgruppen der Formeln (e) bis (m) ausgewählt ist, wobei die Formeln in beiden Richtungen gelesen werden können: 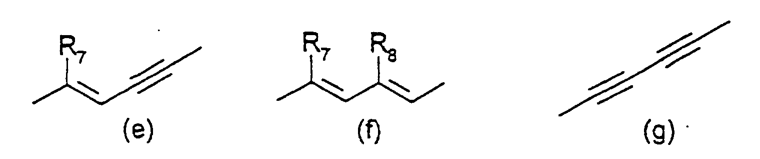 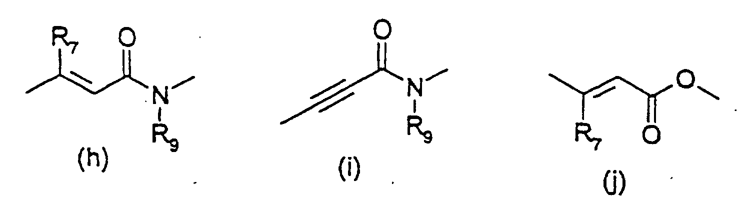 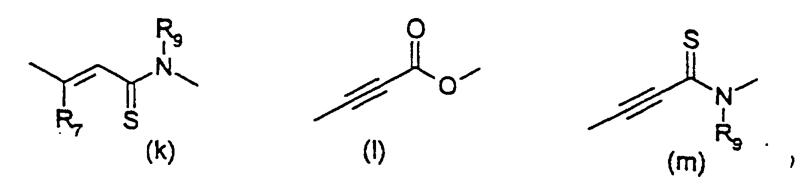 worin R₇, R₈ und R₉ die nachstehend angegebenen Bedeutungen aufweisen;
- Ar₂ ist eine Gruppe, die unter den Gruppen der folgenden Formeln (j) bis (m) ausgewählt ist: 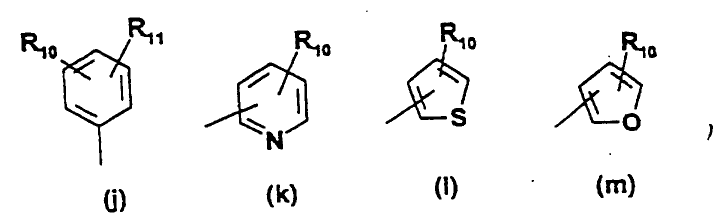 wobei sich Ar₂ im Hinblick auf die Verbindungsgruppe X-Y an der Phenylgruppe in ortho- oder meta-Stellung befindet und wobei R₁₀ und R₁₁ die nachfolgend angegebenen Bedeutungen aufweisen;
- R₂ und R₃, die identisch oder voneinander verschieden sind, Wasserstoff, eine geradkettige oder verzweigte Alkylgruppe mit 1 bis 20 Kohlenstoffatomen, ein Halogenatom, eine Hydroxygruppe, die gegebenenfalls in Form von Acetoxy geschützt ist, eine Alkoxygruppe, eine Polyethergruppe, eine Nitrogruppe oder eine Aminogruppe, die gegebenenfalls in Form von Acetamid geschützt ist oder mit einer oder zwei Alkylgruppen mit 1 bis 6 Kohlenstoffatomen substituiert ist;
- R₄ ein Wasserstoffatom, eine Alkylgruppe mit 1 bis 6 Kohlenstoffatomen oder eine Gruppe -CO-R₁₂, wobei R₁₂ die nachfolgend angegebene Bedeutung hat;
- R₅:
(a) ein Wasserstoffatom,
(b) eine Alkylgruppe mit 1 bis 6 Kohlenstoffatomen,
(c) eine Gruppe der Formel: 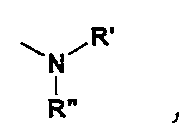 worin R' und R" die nachfolgend angegebenen Bedeutungen aufweisen,
(d) eine Gruppe -OR₁₃, worin R₁₃ die nachfolgend angegebene Bedeutung hat;
- R₆ ein Wasserstoffatom, ein Halogenatom, eine geradkettige oder verzweigte Alkylgruppe mit 1 bis 20 Kohlenstoffatomen, eine Hydroxygruppe, eine Gruppe -OR₁₄, eine Gruppe -OCOR₁₄ oder eine Polyethergruppe, wobei R₁₄ die nachfolgend angegebenen Bedeutungen aufweist;
- R₇ und R₈, die identisch oder voneinander verschieden sind, ein Wasserstoffatom oder eine Alkylgruppe mit 1 bis 6 Kohlenstoffatomen;
- R₉ ein Wasserstoffatom oder eine Alkylgruppe mit 1 bis 6 Kohlenstoffatomen;
- R₁₀ und R₁₁, die identisch oder voneinander verschieden sind, ein Wasserstoffatom, eine geradkettige oder verzweigte Alkylgruppe mit 1 bis 20 Kohlenstoffatomen, ein Halogenatom, eine gegebenenfalls in Form von Acetoxy geschützte Hydroxygruppe, eine Alkoxygruppe, eine Polyethergruppe, eine Nitrogruppe, eine Aminogruppe, die gegebenenfalls in Form von Acetamid geschützt oder mit einer oder zwei Alkylgruppen mit 1 bis 6 Kohlenstoffatomen substituiert ist, eine Gruppe CF₃, eine Alkenylgruppe oder eine Gruppe -(CH₂)ₙ-R₁₅, wobei n und R₁₅ die nachfolgend angegebenen Bedeutungen aufweisen;
- R' und R", die identisch oder voneinander verschieden sind, ein Wasserstoffatom, eine Alkylgruppe mit 1 bis 6 Kohlenstoffatomen, eine Mono- oder Polyhydroxyalkylgruppe, eine gegehenenfalls substituierte Arylgruppe oder einen Aminosäurerest oder Peptidrest oder die Gruppen R' und R" bilden gemeinsam einen Heterocyclus;
- R₁₂ eine Alkylgruppe mit 1 bis 6 Kohlenstoffatomen;
- R₁₃ ein Wasserstoffatom, eine geradkettige oder verzweigte Alkylgruppe mit 1 bis 20 Kohlenstoffatomen, eine Alkenylgruppe, eine Mono- oder Polyhydroxyalkylgruppe, eine Aryl- oder Aralkylgruppe, die gegebenenfalls substituiert sind oder einen Zuckerrest;
- R₁₄ eine Alkylgruppe mit 1 bis 6 Kohlenstoffatomer;
- R₁₅ eine Hydroxygruppe, die gegebenenfalls in Form von Acetoxy geschützt ist, eine Alkoxygruppe oder eine Polyethergruppe; und
- n eine ganze Zahl von 1 bis 6; und
die optischen und geometrischen Isomere der Verbindungen der Formel (I) sowie ihre Salze.

2. Verbindungen nach Anspruch 1, **dadurch gekennzeichnet, daß** sie in Form von Alkalimetallsalzen, Erdalkalimetallsalzen, Zinksalzen oder Salzen von organischen Aminen oder anorganischen oder organischen Säuren vorliegen.

3. Verbindungen nach Anspruch 1, **dadurch gekennzeichnet, daß** sie unter den folgenden Verbindungen oder deren Gemischen ausgewählt sind:
4-[4-(Biphenyl-2-yl)-but-3-en-1-inyl]-benzoesäure,
4-[4-(4'-Methyl-biphenyl-2-yl)-but-3-en-1-inyl]-benzoesäure,
4-[3-(4'-Methyl-biphenyl-2-yl)-acryloylamino]-benzoesäure,
4-[3-(4'-Methyl-biphenyl-2-yl)-(E)-thioacryloylamino] benzoesäure,
4-[3-(4'-Methyl-biphenyl-2-yl)-acryloyloxy]-benzoesäure,
4-[4-(4'-Methyl-biphenyl-2-yl)-buta-1(E), 3(Z)-dienyl]-benzoesäure,
4-[4-(4'-Methyl-biphenyl-2-yl)-buta- 1(E), 3(E)-dienyl]- benzoesäure,
4- [3-(4'-Methyl-biphenyl-2-yl)-propinoyloxy]-benzoesäure,
4-[4-(4'-Methyl-biphenyl-2-yl)-(E)/(Z)-but-1-en-3-inyl]-benzoesäuren,
4-[4-(4'-Methyl-biphenyl-2-yl)-buta-1,3-diinyl]-benzoesäure,
4- [4-(3-Fluoro-4'-methyl-biphenyl-2-yl)-but-3-en-(E)-1-inyl]-benzoesäure,
4-[4-(4,4'-Dimethyl-biphenyl-2-yl)-but-3-en-1-inyl]-benzoesäure,
4-[4-(5,4'-Dimethyl-biphenyl-2-yl)-but-3-en-1-inyl]-benzoesäure,
4-[4-(6,4'-Dimethyl-biphenyl-2-yl)-but-3-en-(E)-1-inyl]-benzoesäure,
4-[4-(4-Hydroxy-4'-methyl-biphenyl-2-yl)-but-3-en-(E)-1-inyl]-benzoesäure,
4-[4-(5-Hydroxy-4'-methyl-biphenyl-2-yl)-but-3-en-(E)-1-inyl]-benzoesäure,
4-[4-(6-Hydroxy-4'-methyl-biphenyl-2-yl)-but-3-en-(E)-1-inyl]-benzoesäure,
4-[4-(4'-Methyl-biphenyl-2-yl)-pent-3-en-(E)-1-inyl]-benzoesäure,
4- [3-(4'-Methyl-biphenyl-2-yl)-propinoylamino]-benzoesäure,
4-[3-(4'-Methyl-biphenyl-2-yl)-propinthioylamino]-benzoesäure,
4-[4-(3'-Methyl-biphenyl-2-yl)-but-3-en-1-inyl]-benzoesäure,
4-[4-(2'-Methyl-biphenyl-2-yl)-but-3-en-(E)-1-inyl]-benzoesäure,
4-[4-(4'-Chlor-biphenyl-2-yl)-but-3-en-1-inyl]-benzoesäure,
4-[4-(3'-Chlor-biphenyl-2-yl)-but-3-en-(E)-1-inyl]-benzoesäure,
4-[4-(4'-Fluor-biphenyl-2-yl)-but-3-en-(E)-1-inyl]-benzoesäure,
4-[4-(4'-Propyl-biphenyl-2-yl)-but-3-en-(E)-1-inyl]-benzoesäure,
4-[4-(4'-Vinyl-biphenyl-2-yl)-but-3-en-(E)-1-inyl]-benzoesäure,
4-[4-(4'-Methoxymethoxy-biphenyl-2-yl)-but-3-en-(E)-1-inyl]-benzoesäure,
4-[4-(3'-Methoxymethoxy-biphenyl-2-yl)-but-3-en-(E)-1-inyl]-benzoesäure,
4-[4-(2-Thiophen-3-yl-phenyl)-but-3-en-(E)-1-inyl]- benzoesäure,
4-[4-(2-Thiophen-2-yl-phenyl)-but-3-en-(E)-1-inyl]-benzoesäure,
4-[4-(4'-Hydroxy-biphenyl-2-yl)-but-3-en-(E)-1-inyl]-benzoesäure,
4-[4-(4'-Methoxy-biphenyl-2-yl)-but-3-en-(E)-1-inyl]-benzoesäure,
4-[4-(4'-Propoxy-biphenyl-2-yl)-but-3-en-(E)-1-inyl]-benzoesäure,
4-[4-(3'-Hydroxy-biphenyl-2-yl)-but-3-en-(E)-1-inyl] benzoesäure,
4-[4-(3'-Methoxy-biphenyl-2-yl)-but-3-en-(E)-1-inyl] benzoesäure,
4-[4-(3'-Propoxy-biphenyl-2-yl)-but-3-en-(E)-1-inyl]-benzoesäure,
4-[4-(4'-Methyl-4-hydroxy-biphenyl-2-yl)-but-3-en-(E)-1-inyl]-benzoesäure,
4-[4-(4'-Methyl-4-methoxy-biphenyl-2-yl)-but-3-en-(E)-1-inyl]-benzoesäure,
4-[4-(4'-Methyl-4-propoxy-biphenyl-2-yl)-but-3-en-(E)-1-inyl]-benzoesäure,
4-[4-(4'-Methyl-5-hydroxy-biphenyl-2-yl)-but-3-en-(E )-1-inyl]-benzoesäure,
4-[4-(4'-Methyl-5-methoxy-biphenyl-2-yl) -but-3-en-(E)-1-inyl]-benzoesäure,
4-[4-(4'-Methyl-5-propoxy-biphenyl-2-yl)-but-3-en-(E-1-inyl]-benzoesäure,
4-[4-(4'-Methyl-6-hydroxy-biphenyl-2-yl)-but-3-en-(E)-1-inyl]-benzoesäure,
4-[4-(4'-Methyl-6-methoxy-biphenyl-2-yl)-but-3-en-(E)-1-inyl]-benzoesäure,
4-[4-(4'-Trifluormethyl-biphenyl-2-yl)-but-3-en-(E)-1-inyl]-benzoesäure,
4-[4-(4'-Hydroxymethyl-biphenyl-2-yl) -but-3-en-(E)-1-inyl]-benzoesäure,
4-{4-[4'-(2-Hydroxy-ethyl)-biphenyl-2-yl]-but-3-en-(E)-1-inyl}-benzoesäure,
4-[4-(3'-Methyl-biphenyl-3-yl)-but-3-en-(E)-1-inyl]-benzoesäure,
4-[4-(2-Pyridin-4-yl-phenyl)-but-3-en-(E)-1-inyl]-benzoesäure,
4-[4-(2-Pydridin-3-yl-phenyl)-but-3-en-(E)-1-inyl]-benzoesäure,
4-[4-(3-Methoxymethoxy-4'-methyl-biphenyl-2-yl)-but-3-en-(E)-1-inyl]-benzoesäure,
4-[4-(3-Hydroxy-4'-methyl-biphenyl-2-yl)-but-3-en-(E)-1-inyl]-benzoesäure,
4-[4-(3-Methoxy-4'-methyl-biphenyl-2-yl)-but-3-en-(E)-1-inyl]-benzoesäure,
4-[4-(4'-Ethoxymethoxymethyl-biphenyl-2-yl)-but-3-en-(E)-1-inyl]-benzoesäure,
Methyl-4-[4-(4'-ethoxymethoxyethyl-biphenyl-2-yl)-b it-3-en-(E)-1-inyl]-benzoat,
2-Methyl-4-[4-(4'-methyl-biphenyl-2-yl)-but-3-en-(E)-1-inyl]-benzoesäure,
2-Hydroxy-4-[4-(4'-methyl-biphenyl-2-yl)-but-3-en-(E)-1-inyl]-benzoesäure,
6-[4-(4'-Methyl-biphenyl-2-yl)-but-3-en-(E)-1-inyl]-pyridin-3-carbonsäure,
5-[4-(4'-Methyl-biphenyl-2-yl)-but-3-en-(E)-1-inyl]-pyridin-2-carbonsäure,
3-Methoxymethoxy-4-[4-(4'-methyl-biphenyl-2-yl)-but-3-en-(E)-1-inyl]-benzoesäure,
3-Hydroxy-4-[4-(4'-methyl-biphenyl-2-yl)-but-3-en-(E)-1-inyl]-benzoesäure,
3-Methoxy-4-[4-(4'-methyl-biphenyl-2-yl)-but-3-en-(E)-1-inyl]-benzoesäure,
{4-[4-(4'-Methyl-biphenyl-2-yl)-but-3-en-(E)-1-inyl]-phenyl}-methanol,
4-[4-(4'-Methyl-biphenyl-2-yl)-but-3-en-(E)-1-inyl]-benzaldehyd,
4-[4-(4'-Methyl-biphenyl-2-yl)-but-3-en-(E)-1-inyl]-phenol,
4-[4-(4'-Methyl-biphenyl-2-yl)-but-3-en-(E)-1-inyl]-benzamid,
N-Ethyl-4-[4-(4'-methyl-biphenyl-2-yl)-but-3-en-(E)-1-inyl]-benzamid,
{4-[4-(4'-Methyl-biphenyl-2-yl)-but-3-en-(E)-1-inyl]-phenyl}-morpholin-4-yl-methanon, und
N-(4-Hydroxy-phenyl)-4-[4-(4'-methyl-biphenyl-2-yl)-but-3-en-(E)-1-inyl]-benzamid.

4. Verbindungen nach Anspruch 1, **dadurch gekennzeichnet, daß** sie mindestens eine der folgenden Eigenschaften aufweisen:
- R₁ bedeutet eine Gruppe -CO-R₅,
- Ar₁ bedeutet die Gruppe der Formel (a) oder der Formel (b),
- X-Y bedeutet eine Verbindungsgruppe der Formel (e), (f) oder (h), und
- Ar₂ bedeutet die Gruppe der Formel (j).

5. Verbindungen nach einem der vorhergehenden Ansprüche, **dadurch gekennzeichnet, daß** R₁ eine Gruppe -CD-DR₁₃ bedeutet, wobei R₁₃ ein Wasserstoffatom oder eine geradkettige oder verzweigte Alkylgruppe mit 1 bis 20 Kohlenstoffatomen ist.

6. Verbindungen nach einem der vorhergehenden Ansprüche zur Verwendung als Arzneimittel.

7. Verwendung einer Verbindung nach Anspruch 1 zur Herstellung eines Arzneimittels, das zur Behandlung der folgenden Störungen und Erkrankungen vorgesehen ist: dermatologische Erkrankungen, die mit einer Verhornungsstörung einhergehen, welche mit der Differenzierung und Proliferation zusammenhängt, insbesondere Acne vulgaris, Acne comedonica, polymorphe Acne, Acne rosaceae, nodulocystische Acne, Acne conglobata, Acne senilis, sekundäre Acneformen, wie Acne solaris, Acne medikamentosa oder Acne professionalis; weitere Arten von Keratinisierungsstörungen, insbesondere Ichtyosis, ichtyosisartige Zustände, Darier Krankheit, Palmoplantarkeratosen, Leukoplakien und leukoplakieforme Zustände und Lichen der Haut oder der Schleimhäute (buccalis); weitere dermatologische Erkrankungen, die mit einer Keratinisierungsstörung mit entzündlicher und/oder immunoallergischer Komponente verbunden sind, und insbesondere alle Arten von Psoriasis der Haut, der Schleimhäute oder der Nägel, Psoriasis arthropathica. Atopie der Haut, beispielsweise Ekzeme, oder Atopie der Atemwege oder auch Hypertrophie des Zahnfleisches; verschiedene entzündliche Erkrankungen, die keine Keratinisierungsstörungen aufweisen; alle Proliferationen der Dermis oder Epidermis, die gutartig oder bösartig und gegebenenfalls viralen Ursprungs sein können, beispielsweise Verrucae vulgares, Verrucae planae und E-pidermodysplasia verruciformis, Papillomatosis oralis oder florida und Proliferationen, die durch UV-Strahlung hervorgerufen werden können, insbesondere im Falle von Epithelioma basocellulare und spinocellulare; weitere dermatologische Erkrankungen, wie beispielsweise bullöse Dermatosen und Erkrankungen des Kollagens; verschiedene ophthalmologische Störungen, insbesondere Corneopathien; Hautalterung, die lichtinduziert oder altersbedingt sein kann, oder Pigmentierungen und aktinische Keratosen oder alle Erkrankungen, die mit der altersbedingten oder aktinischen Hautalterung verbunden sind; Wundmale der epidermalen und/oder dermalen Atrophie, die durch lokal oder systemisch verabreichte Corticosteroide hervorgerufen wird, oder beliebige weitere Formen der Atrophie der Haut, Störungen der Wundheilung oder Streifen; Narbenbildung; Funktionsstörungen der Talgdrüsen, beispielsweise Hyperseborrhoe bei Acne oder Seborrhoe simplex; krebsartige oder präcanceröse Zustände und insbesondere promyelocytäre Leukämie; entzündliche Erkrankungen, wie Arthritis, beliebige Erkrankungen viralen Ursprungs der Haut oder allgemeine virale Erkrankungen; Alopezie; dermatologische Erkrankungen mit Immunkomponente; Erkrankungen des cardiovasculären Systems, beispielsweise Arteriosklerose, Bluthochdruck, nicht insulinpflichtige Diabetes sowie Adipositas und Hautstörungen, die von einer Exposition gegenüber UV-Strahlung herrühren.

8. Pharmazeutische Zusammensetzung, **dadurch gekennzeichnet, daß** sie in einem pharmazeutisch akzeptablen Träger mindestens eine Verbindung nach einem der Ansprüche 1 bis 5 enthält.

9. Zusammensetzung nach Anspruch 8, **dadurch gekennzeichnet, daß** die Konzentration der Verbindung(en) nach einem der Ansprüche 1 bis 5 im Bereich von 0,001 bis 5 Gew.-% bezogen auf die gesamte Zusammensetzung, liegt.

10. Kosmetische Zusammensetzung, **dadurch gekennzeichnet, daß** sie in einem kosmetisch akzeptablen Träger mindestens eine Verbindung nach einem der Ansprüche 1 bis 5 enthält.

11. Zusammensetzung nach Anspruch 10, **dadurch gekennzeichnet, daß** die Konzentration der Verbindung(en) nach einem der Ansprüche 1 bis 5 im Bereich von 0,001 bis 3 Gew.-%, bezogen auf die gesamte Zusammensetzung, liegt.

12. Verwendung einer kosmetischen Zusammensetzung nach einem der Ansprüche 10 oder 11 zur Körper- oder Haarpflege.

## Claims

1. Triaromatic compounds, **characterized in that** they correspond to the general formula (I) below: 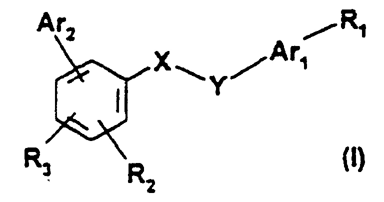 in which:
- R₁ represents
(i) the -CH₃ radical
(ii) the -CH₂OH radical
(iii) the -O-R₄ radical
(iv) the -CO-R₅ radical,
R₄ and R₅ having the meanings given below.
- Ar₁ is a radical chosen from the radicals of formulae (a) or (b) below: 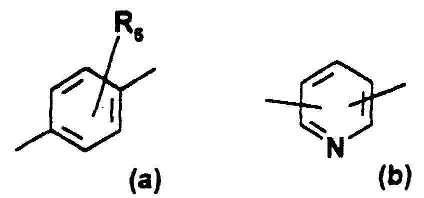 R₆ having the meaning given below.
- X-Y represents a bond chosen from the bonds of formulae (e) to (m) below, which can be read from left to right or vice-versa: 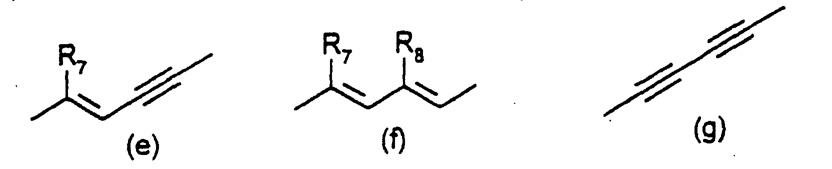 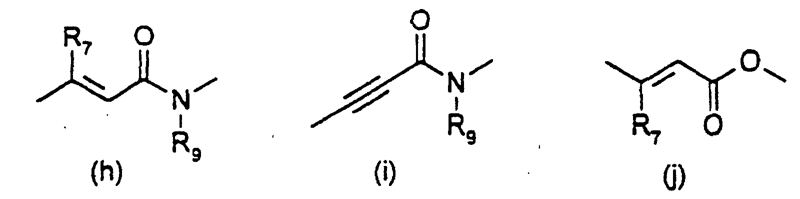 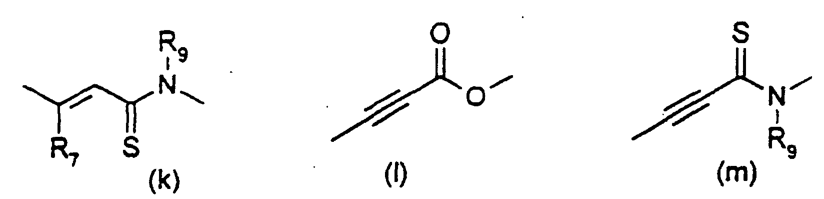 R₇, R₈ and R₉ having the meanings given below,
- Ar₂ is a radical chosen from the radicals of formulae (j) to (m) below: 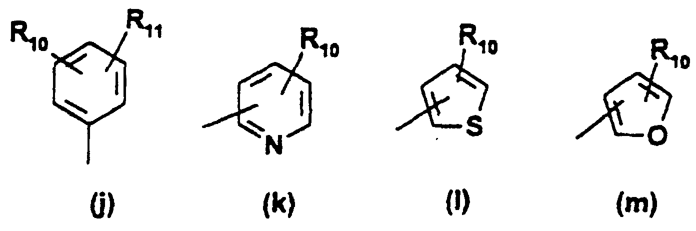 Ar₂ being in an ortho or meta position on the phenyl relative to the X-Y bond
R₁₀ and R₁₁ having the meanings giver below,
- R₂ and R₃, which may be identical or different, represent a hydrogen atom, a linear or branched alkyl radical having from 1 to 20 carbon atoms, a halogen atom, a hydroxyl radical optionally protected in acetoxy form, an alkoxy radical, a polyether radical, a nitro radical, an amino radical optionally protected in acetamide form or substituted with one or two alkyl groups having from 1 to 6 carbon atoms,
- R₄ represents a hydrogen atom, an alkyl radical having from 1 to 6 carbon atoms or a radical -CO-R₁₂,
R₁₂ having the meaning given below,
- R₅ represents:
(a) a hydrogen atom,
(b) an alkyl radical having from 1 to 6 carbon atoms
(c) a radical of formula: 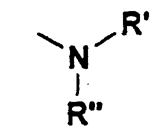 R' and R" having the meanings given below,
(d) a radical -OR₁₃,
R₁₃ having the meaning given below,
- R₆ represents a hydrogen atom, a halogen atom, a linear or branched alkyl radical having from 1 to 20 carbon atoms, a hydroxyl radical, a radical -OR₁₄ or -OCOR₁₄ or a polyether radical,
R₁₄ having the meaning given below,
- R₇ and R₈, which may be identical or different, represent a hydrogen atom, an alkyl radical having from 1 to 6 carbon atoms,
- R₉ represents a hydrogen atom or an alkyl radical having from 1 to 6 carbon atoms,
- R₁₀ and R₁₁, which may be identical or different, represent a hydrogen atom, a linear or branched alkyl radical having from 1 to 20 carbon atoms, a halogen atom, a hydroxyl radical optionally protected in acetoxy form, an alkoxy radical, a polyether radical, a nitro radical, an amino radical optionally protected in acetamide form or substituted with one or two alkyl groups having from 1 to 6 carbon atoms, a CF₃ radical, an alkenyl radical or a radical -(CH₂)ₙ-R₁₅,
n and R₁₅ having the meanings given below,
- R' and R", which may be identical or different, represent a hydrogen atom, an alkyl radical having from 1 to 6 carbon atoms, a mono- or polyhydroxyalkyl radical, an optionally substituted aryl radical or an amino acid or peptide residue, or alternatively, taken together, form a heterocycle,
- R₁₂ represents an alkyl radical having from 1 to 6 carbon atoms,
- R₁₃ represents a hydrogen atom, a linear or branched alkyl radical having from 1 to 20 carbon atoms, an alkenyl radical, a mono- or polyhydroxyalkyl radical, an optionally substituted aryl or aralkyl radical or a sugar residue,
- R₁₄ represents an alkyl radical having from 1 to 6 carbon atoms,
- R₁₅ represents a hydroxyl radical optionally protected in acetoxy form, an alkoxy radical or a polyether radical,
- n represents an integer between 1 and 6 inclusive,
and the optical and geometrical isomers of the said compounds of formula (I), as well as the salts thereof.

2. Compounds according to Claim 1, **characterized in that** they are in the form of salts of an alkali metal or alkaline-earth metal, of zinc, of an organic amine or of an inorganic or organic acid.

3. Compounds according to Claim 1, **characterized in that** they are taken, alone or as mixtures, from the group consisting of:
4-[4-(Biphenyl-2-yl)but-3-en-1-ynyl)benzoic acid,
4-[4-(4'-Methylbiphenyl-2-yl)but-3-en-1-ynyl]benzoic acid,
4-[3-(4'-Methylbiphenyl-2-yl)acryloylamino]benzoic acid,
4-[3-(4'-Methylbiphenyl-2-yl)-(E)-thioacryloylamino]-benzoic acid,
4-[3-(4'-Methylbiphenyl-2-yl)acryloyloxy]benzoic acid,
4-[4-(4'-Methylbiphenyl-2-yl)buta-1(E), 3(Z)-dienyl]-benzoic acid,
4-[4-(4'-Methylbiphenyl-2-yl)buta-1(E),3(E)-dienyl]-benzoic acid,
4-[3-(4'-Methylbiphenyl-2-yl)propynoyloxy]benzoic acid,
4-[4-(4'-Methylbiphenyl-2-yl)-(E)/(Z)-but-1-en-3-ynyl]-benzoic acids,
4-[4-(4'-Methylbiphenyl-2-yl)buta-1,3-diynyl]benzoic acid,
4-[4-(3-Fluoro-4'-methylbiphenyl-2-yl)but-3-en-(E)-1-ynyl]benzoic acid,
4-[4-(4,4'-Dimethylbiphenyl-2-yl)but-3-en-1-ynyl]-benzoic acid,
4-[4-(5,4'-Dimethylbiphenyl-2-yl)but-3-en-1-ynyl]-benzoic acid,
4-[4-(6,4'-Dimethylbiphenyl-2-yl)but-3-en-(E)-1-ynyl]-benzoic acid,
4-[4-(4-Hydroxy-4'-methylbiphenyl-2-yl)but-3-en-(E)-1-ynyl]benzoic acid,
4-[4-(5-Hydroxy-4'-methylbiphenyl-2-yl)but-3-en-(E)-1-ynyl]benzoic acid,
4-[4-(6-Hydroxy-4'-methylbiphenyl-2-yl)but-3-en-(E)-1-ynyl]benzoic acid,
4-[4-(4'-Methylbiphenyl-2-yl)pent-3-en-(E)-1-ynyl]-benzoic acid,
4-[3-(4'-Methylbiphenyl-2-yl)propynoylamino]benzoic acid,
4-[3-(4'-Methylbiphenyl-2-yl)propynethioylamiro]benzoic acid,
4-[4-(3'-Methylbiphenyl-2-yl)but-3-en-1-ynyl)benzoic acid,
4-[4-(2'-Methylbiphenyl-2-yl)but-3-en-(E)-1-ynyl]-benzoic acid,
4-[4-(4'-Chlorobiphenyl-2-yl)but-3-en-1-ynyl]benzoic acid,
4- [4- (3'-Chlorobiphenyl-2-yl)but-3-en-(E)-1-ynyl]-benzoic acid,
4-[4-(4'-Fluorobiphenyl-2-yl)but-3-en-(E)-1-ynyl]-benzoic acid,
4-[4-(4'-Propylbiphenyl-2-yl)but-3-en-(E)-1-ynyl]-benzoic acid,
4-[4-(4'-Vinylbiphenyl-2-yl)but-3-en-(E)-1-ynyl]benzoic acid,
4-[4-(4'-Methoxymethoxybiphenyl-2-yl)but-3-en-(E)-1-ynyl]benzoic acid,
4-[4-(3'-Methoxymethoxybiphenyl-2-yl)but-3-en-(E)-1-ynyl]benzoic acid,
4-[4-(2-Thiophene-3-ylphenyl)but-3-en-(E)-1-ynyl]-benzoic acid,
4-[4-(2-Thiophene-2-ylphenyl)but-3-en-(E)-1-ynyl]-benzoic acid,
4-[4-(4'-Hydroxybiphenyl-2-yl)but-3-en-(E)-1-ynyl]-benzoic acid,
4-[4-(4'-Methoxybiphenyl-2-yl)but-3-en-(E)-1-ynyl]-benzoic acid,
4-[4-(4'-Propoxybiphenyl-2-yl)but-3-en-(E)-1-ynyl]-benzoic acid,
4-[4-(3'-Hydroxybiphenyl-2-yl)but-3-en-(E)-1-ynyl]-benzoic acid,
4-[4-(3'-Methoxybiphenyl-2-yl)but-3-en-(E)-1-ynyl]-benzoic acid,
4-[4-(3'-Propoxybiphenyl-2-yl)but-3-en-(E)-1-ynyl]-benzoic acid,
4- [4- (4'-Methyl-4-hydroxybiphenyl-2-yl)but-3-en-(E)-1-ynyl]benzoic acid,
4- [4- (4'-Methyl-4-methoxybiphenyl-2-yl)but-3-en-(E)-1-ynyl]benzoic acid,
4- [4- (4'-Methyl-4-propoxybiphenyl-2-yl)but-3-en-(E)-1-ynyl]benzoic acid,
4-[4-(4'-Methyl-5-hydroxybiphenyl-2-yl)but-3-en-(E)-1-ynyl]benzoic acid,
4-[4-(4'-Methyl-5-methoxybiphenyl-2-yl)but-3-en-(E)-1-ynyl]benzoic acid,
4-[4-(4'-Methyl-5-propoxybiphenyl-2-yl)but-3-en-(E)-1-ynyl]benzoic acid,
4-[4-(4'-Methyl-6-hydroxybiphenyl-2-yl)but-3-en-(E)-1-ynyl]benzoic acid,
4-[4-(4'-Methyl-6-methoxybiphenyl-2-yl)but-3-en-(E)-1-ynyl]benzoic acid,
4-[4-(4'-Trifluoromethylbiphenyl-2-yl)but-3-en-(E)-1-ynyl]benzoic acid,
4-[4-(4'-Hydroxymethylbiphenyl-2-yl)but-3-en-(E)-1-ynyl]benzoic acid,
4-{4-[4'-(2-Hydroxyethyl)biphenyl-2-yl]but-3-en-(E)-1-ynyl}benzoic acid,
4-[4- (3'-Methylbiphenyl-3-yl)but-3-en-(E)-1-ynyl]-benzoic acid,
4-[4-(2-Pyrid-4-ylphenyl)but-3-en-(E)-1-ynyl]benzoic acid,
4-[4-(2-Pyrid-3-ylphenyl)but-3-en-(E)-1-ynyl]benzoic acid,
4-[4-(3-Methoxymethoxy-4'-methylbiphenyl-2-yl)-but-3-en-(E)-1-ynyl]benzoic acid,
4-[4-(3-Hydroxy-4'-methylbiphenyl-2-yl)but-3-en-(E)-1-ynyl]benzoic acid,
4-[4-(3-Methoxy-4'-methylbiphenyl-2-yl)but-3-en-(E)-1-ynyl]benzoic acid,
4-[4-(4'-Ethoxymethoxymethylbiphenyl-2-yl)but-3-en-(E)-1-ynyl]benzoic acid,
4- [4- (4'-Ethoxymethoxyethylbiphenyl-2-yl).but-3-en-(E)-1-ynyl]benzoic acid,
2-Methyl-4-[4-(4'-methylbiphenyl-2-yl)but-3-en-(E)-1-ynyl]benzoic acid,
2-Hydroxy-4-[4-(4'-methylbiphenyl-2-yl)but-3-en-(E)-1-ynyl]benzoic acid,
6-[4-(4'-Methylbiphenyl-2-yl)but-3-en-(E)-1-ynyl]-pyridine-3-carboxylic acid,
5-[4-(4'-Methylbiphenyl-2-yl)but-3-en-(E)-1-ynyl]-pyridine-2-carboxylic acid,
3-Methoxymethoxy-4-[4-(4'-methylbiphenyl-2-yl)but-3-en-(E)-1-ynyl]benzoic acid,
3-Hydroxy-4-[4-(4'-methylbiphenyl-2-yl)but-3-en-(E)-1-ynyl]benzoic acid,
3-Methoxy-4- [4- (4'-methylbiphenyl-2-yl)but-3-en-(E)-1-ynyl]benzoic acid,
{4-[4-(4'-Methylbiphenyl-2-yl)but-3-en-(E)-1-ynyl]-phenyl}methanol,
4-[4-(4'-Methylbiphenyl-2-yl)but-3-en-(E)-1-ynyl]-benzaldehyde,
4-[4-(4'-Methylbiphenyl-2-yl)but-3-en-(E)-1-ynyl]-phenol,
4-[4-(4'-Methylbiphenyl-2-yl)but-3-en-(E)-1-ynyl]-benzamide,
N-Ethyl-4-[4-(4'-methylbiphenyl-2-yl)but-3-en-(E)-1-ynyl]benzamide,
{4-[4-(4'-Methylbiphenyl-2-yl)but-3-en-(E)-1-ynyl]-phenyl}morpholin-4-ylmethanone,
N-(4-Hydroxyphenyl)-4-[4-(4'-methylbiphenyl-2-yl)but-3-en-(E)-1-ynyl]benzamide.

4. Compounds according to Claim 1, **characterized in that** they have at least one of the following characteristics:
- R₁ is the radical -CO-R₅,
- Ar₁ represents the radicals of formula (a) or (b),
- X-Y represents the bonds of formula (e), (f) or (h),
- Ar₂ represents the radical of formula (j).

5. Compounds according to any one of the preceding claims, **characterized in that** R₁ is the radical -CO-OR₁₃, with R₁₃ representing a hydrogen atom or a linear or branched alkyl radical having from 1 to 20 carbon atoms.

6. Compounds according to any one of the preceding claims, for use as medicaments.

7. Use of a compound according to Claim 1 in the manufacture of a medicament intended for the treatment of dermatological complaints associated with a keratinization disorder which has a bearing on differentiation and on proliferation, in particular for treating common acne, comedones, polymorphonuclear leukocytes, acne rosacea, nodulocystic acne, acne conglobata, senile acne and secondary acnes such as solar acne, medication-induced acne or occupational acne; other types of keratinization disorders, in particular ichthyosis, ichthyosiform states, Darier's disease, palmoplantar keratoderma, leucoplasias and leucoplasiform states, and cutaneous or mucous (buccal) lichen; other dermatological complaints associated with a keratinization disorder having an inflammatory and/or immunoallergic component and, in particular, all forms of psoriasis, whether it is cutaneous, mucous or ungual psoriasis, and even psoriatic rheumatism, or alternatively cutaneous atopy, such as eczema, or respiratory atopy or alternatively gingival hypertrophy; certain inflammatory complaints which do not exhibit a keratinization disorder; all dermal or epidermal proliferations, whether benign or malignant and whether or not they are of viral origin, such as common warts, flat warts and verruciform epidermodysplasia, it being possible for the oral or florid papillomatoses and the proliferations to be induced by ultraviolet radiation, in particular in the case of basocellular and spinocellular epitheliomas; other dermatological disorders such as bullosis and collagen diseases; certain ophthalmological disorders, in particular corneopathies; both light-induced and chronological ageing of the skin or actinic keratoses and pigmentations, or any pathology associated with chronological or actinic ageing; stigmata of epidermal and/or dermal atrophy induced by local or systemic corticosteroids, or any other form of skin atrophy; cicatrization disorders or vibices; cicatrization, disorders of sebaceous functioning such as the hyperseborrhoea of acne or simple seborrhoea; cancerous or precancerous states, more particularly promyelocytic leukaemias; inflammatory complaints such as arthritis; any complaint of viral origin on the skin or generally; alopecia; dermatological complaints having an immune component; complaints of the cardiovascular system such as arteriosclerosis, hypertension, insulin-independent diabetes and obesity, skin disorders due to exposure to UV radiation.

8. Pharmaceutical composition, **characterized in that** it comprises, in a pharmaceutically acceptable support, at least one of the compounds as defined in any one of Claims 1 to 5.

9. Composition according to Claim 8, **characterized in that** the concentration of compound(s) according to one of Claims 1 to 5 is between 0.001% and 5% by weight relative to the composition as a whole.

10. Cosmetic composition, **characterized in that** it comprises, in a cosmetically acceptable support, at least one of the compounds as defined in ary one of Claims 1 to 5.

11. Composition according to Claim 10, **characterized in that** the concentration of compound(s) according to one of Claims 1 to 5 is between 0.001% and 3% by weight relative to the composition as a whole.

12. Use of a cosmetic composition as defined in either of Claims 10 and 11, for body or hair hygiene.
